# EUROPEAN PATENT APPLICATION

(11) **EP 4 382 514 A1**
(43) Date of publication of application: **12.06.2024**
(21) Application number: 22853399.8
(22) Date of filing: 01.08.2022
(51) Int. Cl.: C07D 239/48, A61K 31/505, A61K 31/5377, A61K 31/54, A61K 45/06, A61P 35/00, C07D 403/12, C07D 401/12, C07D 401/14, C07D 417/12

(54) **PYRIMIDINE DERIVATIVE, METHOD FOR PREPARING SAME, AND PHARMACEUTICAL COMPOSITION FOR PREVENTING OR TREATING CANCER COMPRISING SAME AS ACTIVE INGREDIENT**

(30) Priority: 05.08.2021 KR 20210102838; 28.07.2022 KR 20220093706
(71) Applicant: Korea Research Institute of Chemical Technology, Daejeon 34114 (KR); Industry-Academic Cooperation Foundation, Yonsei University, Seoul 03722 (KR)
(72) Inventor: LEE, Kwangho, Daejeon 34114 (KR); DUGGIRALA, Krishna Babu, Daejeon 34114 (KR); CHOI, Gil Don, Daejeon 34114 (KR); CHAE, Chong Hak, Daejeon 34114 (KR); JUNG, Myoung Eun, Daejeon 34114 (KR); LEE, Yujin, Gimcheon-si Gyeongsangbuk-do 39589 (KR); CHO, Byoung Chul, Seoul 04425 (KR)
(74) Representative: Dehns
(86) International application number: PCT/KR2022/011322
(87) International publication number: WO 2023/014022

(57) **Abstract**

The present invention relates to a pyrimidine derivative, a method for preparing same, and a pharmaceutical composition for preventing or treating cancer comprising same as an active ingredient. The pyrimidine derivative of the present invention exhibits a high inhibitory ability against EGFR mutation and HER2 mutation, and thus can be effectively used in treatment of cancer in which EGFR mutation and HER2 mutation have occurred.

## Description

### Technical Field

The present invention relates to a pyrimidine derivative, a preparation method thereof, and a pharmaceutical composition for preventing or treating cancer containing the same as an active ingredient.

### Background Art

Cancer incidence is associated with various environmental factors, including chemicals, radiation, and viruses, and changes in oncogenes, tumor suppressor genes, genes related to apoptosis and DNA repair, *etc.* and with the recent understanding of the molecular mechanism of cancer, has led to a new therapeutic approach to targeted cancer therapy.

Targeted therapeutic agents are generally prepared to target the molecules characteristically possessed by cancer cells so that they can exhibit their effects, and those which become the molecular targets are the genes involved in signal transduction pathway, angiogenesis, matrix, cell cycle regulators, apoptosis, *etc.* Examples of important targeted therapies currently used in treatment are tyrosine 'signal transduction pathway inhibitors' and 'angiogenesis inhibitors', including tyrosine kinase inhibitors.

Protein tyrosine kinase has been known to play an important role in many malignant tumors. In particular, the epidermal growth factor receptor (EGFR), which is a receptor tyrosine kinase of the erbB family, tyrosine kinase is abnormally activated in many epithelial cell tumors including non-small cell lung cancer (NSCLC), breast cancer, glioma, squamous cell carcinoma of the head and neck, colon cancer, intestinal carcinoma, head and neck cancer, gastric cancer, and prostate cancer, and it is known that activation of the EGFR-tyrosine kinase causes persistent cell proliferation, invasion to surrounding tissues, distant metastasis, angiogenesis, and increased cell survival.

Specifically, the EGFR, which is one of the ErbB tyrosine kinase receptors family (EGFR, HER-2, ErbB-3, and ErbB-4), is a transmembrane tyrosine kinase having an intracellular domain that includes an extracellular ligand binding domain and a tyrosine kinase domain. When a ligand binds to a receptor consisting of homodimers or heterodimers, the tyrosine kinase in the cell is activated and the signal stimulated by EGFR as such activates phosphatidylinositol 3-kinase (PI3K)/AKT/mTOR, RAS/RAF/MAPK, and JAK/STAT signal transduction pathways (Nat Rev Cancer 2007; 7: 169-81).

Since EGFR is especially overexpressed in more than half of non-small cell lung cancer (NSCLC), many studies have been conducted using EGFR as a target of treatment. EGFR tyrosine kinase inhibitor (TKI), which inhibits EGFR tyrosine kinase activity, has been developed, and representative agents include gefitinib (IRESSA^{™}), erlotinib (TARCEVA^{™}), and lapatinib (TYKERB^{™}, TYVERB^{™}).

Human EGFR2 (HER2) in cells, which is known as HER2/neu or ErbB2, is a tyrosine kinase receptor belonging to the human epidermal growth factor receptor (HER/EGFR/ERBB) family, and it normally participates in signal transduction pathways to induce cell growth and differentiation. HER2 shows very high structural similarity to the other three EGFR family members (HER1, HER3, and HER4).

However, unlike other anticancer targets, in the case of human epidermal growth factor 2 (Her2), ligands that bind thereto have not yet been known, and Her2 is involved in the increase of cell cycle, regulation of cell proliferation, differentiation, and survival through various signal pathways by forming a heterodimer by partnering with other HER receptors that bind to ligands. In the case of antibodies targeting Her2, antibody treatments of the IgG2 type have been developed and are commercially available, and the main therapeutic effect is neutralizing activity due to antibodies, instead of antibody-dependent cellular cytotoxicity (ADCC) or complement-dependent cytotoxicity (CDC).

As such, while making efforts to develop cancer treatments that inhibit EGFR mutations and HER2 mutations, it was found that the pyrimidine derivative according to the present invention exhibits high inhibitory ability against EGFR mutations and HER2 mutations, so that the pyrimidine derivative may be effectively used in the prevention or treatment of cancer, thereby completing the present invention.

### Disclosure of Invention

### Technical Problem

An object of the present invention is to provide a novel pyrimidine derivative with anticancer activity.

Another object of the present invention is to provide a pharmaceutical composition for preventing or treating cancer containing a novel pyrimidine derivative with anticancer activity as an active ingredient.

### Solution to Problem

In order to achieve the above objects, according to an aspect, the present invention provides a compound represented by the following Formula (1), an isomer thereof, a solvate thereof, a hydrate thereof, or a pharmaceutically acceptable salt thereof. 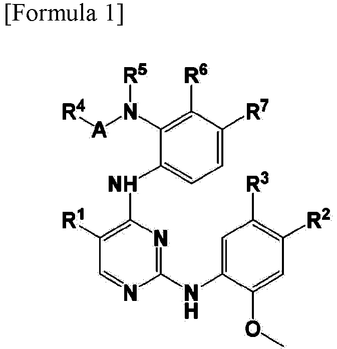
A is -SO₂- or -CO-;
R¹ is hydrogen, halogen, cyano, C₁₋₁₀ alkyl unsubstituted or substituted with one or more halogens, carboxy, or C₁₋₁₀ alkoxycarbonyl; and
R² is -ORₐ or -NR_{b1}R_{b2},
wherein Rₐ is C₁₋₆ alkyl substituted with NR_{b1}R_{b2},
R_{b1} and R_{b2} are each independently hydrogen, C₁₋₆ alkyl unsubstituted or substituted with NR_{c1}R_{c2}, or R_{b1} and R_{b2}, together with the nitrogen to which they are bound, form heterocycloalkyl of 3 to 8 atoms unsubstituted or substituted with C₁₋₆ alkyl or NR_{c1}R_{c2} including one or more heteroatoms selected from the group consisting of N, O, and S,
R_{c1} and R_{c2} are each independently hydrogen, C₁₋₆ alkyl, or R_{c1} and R_{c2}, together with the nitrogen to which they are bound, may form heterocycloalkyl of 3 to 8 atoms unsubstituted or substituted with C₁₋₆ alkyl including one or more heteroatoms selected from the group consisting of N, O, and S;
R³ is 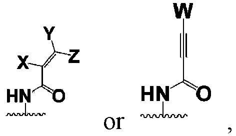
wherein W, X, Y, and Z are independently hydrogen, halogen, or C₁₋₆ alkyl unsubstituted or substituted with NR_{d1}R_{d2},
R_{d1} and R_{d2} are each independently hydrogen or C₁₋₆ alkyl,
Z is hydrogen;
R⁴ to R⁷ are each independently hydrogen, halogen, C₁₋₁₀ alkyl, C₅₋₁₀ aryl unsubstituted or substituted with C₁₋₁₀ alkyl, or any two adjacent ones of R⁴ to R⁷ form 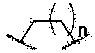 or 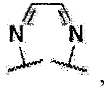 in which one or more single bonds in 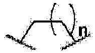 may be substituted with a double bond, and the remaining two are each independently hydrogen or C₁₋₆ alkyl,
n is an integer from 0 to 3, and
the heterocycloalkyl includes at least one N.

Additionally, as represented by Reaction Scheme 1 below, the present invention provides a method for preparing a compound represented by Formula 1, including reacting a compound represented by Formula 2 with a compound represented by Formula 3 to prepare a compound represented by Formula 1: 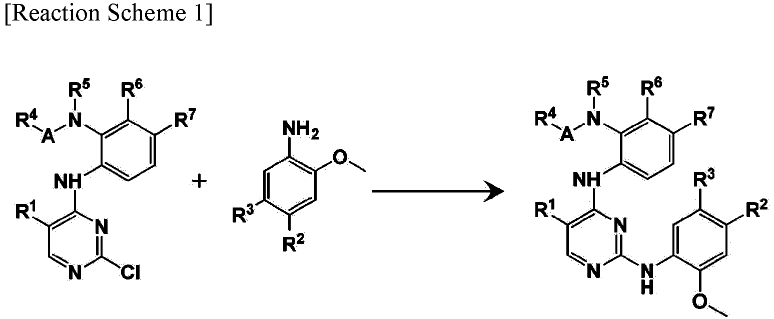 (In Reaction Scheme 1 above, A, R¹, R², R³, R⁴, R⁵, R⁶, and R⁷ are as defined in Formula 1 above.)

According to another aspect, the present invention provides a pharmaceutical composition for the prevention or treatment of cancer including the compound represented by Formula 1 above, an isomer thereof, a solvate thereof, a hydrate thereof, or a pharmaceutically acceptable salt thereof as an active ingredient.

According to still another aspect, the present invention provides a health functional food for the prevention or improvement of cancer including the compound represented by Formula 1 above, an isomer thereof, a solvate thereof, a hydrate thereof, or a pharmaceutically acceptable salt thereof as an active ingredient.

### Advantageous Effects of Invention

Since the pyrimidine derivative of the present invention exhibits high inhibitory ability against EGFR mutations and HER2 mutations, it can be effectively used for the treatment of cancer in which EGFR mutations and HER2 mutations are expressed.

### Best Mode for Carrying out the Invention

Hereinafter, the present invention is described in detail.

In an aspect, the present invention provides a compound represented by Formula 1 below, an isomer thereof, a solvate thereof, a hydrate thereof, or a pharmaceutically acceptable salt thereof.

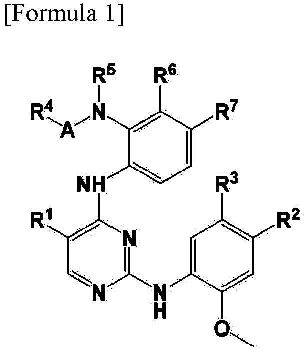

In Formula 1 above,
A is -SO₂- or -CO-;
R¹ is hydrogen, halogen, cyano, C₁₋₁₀ alkyl unsubstituted or substituted with one or more halogens, carboxy, or C₁₋₁₀ alkoxycarbonyl; and
R² is -ORₐ or -NR_{b1}R_{b2},
wherein Rₐ is C₁₋₆ alkyl substituted with NR_{b1}R_{b2},
R_{b1} and R_{b2} are each independently hydrogen, C₁₋₆ alkyl unsubstituted or substituted with NR_{c1}R_{c2}, or R_{b1} and R_{b2}, together with the nitrogen to which they are bound, form heterocycloalkyl of 3 to 8 atoms unsubstituted or substituted with C₁₋₆ alkyl or NR_{c1}R_{c2} including one or more heteroatoms selected from the group consisting of N, O, and S,
R_{c1} and R_{c2} are each independently hydrogen, C₁₋₆ alkyl, or R_{c1} and R_{c2}, together with the nitrogen to which they are bound, are able to form heterocycloalkyl of 3 to 8 atoms unsubstituted or substituted with C₁₋₆ alkyl including one or more heteroatoms selected from the group consisting of N, O, and S;
R³ is 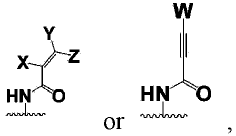
wherein W, X, Y, and Z are independently hydrogen, halogen, or C₁₋₆ alkyl unsubstituted or substituted with NR_{d1}R_{d2},
R_{d1} and R_{d2} are each independently hydrogen or C₁₋₆ alkyl;
R⁴ to R⁷ are each independently hydrogen, halogen, C₁₋₁₀ alkyl, C₅₋₁₀ aryl unsubstituted or substituted with C₁₋₁₀ alkyl, or any two adjacent ones of R⁴ to R⁷ form 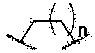 or 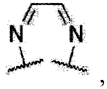 in which one or more single bonds in 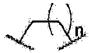 may be substituted with a double bond, and the remaining two are each independently hydrogen or C₁₋₆ alkyl,
n is an integer from 0 to 3, and
the heterocycloalkyl includes at least one N.
In another embodiment of the present invention,
A is -SO₂- or -CO-;
R¹ is hydrogen, halogen, cyano, C₁₋₆ alkyl unsubstituted or substituted with one or more halogens, carboxy, or C₁₋₆ alkoxycarbonyl; and
R² is -ORₐ or -NR_{b1}R_{b2},
wherein Rₐ is C₁₋₄ alkyl substituted with NR_{b1}R_{b2},
R_{b1} and R_{b2} are each independently hydrogen, C₁₋₄ alkyl unsubstituted or substituted with NR_{c1}R_{c2}, or R_{b1} and R_{b2}, together with the nitrogen to which they are bound, form heterocycloalkyl of 5 to 8 atoms unsubstituted or substituted with C₁₋₄ alkyl or NR_{c1}R_{c2} including one or more heteroatoms selected from the group consisting of N, O, and S,
R_{c1} and R_{c2} are each independently hydrogen, C₁₋₃ alkyl, or R_{c1} and R_{c2}, together with the nitrogen to which they are bound, may form heterocycloalkyl of 5 to 8 atoms unsubstituted or substituted with C₁₋₄ alkyl including one or more heteroatoms selected from the group consisting of N, O, and S;
R³ is 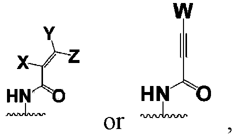
wherein W, X, and Z are independently hydrogen, halogen, or C₁₋₄ alkyl,
Y is hydrogen, or C₁₋₄ alkyl substituted with NR_{d1}R_{d2},
R_{d1} and R_{d2} are each independently hydrogen or C₁₋₄ alkyl;
R⁴ to R⁷ are any one of the following 1) to 4), wherein:
   1) R⁴ to R⁷ are each independently hydrogen, halogen, C₁₋₆ alkyl, or C₆₋₁₀ aryl unsubstituted or substituted with C₁₋₆ alkyl;
   2) R⁴ and R⁵ together form 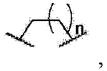 and R⁶ and R⁷ are hydrogen;
   3) R⁵ and R⁶ together form 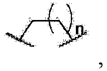 and R⁴ and R⁷ are each independently hydrogen or C₁₋₄ alkyl, in which one or more single bonds in 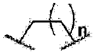 may be substituted with a double bond;
   4) R⁶ and R⁷ together form 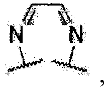 and R⁴ and R⁵ are each independently hydrogen or C₁₋₄ alkyl;
n is an integer from 1 to 3, and
the heterocycloalkyl includes at least one N.

In still another embodiment of the present invention,
A is -SO₂- or -CO-;
R¹ is hydrogen, halogen, cyano, C₁ alkyl unsubstitueted or substituted with one or more halogens, carboxy, or C₁₋₃ alkoxycarbonyl; and
R² is -ORₐ or -NR_{b1}R_{b2},
wherein Rₐ is C₁₋₃ alkyl substituted with NR_{b1}R_{b2},
R_{b1} and R_{b2} are each independently hydrogen, C₁₋₃ alkyl unsubstituted or substituted with NR_{c1}R_{c2}, or R_{b1} and R_{b2}, together with the nitrogen to which they are bound, form heterocycloalkyl of 4 to 6 atoms unsubstituted or substituted with methyl or NR_{c1}R_{c2} including one or more heteroatoms selected from the group consisting of N and O; and
R_{c1} and R_{c2} are each independently hydrogen, methyl, isopropyl, or R_{c1} and R_{c2}, together with the nitrogen to which they are bound, form heterocycloalkyl of 4 to 6 atoms unsubstituted or substituted with methyl including one or more heteroatoms selected from the group consisting of N and O;
R³ is 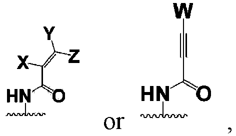
wherein W is methyl,
X is hydrogen or fluorine,
Y is hydrogen or methyl substituted with NR_{d1}R_{d2},
R_{d1} and R_{d2} are each independently hydrogen or methyl,
Z is hydrogen;
R⁴ to R⁷ are any one of the following 1) to 4), wherein:
   1) R⁴ to R⁷ are each independently hydrogen, halogen, C₁₋₃ alkyl, phenyl unsubstituted or substituted with C₁₋₃ alkyl;
   2) R⁴ and R⁵ together form 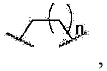 and R⁶ and R⁷ are hydrogen;
   3) R⁵ and R⁶ together form 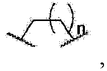 and R⁴ and R⁷ are each independently hydrogen or methyl, in which the single bond in 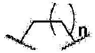 may be substituted with one or more double bonds;
   4) R⁶ and R⁷ together form 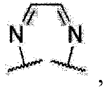 and R⁴ and R⁵ are each independently hydrogen or methyl;
      n is an integer from 1 to 3, and
      the heterocycloalkyl includes at least one N.

In still another embodiment of the present invention,
R¹ is hydrogen, chlorine, fluorine, methyl, cyano, CF₃, 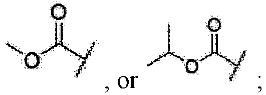
R² is 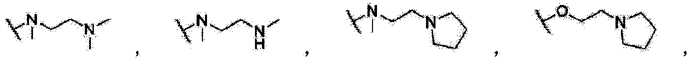 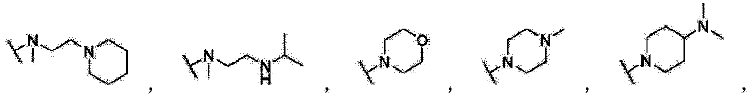 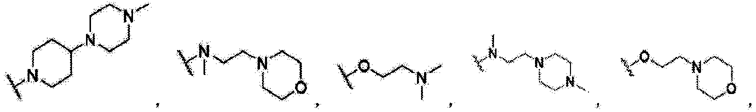 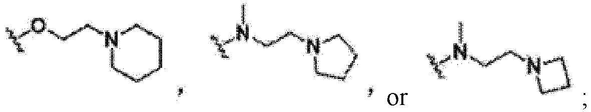
R³ is 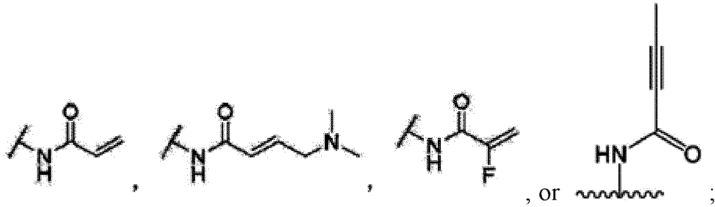 and
R⁴ to R⁷ are any one of the following 1) to 4), wherein:
   1) R⁴ to R⁷ are each independently hydrogen, chlorine, methyl, ethyl, propyl, isopropyl, phenyl, or toluyl;
   2) R⁴ and R⁵ together form 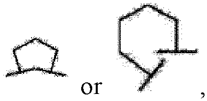 and R⁶ and R⁷ are hydrogen;
   3) R⁵ and R⁶ together form 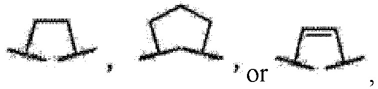 and R⁴ and R⁷ are each independently hydrogen or methyl; and
   4) R⁶ and R⁷ together form 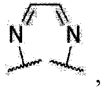 and R⁴ and R⁵ are each independently hydrogen or methyl.

Examples of the compound represented by Formula 1 above according to the present invention may include the following compounds.
<1> N (5-((5-chloro-4-((2-(propylsulfonamido)phenyl)amino)pyrimidin-2-yl)amino)-2-((2-(dimethylamino)ethyl)(methyl)amino)-4-methoxyphenyl)acrylamide;
<2> *N*-(5-((5-chloro-4-((2-(phenylsulfonamido)phenyl)amino)pyrimidin-2-yl)amino)-2-((2-(dimethylamino)ethyl)(methyl)amino)-4-methoxyphenyl)acrylamide;
<3> N (5-((5-chloro-4-((2-((4-methylphenyl)sulfonamido)phenyl)amino)pyrimidin-2-yl)amino)-2-((2-(dimethylamino)ethyl)(methyl)amino)-4-methoxyphenyl)acrylamide;
<4> N (5-((5-chloro-4-((2-(ethylsulfonamido)phenyl)amino)pyrimidin-2-yl)amino)-2-((2-(dimethylamino)ethyl)(methyl)amino)-4-methoxyphenyl)acrylamide;
<5> *N-*(5-((5-chloro-4-((2-((1-methylethyl)sulfonamido)phenyl)amino)pyrimidin-2-yl)amino)-2-((2-(dimethylamino)ethyl)(methyl)amino)-4-methoxyphenyl)acrylamide;
<6> N (2-((2-(dimethylamino)ethyl)(methyl)amino)-4-methoxy-5-((4-((2-(phenylsulfonamido)phenyl)amino)pyrimidin-2-yl)amino)phenyl)acrylamide;
<7> N (2-((2-(dimethylamino)ethyl)(methyl)amino)-4-methoxy-5-((4-((2-(methylsulfonamido)phenyl)amino)pyrimidin-2-yl)amino)phenyl)acrylamide;
<8> N (2-((2-(dimethylamino)ethyl)(methyl)amino)-4-methoxy-5-((4-((2-(propylsulfonamido)phenyl)amino)pyrimidin-2-yl)amino)phenyl)acrylamide;
<9> *N*-(2-((2-(dimethylamino)ethyl)(methyl)amino)-4-methoxy-5-((4-((2-((4-methylphenyl)sulfonamido)phenyl)amino)pyrimidin-2-yl)amino)phenyl)acrylamide;
<10> N (2-((2-(dimethylamino)ethyl)(methyl)amino)-5-((4-((2-(ethylsulfonamido)phenyl)amino)pyrimidin-2-yl)amino)-4-methoxyphenyl)acrylamide;
<11> N (2-((2-(dimethylamino)ethyl)(methyl)amino)-5-((5-fluoro-4-((2-(methylsulfonamido)phenyl)amino)pyrimidin-2-yl)amino)-4-methoxyphenyl)acrylamide;
<12> N-(2-((2-(dimethylamino)ethyl)(methyl)amino)-5-((4-((2-(ethylsulfonamido)phenyl)amino)-5-fluoropyrimidin-2-yl)amino)-4-methoxyphenyl)acrylamide;
<13> N (2-((2-(dimethylamino)ethyl)(methyl)amino)-5-((5-fluoro-4-((2-(propylsulfonamido)phenyl)amino)pyrimidin-2-yl)amino)-4-methoxyphenyl)acrylamide;
<14> N (2-((2-(dimethylamino)ethyl)(methyl)amino)-5-((5-fluoro-4-((2-(phenylsulfonamido)phenyl)amino)pyrimidin-2-yl)amino)-4-methoxyphenyl)acrylamide;
<15> N (2-((2-(dimethylamino)ethyl)(methyl)amino)-5-((5-fluoro-4-((2-((4-methylphenyl)sulfonamido)phenyl)amino)pyrimidin-2-yl)amino)-4-methoxyphenyl)acrylamide;
<16> *N-*(2-((2-(dimethylamino)ethyl)(methyl)amino)-4-methoxy-5-((5-methyl-4-((2-(methylsulfonamido)phenyl)amino)pyrimidin-2-yl)amino)phenyl)acrylamide;
<17> *N*-(2-((2-(dimethylamino)ethyl)(methyl)amino)-4-methoxy-5-((5-methyl-4-((2-((4-methylphenyl)sulfonamido)phenyl)amino)pyrimidin-2-yl)amino)phenyl)acrylamide;
<18> *N*-(2-((2-(dimethylamino)ethyl)(methyl)amino)-4-methoxy-5-((5-methyl-4-((2-(propylsulfonamido)phenyl)amino)pyrimidin-2-yl)amino)phenyl)acrylamide;
<19> *N*-(2-((2-(dimethylamino)ethyl)(methyl)amino)-5-((4-((2-(ethylsulfonamido)phenyl)amino)-5-methylpyrimidin-2-yl)amino)-4-methoxyphenyl)acrylamide;
<20> *N*-(2-((2-(dimethylamino)ethyl)(methyl)amino)-4-methoxy-5-((5-methyl-4-((2-(phenylsulfonamido)phenyl)amino)pyrimidin-2-yl)amino)phenyl)acrylamide;
<21> N (5-((5-chloro-4-((2-(methylsulfonamido)phenyl)amino)pyrimidin-2-yl)amino)-4-methoxy-2-(methyl(2-(pyrrolidin-1-yl)ethyl)amino)phenyl)acrylamide;
<22> N (5-((5-chloro-4-((2-(methylsulfonamido)phenyl)amino)pyrimidin-2-yl)amino)-4-methoxy-2-(2-(pyrrolidin-1-yl)ethoxy)phenyl)acrylamide;
<23> N (5-((5-chloro-4-((2-(methylsulfonamido)phenyl)amino)pyrimidin-2-yl)amino)-4-methoxy-2-(methyl(2-(piperidin-1-yl)ethyl)amino)phenyl)acrylamide;
<24> N (5-((5-chloro-4-((2-(methylsulfonamido)phenyl)amino)pyrimidin-2-yl)amino)-2-((2-(isopropylamino)ethyl)(methyl)amino)-4-methoxyphenyl)acrylamide;
<25> N (5-((5-chloro-4-((2-(methylsulfonamido)phenyl)amino)pyrimidin-2-yl)amino)-4-methoxy-2-(methyl(2-(methylamino)ethyl)amino)phenyl)acrylamide;
<26> (E)-N (5-((5-chloro-4-((2-(methylsulfonamido)phenyl)amino)pyrimidin-2-yl)amino)-4-methoxy-2-morpholinophenyl)-4-(dimethylamino)buten-2-amide;
<27> (E)-N (5-((5-chloro-4-((2-(methylsulfonamido)phenyl)amino)pyrimidin-2-yl)amino)-4-methoxy-2-(4-methylpiperazin-1-yl)phenyl)-4-(dimethylamino)buten-2-amide;
<28> (E)-N (5-((5-chloro-4-((2-(methylsulfonamido)phenyl)amino)pyrimidin-2-yl)amino)-2-((2-(dimethylamino)ethyl)(methyl)amino)-4-methoxyphenyl)-4-(dimethylamino)buten-2-amide;
<29> N (5-((5-chloro-4-((2-(N methylmethylsulfonamido)phenyl)amino)pyrimidin-2-yl)amino)-2-((2-(dimethylamino)ethyl)(methyl)amino)-4-methoxyphenyl)acrylamide;
<30> N (5-((5-chloro-4-((2-(N methylethylsulfonamido)phenyl)amino)pyrimidin-2-yl)amino)-2-((2-(dimethylamino)ethyl)(methyl)amino)-4-methoxyphenyl)acrylamide;
<31> N (5-((5-chloro-4-((2-(N ethylmethylsulfonamido)phenyl)amino)pyrimidin-2-yl)amino)-2-((2-(dimethylamino)ethyl)(methyl)amino)-4-methoxyphenyl)acrylamide;
<32> N (5-((5-chloro-4-((2-(N isopropylmethylsulfonamido)phenyl)amino)pyrimidin-2-yl)amino)-2-((2-(dimethylamino)ethyl)(methyl)amino)-4-methoxyphenyl)acrylamide;
<33> N (5-((5-chloro-4-((2-(N ethylethylsulfonamido)phenyl)amino)pyrimidin-2-yl)amino)-2-((2-(dimethylamino)ethyl)(methyl)amino)-4-methoxyphenyl)acrylamide;
<34> N (5-((5-chloro-4-((3-chloro-2-(methylsulfonamido)phenyl)amino)pyrimidin-2-yl)amino)-2-((2-(dimethylamino)ethyl)(methyl)amino)-4-methoxyphenyl)acrylamide;
<35> N-(5-((5-chloro-4-((3-chloro-2-(N-methylmethylsulfonamido)phenyl)amino)pyrimidin-2-yl)amino)-2-((2-(dimethylamino)ethyl)(methyl)amino)-4-methoxyphenyl)acrylamide;
<36> N (5-((5-chloro-4-((3-methyl-2-(methylsulfonamido)phenyl)amino)pyrimidin-2-yl)amino)-2-((2-(dimethylamino)ethyl)(methyl)amino)-4-methoxyphenyl)acrylamide;
<37> *N*-(5-((5-chloro-4-((3-isopropyl-2-(*N-*methylmethylsulfonamido)phenyl)amino)pyrimidin-2-yl)amino)-2-((2-(dimethylamino)ethyl)(methyl)amino)-4-methoxyphenyl)acrylamide;
<38> N-(5-((5-chloro-4-((3-methyl-2-(N-methylmethylsulfonamido)phenyl)amino)pyrimidin-2-yl)amino)-2-((2-(dimethylamino)ethyl)(methyl)amino)-4-methoxyphenyl)acrylamide;
<39> N (5-((5-chloro-4-((2-(N ethylmethylsulfonamido)-3-methylphenyl)amino)pyrimidin-2-yl)amino)-2-((2-(dimethylamino)ethyl)(methyl)amino)-4-methoxyphenyl)acrylamide;
<40> N (5-((5-chloro-4-((3-ethyl-2-(methylsulfonamido)phenyl)amino)pyrimidin-2-yl)amino)-2-((2-(dimethylamino)ethyl)(methyl)amino)-4-methoxyphenyl)acrylamide;
<41> N-(5-((5-chloro-4-((3-ethyl-2-(N-methylmethylsulfonamido)phenyl)amino)pyrimidin-2-yl)amino)-2-((2-(dimethylamino)ethyl)(methyl)amino)-4-methoxyphenyl)acrylamide;
<42> N (5-((5-chloro-4-((3-ethyl-2-(ethylsulfonamido)phenyl)amino)pyrimidin-2-yl)amino)-2-((2-(dimethylamino)ethyl)(methyl)amino)-4-methoxyphenyl)acrylamide;
<43> N-(5-((5-chloro-4-((3,4-dimethyl-2-(N-methylmethylsulfonamido)phenyl)amino)pyrimidin-2-yl)amino)-2-((2-(dimethylamino)ethyl)(methyl)amino)-4-methoxyphenyl)acrylamide;
<44> *N*-(5-((5-chloro-4-((3,4-dimethyl-2-(methylsulfonamido)phenyl)amino)pyrimidin-2-yl)amino)-2-((2-(dimethylamino)ethyl)(methyl)amino)-4-methoxyphenyl)acrylamide;
<45> N-(5-((5-chloro-4-((4-methyl-2-(N-methylmethylsulfonamido)phenyl)amino)pyrimidin-2-yl)amino)-2-((2-(dimethylamino)ethyl)(methyl)amino)-4-methoxyphenyl)acrylamide;
<46> N (5-((5-chloro-4-((4-methyl-2-(methylsulfonamido)phenyl)amino)pyrimidin-2-yl)amino)-2-((2-(dimethylamino)ethyl)(methyl)amino)-4-methoxyphenyl)acrylamide;
<47> N (5-((5-chloro-4-((1-(methylsulfonyl)-1,2,3,4-tetrahydroquinolin-8-yl)amino)pyrimidin-2-yl)amino)-2-((2-(dimethylamino)ethyl)(methyl)amino)-4-methoxyphenyl)acrylamide;
<48> N (5-((5-chloro-4-((1-(methylsulfonyl)-1,2,3,4-tetrahydroquinolin-8-yl)amino)pyrimidin-2-yl)amino)-4-methoxy-2-(methyl(2-(methylamino)ethyl)amino)phenyl)acrylamide;
<49> *N*-(5-((5-chloro-4-((1-(methylsulfonyl)-1,2,3,4-tetrahydroquinolin-8-yl)amino)pyrimidin-2-yl)amino)-2-(4-(dimethylamino)piperidin-1-yl)-4-methoxyphenyl)acrylamide;
<50> *N*-(5-((5-chloro-4-((1-(methylsulfonyl)-1,2,3,4-tetrahydroquinolin-8-yl)amino)pyrimidin-2-yl)amino)-4-methoxy-2-(4-(4-methylpiperazin-1 -yl)piperidin-1 - yl)phenyl)acrylamide;
<51> methyl 2-((5-acrylamido-4-((2-(dimethylamino)ethyl)(methyl)amino)-2-methoxyphenyl)amino)-4-((1-(methylsulfonyl)-1,2,3,4-tetrahydroquinolin-8-yl)amino)pyrimidin-5-carboxylate;
<52> isopropyl 2-((5-acrylamido-4-((2-(dimethylamino)ethyl)(methyl)amino)-2-methoxyphenyl)amino)-4-((1-(methylsulfonyl)-1,2,3,4-tetrahydroquinolin-8-yl)amino)pyrimidin-5-carboxylate;
<53> *N*-(5-((5-chloro-4-((1-(methylsulfonyl)-1,2,3,4-tetrahydroquinolin-8-yl)amino)pyrimidin-2-yl)amino)-4-methoxy-2-(methyl(2-morpholinoethyl)amino)phenyl)acrylamide;
<54> *N*-(5-((5-chloro-4-((1-(methylsulfonyl)-1,2,3,4-tetrahydroquinolin-8-yl)amino)pyrimidin-2-yl)amino)-2-((2-(dimethylamino)ethyl)(methyl)amino)-4-methoxyphenyl)-2-fluoroacrylamide;
<55> N (5-((5-chloro-4-((1-(methylsulfonyl)indolin-7-yl)amino)pyrimidin-2-yl)amino)-2-((2-(dimethylamino)ethyl)(methyl)amino)-4-methoxyphenyl)acrylamide;
<56> *N*-(5-((5-chloro-4-((1-(ethylsulfonyl)indolin-7-yl)amino)pyrimidin-2-yl)amino)-2-((2-(dimethylamino)ethyl)(methyl)amino)-4-methoxyphenyl)acrylamide;
<57> *N*-(5-((5-chloro-4-((1-(methylsulfonyl)indolin-7-yl)amino)pyrimidin-2-yl)amino)-4-methoxy-2-(methyl(2-(methylamino)ethyl)amino)phenyl)acrylamide;
<58> *N*-(5-((5-chloro-4-((1-(methylsulfonyl)indolin-7-yl)amino)pyrimidin-2-yl)amino)-2-(4-(dimethylamino)piperidin-1-yl)-4-methoxyphenyl)acrylamide;
<59> *N*-(5-((5-chloro-4-((1-(methylsulfonyl)indolin-7-yl)amino)pyrimidin-2-yl)amino)-4-methoxy-2-(4-(4-methylpiperazin-1 -yl)piperidin-1 -yl)phenyl)acrylamide;
<60> methyl 2-((5-acrylamido-4-((2-(dimethylamino)ethyl)(methyl)amino)-2-methoxyphenyl)amino)-4-((1-(methylsulfonyl)indolin-7-yl)amino)pyrimidin-5-carboxylate;
<61> isopropyl 2-((5-acrylamido-4-((2-(dimethylamino)ethyl)(methyl)amino)-2-methoxyphenyl)amino)-4-((1-(methylsulfonyl)indolin-7-yl)amino)pyrimidin-5-carboxylate;
<62> *N*-(5-((5-cyano-4-((1-(methylsulfonyl)indolin-7-yl)amino)pyrimidin-2-yl)amino)-2-((2-(dimethylamino)ethyl)(methyl)amino)-4-methoxyphenyl)acrylamide;
<63> *N*-(5-((5-chloro-4-((1-(methylsulfonyl)indolin-7-yl)amino)pyrimidin-2-yl)amino)-2-(2-(dimethylamino)ethoxy)-4-methoxyphenyl)acrylamide;
<64> *N*-(5-((5-chloro-4-((1-(methylsulfonyl)indolin-7-yl)amino)pyrimidin-2-yl)amino)-4-methoxy-2-(methyl(2-morpholinoethyl)amino)phenyl)acrylamide;
<65> *N*-(2-((2-(dimethylamino)ethyl)(methyl)amino)-4-methoxy-5-((4-((1-(methylsulfonyl)indolin-7-yl)amino)-5-(trifluoromethyl)pyrimidin-2-yl)amino)phenyl)acrylamide;
<66> *N*-(5-((5-chloro-4-((1-(methylsulfonyl)indolin-7-yl)amino)pyrimidin-2-yl)amino)-2-((2-(dimethylamino)ethyl)(methyl)amino)-4-methoxyphenyl)-2-fluoroacrylamide;
<67> *N-*(5-((5-chloro-4-((1-(methylsulfonyl)-1H-indol-7-yl)amino)pyrimidin-2-yl)amino)-2-((2-(dimethylamino)ethyl)(methyl)amino)-4-methoxyphenyl)acrylamide;
<68> *N-*(5-((5-chloro-4-((5-(*N*-methylmethylsulfonamido)quinoxalin-6-yl)amino)pyrimidin-2-yl)amino)-2-((2-(dimethylamino)ethyl)(methyl)amino)-4-methoxyphenyl)acrylamide;
<69> *N*-(6-((5-chloro-2-((4-((2-(dimethylamino)ethyl)(methyl)amino)-2-methoxyphenyl)amino)pyrimidin-4-yl)amino)quinoxalin-5-yl)methanesulfonamide;
<70> *N*-(5-((5-chloro-4-((2-(1,1-dioxidoisothiazolidin-2-yl)phenyl)amino)pyrimidin-2-yl)amino)-2-((2-(dimethylamino)ethyl)(methyl)amino)-4-methoxyphenyl)acrylamide;
<71> *N*-(5-((5-chloro-4-((2-(1,1-dioxido-1,2-thiazinan-2-yl)phenyl)amino)pyrimidin-2-yl)amino)-2-((2-(dimethylamino)ethyl)(methyl)amino)-4-methoxyphenyl)acrylamide;
<72> *N*-(5-((5-chloro-4-((1-(methylsulfonyl)indolin-7-yl)amino)pyrimidin-2-yl)amino)-4-methoxy-2-(methyl(2-(piperidin-1-yl)ethyl)amino)phenyl)acrylamide;
<73> *N*-(5-((5-chloro-4-((1-(methylsulfonyl)indolin-7-yl)amino)pyrimidin-2-yl)amino)-4-methoxy-2-(methyl(2-(4-methylpiperazin-1-yl)ethyl)amino)phenyl)acrylamide;
<74> *N*-(5-((5-chloro-4-((1-(methylsulfonyl)indolin-7-yl)amino)pyrimidin-2-yl)amino)-4-methoxy-2-(methyl(2-(pyrrolidin-1-yl)ethyl)amino)phenyl)acrylamide;
<75> *N*-(5-((5-chloro-4-((1-(ethylsulfonyl)indolin-7-yl)amino)pyrimidin-2-yl)amino)-4-methoxy-2-(methyl(2-(pyrrolidin-1-yl)ethyl)amino)phenyl)acrylamide;
<76> *N*-(5-((5-chloro-4-((4-methyl-2-(*N-*methylmethylsulfonamido)phenyl)amino)pyrimidin-2-yl)amino)-4-methoxy-2-(methyl(2-(pyrrolidin-1-yl)ethyl)amino)phenyl)acrylamide;
<77> *N*-(5-((5-chloro-4-((1-(methylsulfonyl)indolin-7-yl)amino)pyrimidin-2-yl)amino)-4-methoxy-2-(2-morpholinoethoxy)phenyl)acrylamide;
<78> *N*-(5-((5-chloro-4-((1-(methylsulfonyl)indolin-7-yl)amino)pyrimidin-2-yl)amino)-4-methoxy-2-(2-(piperidin-1-yl)ethoxy)phenyl)acrylamide;
<79> *N*-(5-((5-chloro-4-((1-(methylsulfonyl)indolin-7-yl)amino)pyrimidin-2-yl)amino)-4-methoxy-2-(2-(pyrrolidin-1-yl)ethoxy)phenyl)acrylamide;
<80> *N*-(2-((2-(azetidin-1-yl)ethyl)(methyl)amino)-5-((5-chloro-4-((1-(methylsulfonyl)indolin-7-yl)amino)pyrimidin-2-yl)amino)-4-methoxyphenyl)acrylamide;
<81> *N*-(2-((2-(azetidin-1-yl)ethyl)(methyl)amino)-5-((5-chloro-4-((1-(ethylsulfonyl)indolin-7-yl)amino)pyrimidin-2-yl)amino)-4-methoxyphenyl)acrylamide;
<82> *N*-(2-((2-(azetidin-1-yl)ethyl)(methyl)amino)-5-((5-chloro-4-((4-methyl-2-(*N* methylmethylsulfonamido)phenyl)amino)pyrimidin-2-yl)amino)-4-methoxyphenyl)acrylamide;
<83> *N*-(5-((5-chloro-4-((3,4-dimethyl-2-(N-methylacetamido)phenyl)amino)pyrimidin-2-yl)amino)-2-((2-(dimethylamino)ethyl)(methyl)amino)-4-methoxyphenyl)acrylamide;
<84> *N*-(5-((4-((2-acetamido-3,4-dimethylphenyl)amino)-5-chloropyrimidin-2-yl)amino)-2-((2-(dimethylamino)ethyl)(methyl)amino)-4-methoxyphenyl)acrylamide;
<85> *N-*(5-((5-chloro-4-((3-methyl-2-(*N*-methylacetamido)phenyl)amino)pyrimidin-2-yl)amino)-2-((2-(dimethylamino)ethyl)(methyl)amino)-4-methoxyphenyl)acrylamide;
<86> *N*-(5-((4-((2-acetamido-3-methylphenyl)amino)-5-chloropyrimidin-2-yl)amino)-2-((2-(dimethylamino) ethyl)(methyl)amino)-4-methoxyphenyl)acrylamide;
<87> *N*-(5-((5-chloro-4-((4-methyl-2-(*N*-methylacetamido)phenyl)amino)pyrimidin-2-yl)amino)-2-((2-(dimethylamino)ethyl)(methyl)amino)-4-methoxyphenyl)acrylamide;
<88> *N-*(5-((4-((2-acetamido-4-methylphenyl)amino)-5-chloropyrimidin-2-yl)amino)-2-((2-(dimethylamino) ethyl)(methyl)amino)-4-methoxyphenyl)acrylamide;
<89> *N-*(5-((5-chloro-4-((2-(*N*-methylacetamido)phenyl)amino)pyrimidin-2-yl)amino)-2-((2-(dimethylamino) ethyl)(methyl)amino)-4-methoxyphenyl)acrylamide;
<90> *N-*(5-((4-((2-acetamidophenyl)amino)-5-chloropyrimidin-2-yl)amino)-2-((2-(dimethylamino) ethyl)(methyl)amino)-4-methoxyphenyl)acrylamide;
<91> *N*-(5-((4-((1-acetylindolin-7-yl)amino)-5-chloropyrimidin-2-yl)amino)-2-((2-(dimethylamino)ethyl)(methyl)amino)-4-methoxyphenyl)acrylamide;
<92> *N*-(5-((4-((1-acetylindolin-7-yl)amino)-5-chloropyrimidin-2-yl)amino)-4-methoxy-2-(methyl(2-(methylamino)ethyl)amino)phenyl)acrylamide;
<93> *N*-(5-((5-chloro-4-((1-propionylindolin-7-yl)amino)pyrimidin-2-yl)amino)-2-((2-(dimethylamino) ethyl)(methyl)amino)-4-methoxyphenyl)acrylamide;
<94> *N*-(5-((5-chloro-4-((1-propionyl-1,2,3,4-tetrahydroquinolin-8-yl)amino)pyrimidin-2-yl)amino)-2-((2-(dimethylamino)ethyl)(methyl)amino)-4-methoxyphenyl)acrylamide;
<95> *N-*(5-((4-((1-acetyl-1,2,3,4-tetrahydroquinolin-8-yl)amino)-5-chloropyrimidin-2-yl)amino)-2-((2-(dimethylamino)ethyl)(methyl)amino)-4-methoxyphenyl)acrylamide;
<96> *N*-(5-((5-chloro-4-((1-(methylsulfonyl)indolin-7-yl)amino)pyrimidin-2-yl)amino)-2-((2-(dimethylamino)ethyl)(methyl)amino)-4-methoxyphenyl)but-2-inamide;
<97> *N-*(5-((4-((1-acetylindolin-7-yl)amino)-5-chloropyrimidin-2-yl)amino)-2-((2-(dimethylamino) ethyl)(methyl)amino)-4-methoxyphenyl) but-2-inamide;
<98> *N*-(5-((5-chloro-4-((4-methyl-2-(*N-*methylmethylsulfonamido)phenyl)amino)pyrimidin-2-yl)amino)-2-((2-(dimethylamino)ethyl)(methyl)amino)-4-methoxyphenyl) but-2-inamide;
<99> *N*-(5-((4-((1-acetylindolin-7-yl)amino)-5-cyanopyrimidin-2-yl)amino)-2-((2-(dimethylamino) ethyl)(methyl)amino)-4-methoxyphenyl)acrylamide;
<100> *N*-(5-((4-((1-acetylindolin-7-yl)amino)pyrimidin-2-yl)amino)-2-((2-(dimethylamino)ethyl) (methyl)amino)-4-methoxyphenyl)acrylamide; and
<101> *N*-(5 -((4-((1 -acetylindolin-7-yl)amino)-5 -fluoropyrimidin-2-yl)amino)-2-((2-(dimethylamino)ethyl) (methyl)amino)-4-methoxyphenyl)acrylamide.

In the present invention, alkyl refers to a saturated hydrocarbon and, unless specifically limited, includes all linear chain or branched chain saturated hydrocarbons. Branched chain saturated hydrocarbons apply to saturated hydrocarbons with 3 hydrocarbons or more. Examples of alkyl include methyl, ethyl, propyl, isopropyl, butyl, isobutyl, t-butyl, pentyl, hexyl, *etc.*

In the present invention, cycloalkyl, which is a hydrocarbon compound with a ring structure, refers to a hydrocarbon compound with a fully saturated ring structure, unless otherwise specified. Examples include cyclopropyl, cyclobutyl, cyclopentyl, cyclohexal, *etc.*

In the present invention, heterocycloalkyl means that in cycloalkyl, one or more carbons forming a ring are substituted with a heteroatom, for example, a heteroatom selected from N, O, S, P, *etc.,* and one or more carbons may be substituted with heteroatoms, and one or more carbons may be substituted with the same or different heteroatoms. Examples include aziridine, oxirane, thiirane, azetidine, oxetane, thietane, diazetidine, pyrrolidine, tetrahydrofuran, tetrahydrothiophene, pyrazolidine, dioxolane, oxathiolane, piperidine, piperazine, morpholine, tetrahydropyran, dioxane, *etc.* Heterocycloalkyl includes ring compounds mono-, bi-, and tri rings, and may include compounds with bridged or fused multiple rings.

The compound represented by Formula 1 above of the present invention may be used in the form of a pharmaceutically acceptable salt, and as a salt, an acid addition salt formed by a pharmaceutically acceptable free acid is useful. The acid addition salt is obtained from inorganic acids *(e.g.,* hydrochloric acid, nitric acid, phosphoric acid, sulfuric acid, hydrobromic acid, hydroiodic acid, nitrous acid, phosphorous acid, *etc.),* non-toxic organic acids *(e.g.,* aliphatic mono- and dicarboxylates, phenyl-substituted alkanoates, hydroxy alkanoates and alkanedioates, aromatic acids, aliphatic and aromatic sulfonic acids, *etc.),* and organic acids *(e.g.,* trifluoroacetic acid, acetate, benzoic acid, citric acid, lactic acid, maleic acid, gluconic acid, methanesulfonic acid, 4-toluenesulfonic acid, tartaric acid, fumaric acid, *etc.).* These types of pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitro benzoate, hydroxybenzoate, methoxybenzoate, phthalate, terephthalate, benzenesulfonate, toluenesulfonate, chlorobenzenesulfonate, xylenesulfonate, phenyl acetate, phenyl propionate, phenyl butyrate, citrate, lactate, β-hydroxybutyrate, glycolate, malate, tartrate, methane sulfonate, propane sulfonate, naphthalene-1-sulfonate, naphthalene-2-sulfonate, mandelate, *etc.*

The acid addition salt according to the present invention may be prepared by conventional methods, for example, may be prepared by dissolving the derivative of Formula 1 in an organic solvent *(e.g.,* methanol, ethanol, acetone, methylene chloride, acetonitrile, *etc.),* adding an organic or inorganic acid, and filtering and drying the resulting precipitate, or may be prepared by distilling a solvent and an excess amount of an acid under reduced pressure, drying the resultant, and crystallizing the resultant in an organic solvent.

Additionally, a pharmaceutically acceptable metal salt may be prepared using a base. An alkali metal or alkaline earth metal salt may be prepared, for example, by dissolving the compound in an excess amount of alkali metal hydroxide or alkaline earth metal hydroxide solution, filtering insoluble compound salts, and evaporating and drying the filtrate. In this case, it is pharmaceutically appropriate to prepare sodium, potassium, or calcium salts as metal salts. Additionally, the corresponding salts are obtained by reacting an alkali metal or alkaline earth metal salt with a suitable negative salt *(e.g.,* silver nitrate).

Furthermore, the present invention includes not only the compound represented by Formula 1 and pharmaceutically acceptable salts thereof, but also solvates, optical isomers, hydrates, *etc.* that may be prepared therefrom.

The term "hydrate" refers to a compound of the present invention containing a stoichiometric or non-stoichiometric amount of water bound by a non-covalent intermolecular forces or a salt thereof. The hydrate of the compound represented by Formula 1 of the present invention may contain a stoichiometric or non-stoichiometric amount of water bound by a non-covalent intermolecular force. The hydrate may contain water of 1 equivalent or more, preferably water of 1 to 5 equivalents. Such hydrates may be prepared by crystallizing the compound represented by Formula 1 of the present invention, an isomer thereof, or a pharmaceutically acceptable salt thereof from water or a solvent containing water.

The term "solvate" refers to a compound of the present invention or a salt thereof containing a stoichiometric or non-stoichiometric amount of a solvent bound by a non-covalent intermolecular force. Preferred solvents therefor include volatile, non-toxic, and/or suitable solvents for administration to humans.

The term "isomer" refers to a compound of the present invention or a salt thereof having the same chemical formula or molecular formula, but is structurally or sterically different. Such isomers include structural isomers even including tautomers, stereoisomers. Stereoisomers include all of optical isomers (enantiomers) and diastereomers that appear by having one or more asymmetric carbon centers, and geometric isomers *(trans, cis).* All these isomers and mixtures thereof are also included within the scope of the present invention.

In another aspect, the present invention provides a method for preparing a compound represented by Formula 1, which includes reacting a compound represented by Formula 2 with a compound represented by Formula 3 to prepare a compound represented by Formula 1 as represented by Reaction Scheme 1 below:

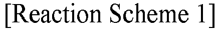

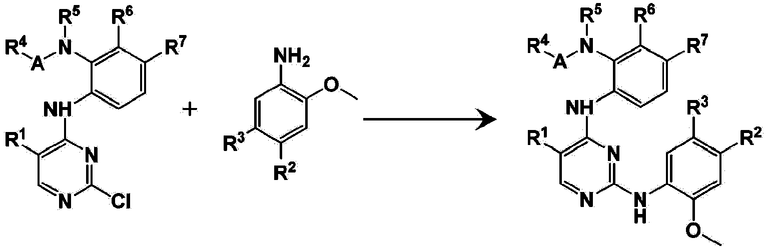

In Reaction Scheme 1 above, A, R¹, R², R³, R⁴, R⁵, R⁶, and R⁷ are as defined in Formula 1 above.

In the reaction scheme above, the reaction between Formula 2 and Formula 3 is a nucleophilic substitution reaction, the reaction conditions are performed under a normal organic solvent condition, includes organic solvents such as *n*-BuOH, tetrahydrofuran, DMF, DMSO, acetone, dichloromethane, and chloroform, and although the reaction temperature is not particularly limited, the reaction may be performed in the range of 0 °C to 100 °C, 10 °C to 50 °C, and may be performed at room temperature.

In another aspect, the present invention provides a pharmaceutical composition for the prevention or treatment of cancer including the compound represented by Formula 1 above, an isomer thereof, a solvate thereof, a hydrate thereof, or a pharmaceutically acceptable salt thereof as an active ingredient.

In particular, the cancer is one or more selected from the group consisting of pseudomyxoma, intrahepatic biliary tract cancer, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphocytic leukemia, basal cell cancer, ovarian epithelial cancer, ovarian germ cell cancer, male breast cancer, brain cancer, pituitary adenoma, multiple myeloma, gallbladder cancer, biliary tract cancer, colorectal cancer, chronic myeloid leukemia, chronic lymphocytic leukemia, retinoblastoma, choroidal melanoma, ampullary cancer of Vater, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small intestine cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, urethral cancer, cancer of unknown primary site, gastric lymphoma, stomach cancer, gastric carcinoid, gastrointestinal stromal cancer, Wilms cancer, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid, vaginal cancer, spinal cord cancer, acoustic neuroma, pancreatic cancer, salivary gland cancer, Kaposi's sarcoma, Paget's disease, tonsil cancer, squamous cell carcinoma, pulmonary adenocarcinoma, lung cancer, lung squamous cell carcinoma, skin cancer, anal cancer, rhabdomyosarcoma, laryngeal cancer, pleural cancer, blood cancer, and thymic cancer, and the cancer may be a cancer expressing mutations in one or more genes selected from the group consisting of EGFR, HER2, ALK, FAK, FLT3, JAK3, KIT, and PLK4, and in an embodiment, the cancer is one in which a mutation has expressed in relation to EGFR or HER2.

In addition, the compound, an isomer thereof, a solvate thereof, a hydrate thereof, or a pharmaceutically acceptable salt thereof may inhibit a mutation in epidermal growth factor receptor (EGFR), and through this inhibition, it has the activity of preventing, improving, or treating cancer, and in particular, the EGFR mutation may include EGFR del19, EGFR A763_Y764insFHEA, EGFR V769_D770insASV, EGFR D770_N771insSVD, Ba/F3 EGFR V769_D770insASV, Ba/F3 EGFR D770_N771insSVD, *etc.* In particular, the cancer is as described above.

In addition, the compound, an isomer thereof, a solvate thereof, a hydrate thereof, or a pharmaceutically acceptable salt thereof may inhibit a HER2 mutation, and through this inhibition, it has the activity of preventing, improving, or treating cancer, and in particular, the HER2 mutation may include HER2 A775_G776insYVMA, Ba/F3 HER2 A775_G776insYVMA, Ba/F3 HER2 G776delinsVC, *etc.* In particular, the cancer is as described above.

The compound, an isomer thereof, a solvate thereof, a hydrate thereof, or a pharmaceutically acceptable salt thereof may have growth inhibitory activity against mutant cancer cells, which may be cells expressing EGFR mutations and/or HER2 mutations as described above, and in an embodiment, may be a cancer cell in which any one of EGFR V769_D770insASV, EGFR D770_N771insSVD, HER2 A775_G776insYVMA, HER2 G776delinsVC, *etc.* has expressed.

In addition, the pharmaceutical composition for the prevention or treatment of cancer including the compound represented by Formula 1 above, an isomer thereof, or a pharmaceutically acceptable salt thereof as an active ingredient may be administered as an individual treatment or used in combination with other anticancer drugs in use.

In addition, the pharmaceutical composition for the prevention or treatment of cancer including the compound represented by Formula 1 above, an isomer thereof, or a pharmaceutically acceptable salt thereof as an active ingredient may enhance the anticancer effect by administering the same in combination with an anticancer agent.

The compound represented by Formula 1 above or a pharmaceutically acceptable salt thereof may be administered in various oral and parenteral dosage forms during clinical administration. When formulated, it is prepared using diluents or excipients such as commonly used fillers, extenders, binders, humectants, disintegrants, and surfactants. Solid preparations for oral administration include tablets, pills, powders, granules, capsules, *etc.,* and these solid preparations are prepared by mixing one or more compounds with one or more excipients, such as starch, calcium carbonate, sucrose or lactose, and gelatin. In addition to simple excipients, lubricants such as magnesium stearate and talc are also used. Liquid preparations for oral administration include suspensions, liquid preparations for internal use, emulsions, and syrups, and various excipients such as humectants, sweeteners, fragrances, and preservatives may be included in addition to the commonly used simple diluents such as water and liquid paraffin. Preparations for parenteral administration include sterilized aqueous solutions, non-aqueous solvents, suspensions, and emulsions. Non-aqueous solvents and suspensions may include propylene glycol, polyethylene glycol, vegetable oil *(e.g.,* olive oil), and injectable esters (e.g., ethyl oleate).

The pharmaceutical composition including the compound represented by Formula 1 or a pharmaceutically acceptable salt thereof as an active ingredient may be administered parenterally, and the parenteral administration is performed by a method of subcutaneous injection, intravenous injection, intramuscular injection, or intrathoracic injection.

In particular, in order to prepare the formulation for parenteral administration, the compound represented by Formula 1 or a pharmaceutically acceptable salt thereof, the salt may be mixed in water with a stabilizer or buffer to prepare a solution or suspension, and may be prepared in ampoule or vial unit dosage form. The composition may be sterile and/or contain adjuvants (e.g., preservatives, stabilizers, wetting agents or emulsification accelerators, salts and/or buffers for adjusting osmotic pressure) and other therapeutically useful substances, and may be formulated according to conventional mixing, granulating, or coating methods.

Formulations for oral administration include, for example, tablets, pills, hard/soft capsules, solutions, suspensions, emulsifiers, syrups, granules, elixirs, troches, *etc.,* and these formulations contain diluents *(e.g.,* lactose, dextrose, sucrose, mannitol, sorbitol, cellulose, and/or glycine), lubricants *(e.g.,* silica, talc, stearic acid and magnesium or calcium salts thereof, and/or or polyethylene glycol) in addition to the active ingredients. Tablets may contain binders *(e.g.,* magnesium aluminum silicate, starch paste, gelatin, methylcellulose, sodium carboxymethylcellulose, and/or polyvinylpyrrolidine, *etc.)* and in some cases, may contain disintegrants *(e.g.,* starch, agar, alginic acid or its sodium salt, *etc.)* or boiling mixtures and/or absorbents, colorants, flavoring agents, and sweeteners.

Still another aspect of the present invention provides a health functional food for the prevention or improvement of cancer including the compound represented by Formula 1 above, an isomer thereof, a solvate thereof, a hydrate thereof, or a pharmaceutically acceptable salt thereof as an active ingredient. In this case, the cancer is as described above.

The compound represented by Formula 1 above according to the present invention may be added directly to food or used with other foods or food ingredients, and may appropriately be used according to conventional methods. The mixing amount of the active ingredient may appropriately be determined depending on the purpose of use (for prevention or improvement). In general, the amount of the compound in health food may be 0.1 parts by weight to 90 parts by weight of the total weight of the food. However, in the case of long-term intake for health and hygiene purposes or health control, the amount may be below the above range, and since there is no problem in terms of safety, the active ingredient may be used in amounts exceeding the above range.

Still another aspect of the present invention provides a method for preventing, improving, or treating cancer, which includes administering the compound represented by Formula 1 above, an isomer thereof, a solvate thereof, a hydrate thereof, or a pharmaceutically acceptable salt thereof to a subject in need thereof.

Still another aspect of the present invention provides a use or utilization of the compound represented by Formula 1 above, an isomer thereof, a solvate thereof, a hydrate thereof, or a pharmaceutically acceptable salt thereof in preventing, improving, or treating cancer.

The compound represented by Formula 1 above, a stereoisomer thereof, a solvate thereof, a hydrate thereof, or a pharmaceutically acceptable salt thereof or a pharmaceutical composition of the present invention is administered in a "pharmaceutically effective amount". In the present invention, the term "pharmaceutically effective amount" refers to an amount sufficient to treat a condition at a reasonable benefit/risk ratio applicable to medical treatment or improvement, and the effective dose level may be decided depending on factors including the type and severity of the subject, age, sex, activity of drug, sensitivity to drug, time of administration, route of administration and excretion rate, duration of treatment, concurrently-used drugs, and other factors well known in the medical field. For example, an effective amount of 0.001 mg/kg to 1,000 mg/kg, 0.01 mg/kg to 100 mg/kg, or 0.1 mg/kg to 20 mg/kg or 0.1 mg/kg to 500 mg/kg may be included. The amount of the pharmaceutical composition of the present invention may be selected and implemented by those skilled in the art within an appropriate range.

### Mode for Carrying Out the Invention

Hereinafter, the present invention will be described in detail by Examples and Experimental Examples.

However, the following Examples and Experimental Examples only illustrate the present invention, and the content of the present invention is not limited to the following Examples and Experimental Examples.

Hereinbelow, Preparation Examples for the preparation of the compounds in Examples are disclosed.

### <Preparation Examples 1 to 3> Prepared according to the scheme below.

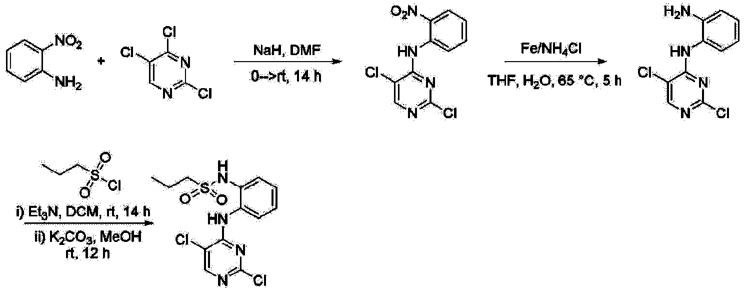

### <Preparation Example 1> Preparation of 2,5-dichloro-N-(2-nitrophenyl)pyrimidin-4-amine

NaH (3.47 g, 144.8 mmol) was added to a stirred solution of 2-nitroaniline (10 g, 72.4 mmol) dissolved in DMF (100 mL) at 0 °C and the mixture was stirred for 30 minutes. Then, 2,4,5-trichloropyrimidine (16.0 g 86.8 mmol) was added thereto at 0 °C. The resulting mixture was warmed to room temperature and maintained thereat for 14 hours. The reaction was monitored by TLC. The reaction mixture was cooled with cold water (300 mL) and stirred for 15 minutes. The precipitated solid was filtered and dried under reduced pressure to obtain the desired Preparation Example 1 as a yellow solid (17 g, 85 %).

¹H NMR (300 MHz, DMSO-*d*₆) δ10.23 (s, 1 H), 8.49 (s, 1 H), 8.10 (dd, *J* = 8.2, 1.4 Hz, 1 H), 7.91 - 7.71 (m, 2 H), 7.53 - 7.43 (m, 1 H).

### <Preparation Example 2> Preparation of N¹-(2,5-dichloropyrimidin-4-yl)benzen-1,2-diamine

Iron powder (16.6 g, 298 mmol) and NH₄Cl (15.9 g, 298.1 mmol) were added to a stirred solution of Preparation Example 1 (17 g, 59.6 mmol) dissolved in THF/H₂O = 1/1 (300 mL). The resulting mixture was warmed at 65 °C for 5 hours. The reaction was monitored by TLC. The reaction mixture was cooled to room temperature, filtered through celite and washed with ethyl acetate. The organic layer was separated, washed with brine, dried over Na₂SO₄, and evaporated under reduced pressure to obtain the desired product Preparation Example 2 as a yellow solid (10 g, 65%). LCMS: 256.8 [M+H⁺].

### <Preparation Example 3> Preparation of N (2-((2,5-dichloropyrimidin-4-yl)amino)phenyl)propan-1-sulfonamide

Et₃N (5 mL, 35.0 mmol) was added at room temperature to a stirred solution of Preparation Example 2 (3 g, 11.5 mmol) dissolved in DCM (50 mL). The reaction mixture was cooled to 0°C and 1-propanesulfonyl chloride (2.2 mL, 23.0 mmol) was added dropwise. The resulting mixture was warmed to room temperature and maintained thereat for 14 hours. The reaction was monitored by TLC. The reaction mixture was quenched with saturated NaHCO₃ (30 mL) and extracted with DCM (30 mL). The organic layer was washed with water and brine, dried over Na₂SO₄ and evaporated under reduced pressure. The obtained crude material was dissolved in methanol (50 mL), K₂CO₃ (2.0 g) was added thereto, and the mixture was stirred for 12 hours. The resulting mixture was evaporated under reduced pressure. The crude material obtained was diluted with H₂O (100 mL) and extracted with DCM (100 mL). The aqueous layer was separated, acidified with 1 N HCl (about pH 4 to pH 5) and extracted with DCM. The organic layer was separated, dried over Na₂SO₄, and concentrated under reduced pressure to obtain the desired product Preparation Example 3 as a pale red solid (2.5 g, 64%). LCMS: 361.2 [M+H⁺].

### <Preparation Examples 4 to 8> Prepared according to the scheme below.

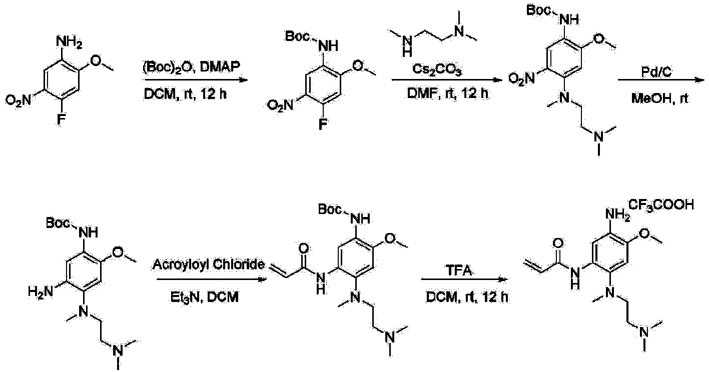

### <Preparation Example 4> Preparation of tert-butyl (4-fluoro-2-methoxy-5-nitrophenyl)carbamate

(Boc)₂O (5.8 g, 26.2 mmol) was added to a stirred solution of fluoro-2-methoxy-5-nitroaniline (4.5 g, 24.1 mmol) and DMAP (300 mg, 2.41 mmol) dissolved in DCM at 0 °C. The reaction mixture was slowly warmed to room temperature and maintained thereat for 12 hours. The reaction mixture was diluted with DCM and quenched with a saturated NaHCOs solution. The separated organic layer was washed with water and brine, dried over Na₂SO₄, and evaporated under reduced pressure. The obtained crude material was purified by column chromatography using hexane/ethyl acetate (7:3) as an eluent to obtain the desired product Preparation Example 4 as a yellow solid (4.0 g, 58%). LCMS: 287.2 [M+H⁺].

### <Preparation Example 5> Preparation of tert-butyl (4-((2-(dimethylamino)ethyl)(methyl)amino)-2-methoxy-5-nitrophenyl)carbamate

*N*¹,*N*¹,*N*²-trimethylethan-1,2-diamine (106 mg, 1.04 mmol) and cesium carbonate (183 mg, 1.56 mmol) were added at room temperature to a stirred solution of Preparation Example 4 (300 mg, 1.04 mmol) dissolved in DMF (5 mL). The resulting mixture was stirred at 70 °C overnight. The reaction mixture was cooled to room temperature and quenched with cold water. The precipitated solid was filtered and dried under reduced pressure to obtain the desired Preparation Example 5 as a yellow solid (250 mg, 65%). LCMS: 369.0 [M+H⁺].

### <Preparation Example 6> Preparation of tert-butyl (5-amino-4-((2-(dimethylamino)ethyl)(methyl)amino)-2-methoxyphenyl)carbamate

10% Pd/C (6.8 mg, 0.06 mmol) was added to a stirred solution of Preparation Example 5 (250 mg, 0.64 mmol) dissolved in methanol (15 mL) under argon atmosphere. The resulting mixture was stirred under 1 atm hydrogen atmosphere for 4 hours. The reaction mixture was filtered through celite and concentrated under reduced pressure to obtain the desired Preparation Example 6 as a brown solid (210 mg, 91%). LCMS: 339.2 [M+H⁺].

### <Preparation Example 7> Preparation of tert-butyl (5-acrylamido-4-((2-(dimethylamino)ethyl(methyl)amino)-2-methoxyphenyl)carbamate

Acryloyl chloride (0.047 ml, 0.59 mmol) was added to a stirred solution of Preparation Example 6 (200 mg, 0.59 mmol) and triethylamine (0.16 mL, 1.18 mmol) in DCM at -30°C. The reaction mixture was slowly warmed to room temperature and maintained thereat for 3 hours. The reaction mixture was diluted with DCM and cooled with a saturated NaHCOs solution. The organic layer was separated, washed with water and brine, dried over Na₂SO₄ and evaporated under reduced pressure. The obtained crude material was purified by column chromatography using DCM/MeOH (9.5:0.5) as an eluent to obtain the desired product Preparation Example 7 as an off-white solid (150 mg, 65%). LCMS: 393.0 [M+H⁺].

### <Preparation Example 8> Preparation of N-(5-amino-2-((2-(dimethylamino)ethyl)(methyl)amino)-4-methoxyphenyl)acrylamide trifluoroacetic acid salt

25% TFA dissolved in DCM (1 mL) was added at room temperature to a stirred solution of Preparation Example 7 (150.0 mg, 0.38 mmol) dissolved in DCM. The resulting mixture was stirred at room temperature for 12 hours. The reaction mixture was monitored by TLC. After completion, the crude material was concentrated under reduced pressure and moved to the next step without further purification. LCMS: 293.2 [M+H⁺].

### <Preparation Example 13> Preparation of N¹-(2-chloropyrimidin-4-yl)benzen-1,2-diamine

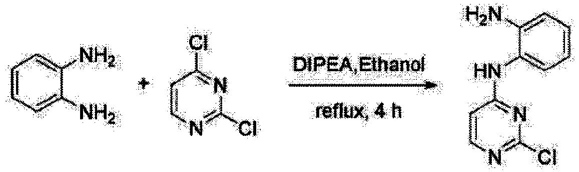

DIPEA (16.15 mL, 92.4 mmol) and 2,4-dichloropyrimidine (6.9 g, 46.2 mmol) were added at room temperature to a stirred solution of benzen-1,2-diamine (5.0 g, 46.2 mmol) dissolved in ethanol (100 mL). The resulting mixture was heated for 4 hours and monitored by TLC. The reaction mixture was cooled to room temperature and the precipitated solid was filtered, washed with ethanol, and dried under vacuum to obtain the desired pure compound N¹-(2-chloropyrimidin-4-yl)benzen-1,2-diamine as an off-white solid (80%). LCMS: 221.2 [M+H⁺].

### <Preparation Example 36> Preparation of N-(2-((2-((5-amino-2-methoxy-4-morpholinophenyl)amino)-5-chloropyrimidin-4-yl)amino)phenyl)methanesulfonamide

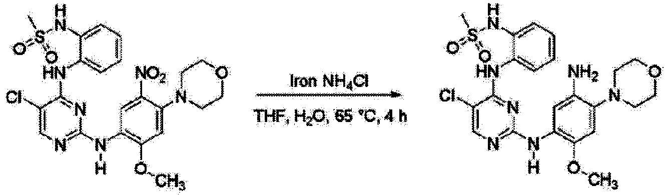

Iron powder (50.7 mg, 0.90 mmol) and NH₄Cl (48.6 mg, 0.90 mmol) were added to *N*-(2-((5-chloro-2-((2-methoxy-4-morpholino-5-nitrophenyl)amino)pyrimidin-4-yl)amino)phenyl)methanesulfonamide (100 mg, 0.18 mmol) dissolved in THF/H₂O [1:1](5 mL). The resulting mixture was heated to 65°C for 5 hours. The reaction mixture was cooled and filtered through a bed of celite. The filtrate was extracted with ethyl acetate. The organic layer was separated, washed with brine, dried over MgSO₄, and evaporated under reduced pressure to obtain the desired Preparation Example 36 as a pure off-white solid (85%). LCMS: 519.8 [M+H⁺].

Table A below summarizes Preparation Examples 1 to 82 prepared based on the Preparation Examples above.

**[Table A]**

| Prepar ation Examp le | Structure | Name | LCMS | Refer red-to Prepa ration Exam ple |
|---|---|---|---|---|
| 1 | 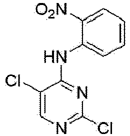 | 2,5-dichloro-*N*-(2-nitrophenyl)pyrimidin-4-amine | - | - |
| 2 | 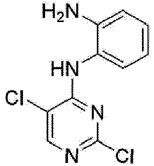 | N¹-(2,5-dichloropyrimidin-4-yl)benzen-1,2-diamine | 256.8 [M+H⁺] | - |
| 3 | 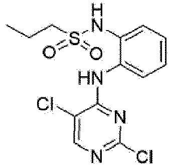 | *N*-(2-((2,5-dichloropyrimidin-4-yl)amino)phenyl)propan-1- sulfonamide | 361.2 [M+H⁺] | - |
| 4 | 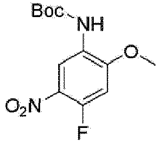 | *tert*-butyl (4-fluoro-2-methoxy-5-nitrophenyl)carbamate | 287.2 [M+H⁺] | - |
| 5 | 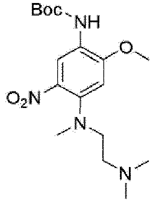 | *tert*-butyl (4-((2-(dimethylamino)ethyl)(methyl)amin o)-2-methoxy-5-nitrophenyl)carbamate | 369.0 [M+H⁺] | - |
| 6 | 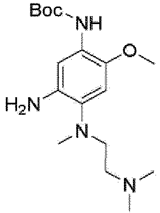 | *tert*-butyl (5-amino-4-((2-(dimethylamino)ethyl)(methyl)amin o)-2-methoxyphenyl)carbamate | 339.2 [M+H⁺] | - |
| 7 | 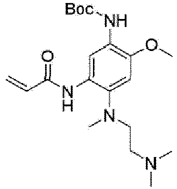 | *tert*-butyl (5-acrylamido-4-((2-(dimethylamino)ethyl(methyl)amin o)-2-methoxyphenyl)carbamate | 393.0 [M+H⁺] | - |
| 8 | 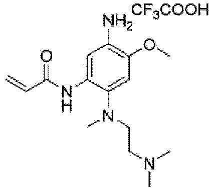 | *N*-(5-amino-2-((2-(dimethylamino)ethyl)(methyl)amin o)-4-methoxyphenyl)acrylamide trifluoroacetic acid salt | 293.2 [M+H⁺] | - |
| 9 | 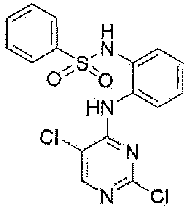 | *N*-(2-((2,5-dichloropyrimidin-4-yl)amino)phenyl)benzenesulfonami de | 396.0 [M+2H⁺] | 3 |
| 10 | 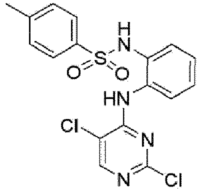 | *N*-(2-((2,5-dichloropyrimidin-4-yl)amino)phenyl)-4-methylbenzenesulfonamide | 410.0 [M+2H⁺] | 3 |
| 11 | 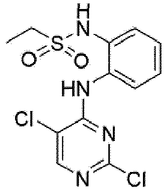 | *N*-(2-((2,5-dichloropyrimidin-4-yl)amino)phenyl)ethanesulfonamide | 348.2 [M+H⁺] | 3 |
| 12 | 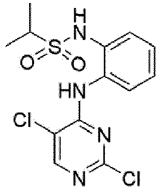 | *N*-(2-((2,5-dichloropyrimidin-4-yl)amino)phenyl)propan-2-sulfonamide | 362.0 [M+2H⁺] | 3 |
| 13 | 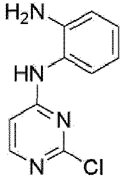 | N¹-(2-chloropyrimidin-4-yl)benzen-1,2-diamine | 221.2 [M+H⁺] | - |
| 14 | 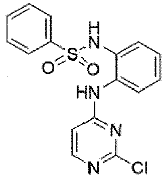 | *N*-(2-((2-chloropyrimidin-4-yl)amino)phenyl)benzenesulfonami de | 361.0 [M+H⁺] | 3 |
| 15 | 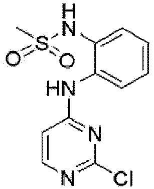 | *N*-(2-((2-chloropyrimidin-4-yl)amino)phenyl)methanesulfonami de | 299.0 [M+H⁺] | 3 |
| 16 | 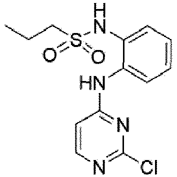 | *N*-(2-((2-chloropyrimidin-4-yl)amino)phenyl)propan-1- sulfonamide | 327.1 [M+H⁺] | 3 |
| 17 | 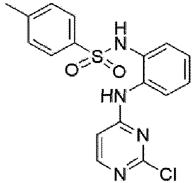 | *N*-(2-((2-chloropyrimidin-4-yl)amino)phenyl)-4-methylbenzenesulfonamide | 375.1 [M+H⁺] | 3 |
| 18 | 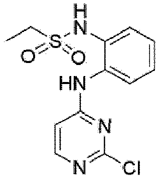 | *N-*(2-((2-chloropyrimidin-4-yl)amino)phenyl)ethanesulfonamide | 313.1 [M+H⁺] | 3 |
| 19 | 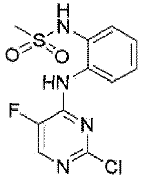 | *N-*(2-((2-chloro-5-fluoropyrimidin-4-yl)amino)phenyl)methanesulfonami de | 317.8 [M+H⁺] | 3 |
| 20 | 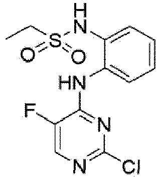 | *N*-(2-((2-chloro-5-fluoropyrimidin-4-yl)amino)phenyl)ethanesulfonamide | 331.0 [M+H⁺] | 3 |
| 21 | 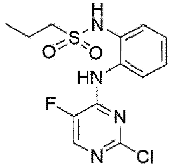 | *N*-(2-((2-chloro-5-fluoropyrimidin-4-yl)amino)phenyl)propan-1- sulfonamide | 345.1 [M+H⁺] | 3 |
| 22 | 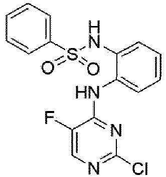 | *N*-(2-((2-chloro-5-fluoropyrimidin-4-yl)amino)phenyl)benzenesulfonami de | 679.1 [M+H⁺] | 3 |
| 23 | 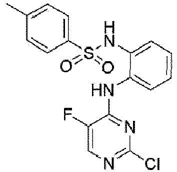 | *N-*(2-((2-chloro-5-fluoropyrimidin-4-yl)amino)phenyl)-4-methylbenzenesulfonamide | 393.1 [M+H⁺] | 3 |
| 24 | 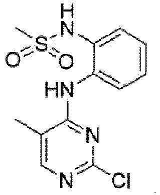 | *N*-(2-((2-chloro-5-methylpyrimidin-4-yl)amino)phenyl)methanesulfonami de | 313.8 [M+H⁺] | 3 |
| 25 | 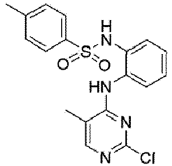 | *N-*(2-((2-chloro-5-methylpyrimidin-4-yl)amino)phenyl)-4-methylbenzenesulfonamide | 389.1 [M+H⁺] | 3 |
| 26 | 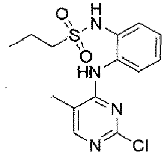 | *N*-(2-((2-chloro-5-methylpyrimidin-4-yl)amino)phenyl)propan-1- sulfonamide | 341.0 [M+H⁺] | 3 |
| 27 | 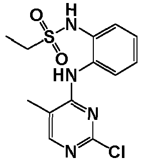 | *N*-(2-((2-chloro-5-methylpyrimidin-4-yl)amino)phenyl)ethanesulfonamide | 327.0 [M+H⁺] | 3 |
| 28 | 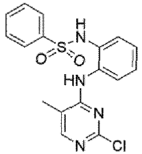 | *N*-(2-((2-chloro-5-methylpyrimidin-4-yl)amino)phenyl)benzenesulfonami de | 375.8 [M+H⁺] | 3 |
| 29 | 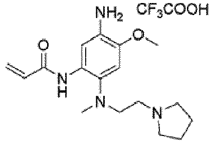 | *N*-(5-amino-4-methoxy-2-(methyl(2-(pyrrolidin-1- yl)ethyl)amino)phenyl)acrylamide trifluoroacetic acid salt | 319.0 [M+H⁺] | 8 |
| 30 | 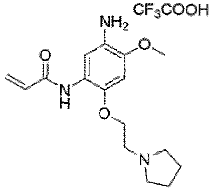 | *N*-(5-amino-4-methoxy-2-(2-(pyrrolidin-1-yl)ethoxy)phenyl)acrylamide trifluoroacetic acid salt | 306.2 [M+H⁺] | 8 |
| 31 | 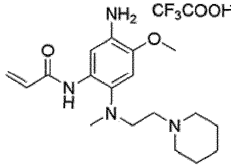 | *N-*(5-amino-4-methoxy-2-(methyl(2-(piperidin-1- yl)ethyl)amino)phenyl)acrylamide trifluoroacetic acid salt | 333.0 [M+H⁺] | 8 |
| 32 | 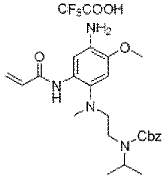 | benzyl (2-((2-acrylamido-4-amino-5-methoxyphenyl)(methyl)amino)ethy l)(isopropyl)carbamate | 441.0 [M+H⁺] | 8 |
| 33 | 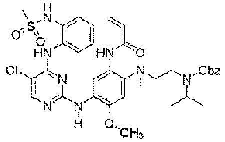 | benzyl (2-((2-acrylamido-4-((5-chloro-4-((2-(methylsulfonamido)phenyl)amino) pyrimidin-2-yl)amino)-5-methoxyphenyl)(methyl)amino)ethy l)(isopropyl)carbamate | 738.0 [M+H⁺] | 13 |
| 34 | 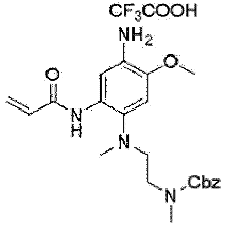 | benzyl (2-((2-acrylamido-4-amino-5-methoxyphenyl)(methyl)amino)ethy l)(methyl)carbamate | 413.0 [M+H⁺] | 8 |
| 35 | 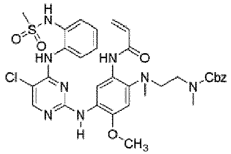 | benzyl (2-((2-acrylamido-4-((5-chloro-4-((2-(methylsulfonamido)phenyl)amino) pyrimidin-2-yl)amino)-5-methoxyphenyl)(methyl)amino)ethy l)(methyl)carbamate | 709.8 [M+H⁺] | 13 |
| 36 | 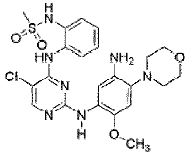 | *N*-(2-((2-((5-amino-2-methoxy-4-morpholinophenyl)amino)-5-chloropyrimidin-4-yl)amino)phenyl)methanesulfonami de | 519.8 [M+H⁺] | - |
| 37 | 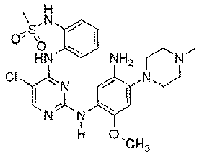 | *N-*(2-((2-((5-amino-2-methoxy-4-(4-methylpiperazin-1- yl)phenyl)amino)-5-chloropyrimidin-4-yl)amino)phenyl)methanesulfonami de | 534.0 [M+H⁺] | 36 |
| 38 | 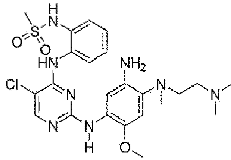 | *N*-(2-((2-((5-amino-4-((2-(dimethylamino)ethyl)(methyl)amin o)-2-methoxyphenyl)amino)-5-chloropyrimidin-4-yl)amino)phenyl)methanesulfonami de | 535.2 [M+H⁺] | 37 |
| 39 | 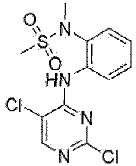 | *N*-(2-((2, 5-dichloropyrimidin-4-yl)amino)phenyl)-*N-*methylmethanesulfonamide | 347.1 [M+H⁺] | 13 |
| 40 | 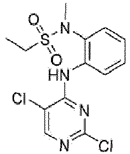 | *N*-(2-((2,5-dichloropyrimidin-4-yl)amino)phenyl)-*N* methylethanesulfonamide | 361.1 [M+H⁺] | 13 |
| 41 | 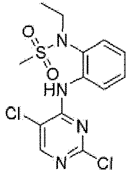 | *N*-(2-((2,S-dichloropyrimidin-4-yl)amino)phenyl)-*N-*ethylmethanesulfonamide | 361.1 [M+H⁺] | 13 |
| 42 | 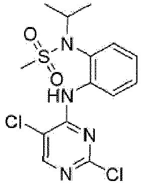 | *N*-(2-((2,S-dichloropyrimidin-4-yl)amino)phenyl)-*N-*isopropylmethanesulfonamide | 375.1 [M+H⁺] | 13 |
| 43 | 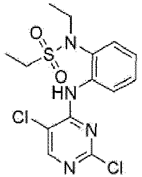 | *N*-(2-((2,S-dichloropyrimidin-4-yl)amino)phenyl)-*N-*ethylethanesulfonamide | 375.1 [M+H⁺] | 13 |
| 44 | 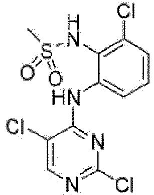 | N-(2-chloro-6-((2,5-dichloropyrimidin-4-yl)amino)phenyl)methanesulfonami de | 367.8 [M+2H⁺] | 13 |
| 45 | 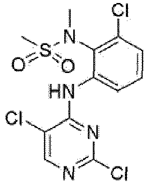 | *N*-(2-chloro-6-((2,5-dichloropyrimidin-4-yl)amino)phenyl)-*N-*methylmethanesulfonamide | 381.2 [M+H⁺] | 13 |
| 46 | 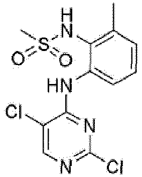 | *N*-(2-((2,5-dichloropyrimidin-4-yl)amino)-6-methylphenyl)methanesulfonamide | 348.8 [M+H⁺] | 3 |
| 47 | 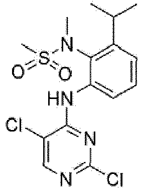 | *N*-(2-((2,5-dichloropyrimidin-4-yl)amino)-6-isopropylphenyl)-*N-*methylmethanesulfonamide | 389.8 [M+H⁺] | 13 |
| 48 | 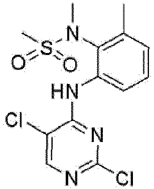 | N-(2-((2,5-dichloropyrimidin-4-yl)amino)-6-methylphenyl)-*N* methylmethanesulfonamide | 362.0 [M+H⁺] | 13 |
| 49 | 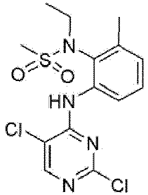 | *N*-(2-((2,5-dichloropyrimidin-4-yl)amino)-6-methylphenyl)-*N* ethylmethanesulfonamide | 376.0 [M+H⁺] | 13 |
| 50 | 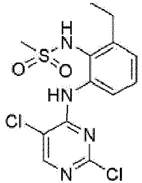 | *N*-(2-((2,5-dichloropyrimidin-4-yl)amino)-6-ethylphenyl)methanesulfonamide | 361.8 [M+H⁺] | 13 |
| 51 | 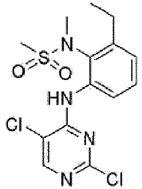 | N-(2-((2,5-dichloropyrimidin-4-yl)amino)-6-ethylphenyl)-*N-*methylmethanesulfonamide | 376.0 [M+H⁺] | 13 |
| 52 | 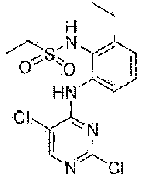 | *N*-(2-((2,5-dichloropyrimidin-4-yl)amino)-6-ethylphenyl)ethanesulfonamide | 376.0 [M+2H⁺] | 13 |
| 53 | 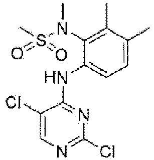 | *N*-(6-((2,5-dichloropyrimidin-4-yl)amino)-2,3-dimethylphenyl)-*N* methylmethanesulfonamide | 376.0 [M+H⁺] | 13 |
| 54 | 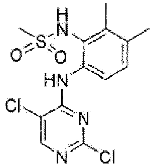 | *N*-(6-((2,5-dichloropyrimidin-4-yl)amino)-2,3-dimethylphenyl)methanesulfonamid e | 361.8 [M+H⁺] | 13 |
| 55 | 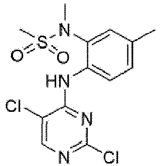 | *N*-(2-((2,5-dichloropyrimidin-4-yl)amino)-5-methylphenyl)-*N-*methylmethanesulfonamide | 361.1 [M+H⁺] | 13 |
| 56 | 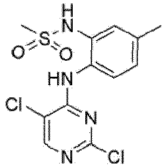 | *N*-(2-((2,5-dichloropyrimidin-4-yl)amino)-5-methylphenyl)methanesulfonamide | 348.0 [M+H⁺] | 13 |
| 57 | 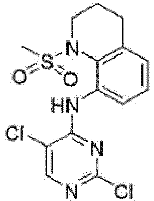 | *N*-(2,5-dichloropyrimidin-4-yl)-1-(methylsulfonyl)-1,2,3,4-tetrahydroquinolin-8-amine | 374.0 [M+H⁺] | 13 |
| 58 | 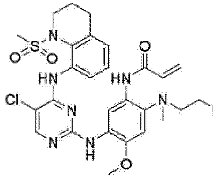 1 | *tert*-butyl (2-((2-acrylamido-4-((5-chloro-4-((1-(methylsulfonyl)-1,2,3,4-tetrahydroquinolin-8-yl)amino)pyrimidin-2-yl)amino)-5-methoxyphenyl)(methyl)amino)ethy l)(methyl)carbamate | 715.2 [M+H⁺] | 7 |
| 59 | 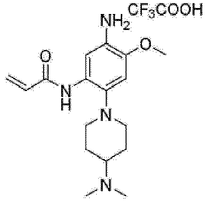 | *N*-(5-amino-2-(4-(dimethylamino)piperidin-1-yl)-4-methoxyphenyl)acrylamide trifluoroacetic acid salt | 319.0 [M+H⁺] | 8 |
| 60 | 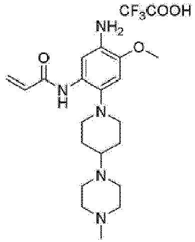 | *N*-(5-amino-4-methoxy-2-(4-(4-methylpiperazin-1-yl)piperidin-1- yl)phenyl)acrylamide trifluoroacetic acid salt | | 8 |
| 61 | 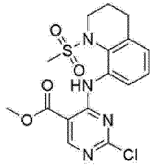 | methyl 2-chloro-4-((1-(methylsulfonyl)-1,2,3,4-tetrahydroquinolin-8-yl)amino)pyrimidin-5-carboxylate | 397.2 [M+H⁺] | 13 |
| 62 | 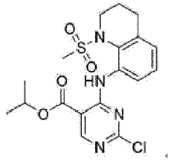 | isopropyl 2-chloro-4-((1-(methylsulfonyl)-1,2,3,4-tetrahydroquinolin-8-yl)amino)pyrimidin-5-carboxylate | 425.1 [M+H⁺] | 13 |
| 63 | 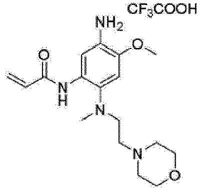 | -(5-amino-4-methoxy-2-(methyl(2-morpholinoethyl)amino)phenyl)acr ylamide | 335.2 [M+H⁺] | 8 |
| 64 | 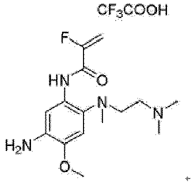 | *N*-(5-amino-2-((2-(dimethylamino)ethyl)(methyl)amin o)-4-methoxyphenyl)-2-fluoroacrylamide trifluoroacetic acid salt | 311.0 [M+H⁺] | 8 |
| 65 | 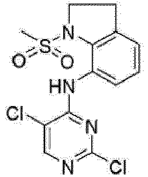 | *N*-(2,5-dichloropyrimidin-4-yl)-1-(methylsulfonyl)indolin-7-amine | 359.8 [M+H⁺] | 13 |
| 66 | 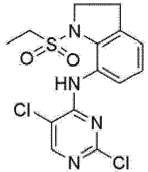 | *N*-(2,5-dichloropyrimidin-4-yl)-1-(ethylsulfonyl)indolin-7-amine | 374.0 [M+2H⁺] | 13 |
| 67 | 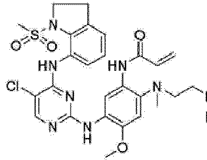 | *tert*-butyl (2-((2-acrylamido-4-((5-chloro-4-((1-(methylsulfonyl)indolin-7-yl)amino)pyrimidin-2-yl)amino)-5-methoxyphenyl)(methyl)amino)ethy l)(methyl)carbamate | 701.8 [M+H⁺] | 7 |
| 68 | 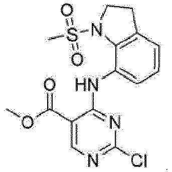 | methyl 2-chloro-4-((1-(methylsulfonyl)indolin-7-yl)amino)pyrimidin-5-carboxylate | 383.1 [M+H⁺] | 13 |
| 69 | 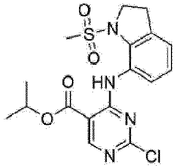 | isopropyl 2-chloro-4-((1-(methylsulfonyl)indolin-7-yl)amino)pyrimidin-5-carboxylate | 411.8 [M+H⁺] | 13 |
| 70 | 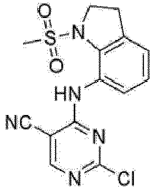 | 2-chloro-4-((1-(methylsulfonyl)indolin-7-yl)amino)pyrimidin-5-carbonitrile | 351.8 [M+H⁺] | 13 |
| 71 | 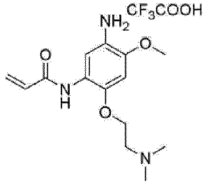 | *N*-(5-amino-2-(2-(dimethylamino)ethoxy)-4-methoxyphenyl)acrylamide trifluoroacetic acid salt | LCMS: 280 [M+H⁺] | 8 |
| 72 | 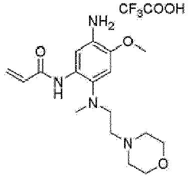 | tert-butyl (5-acrylamido-2-methoxy-4-(methyl(2-morpholinoethyl)amino)phenyl)car bamate | 335.2 [M+H⁺] | 8 |
| 73 | 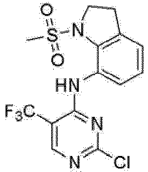 | *N*-(2-chloro-5-(trifluoromethyl)pyrimidin-4-yl)-1- (methylsulfonyl)indolin-7-amine | 394.0 [M+2H⁺] | 13 |
| 74 | 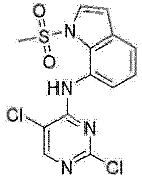 | *N*-(2,5-dichloropyrimidin-4-yl)-1-(methylsulfonyl)-1H-indol-7-amine | 358.8 [M+2H⁺] | 13 |
| 75 | 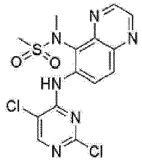 | *N*-(6-((2,5-dichloropyrimidin-4-yl)amino)quinoxalin-5-yl)-*N-*methylmethanesulfonamide | 399.1 [M+H⁺] | 13 |
| 76 | 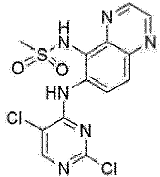 | N-(6-((2,5-dichloropyrimidin-4-yl)amino)quinoxalin-5-yl)methanesulfonamide | 385.8 [M+H⁺] | 13 |
| 77 | 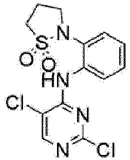 | 2-(2-((2,5-dichloropyrimidin-4-yl)amino)phenyl) isothiazolidine 1,1-dioxide | 359.8 [M+H⁺] | 13 |
| 78 | 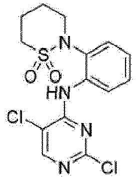 | 2-(2-((2,5-dichloropyrimidin-4-yl)amino)phenyl)-1,2-thiazinane 1,1-dioxide | 374.0 [M+2H⁺] | 13 |
| 79 | 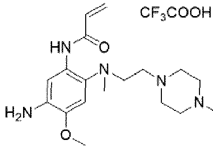 | *N*-(5-amino-4-methoxy-2-(methyl(2-(4-methylpiperazin-1-yl)ethyl)amino)phenyl)acrylamide trifluoroacetic acid salt | 348.5 [M+H⁺] | 8 |
| 80 | 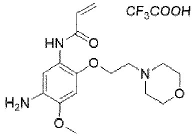 | *N*-(5-amino-4-methoxy-2-(2-morpholinoethoxy)phenyl)acrylami de trifluoroacetic acid salt | 322.4 [M+H⁺] | 8 |
| 81 | 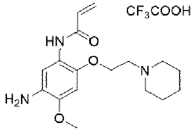 | *N*-(5-amino-4-methoxy-2-(2-(piperidin-1-yl)ethoxy)phenyl)acrylamide trifluoroacetic acid salt | 320.0 [M+H⁺] | 8 |
| 82 | 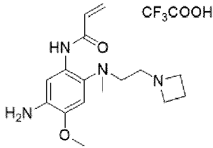 | -(5-amino-2-((2-(azetidin-1-yl)ethyl)(methyl)amino)-4-methoxyphenyl)acrylamide trifluoroacetic acid salt | 305.2 [M+H⁺] | 8 |

### <Preparation Examples 83 to 86> Prepared according to the scheme below.

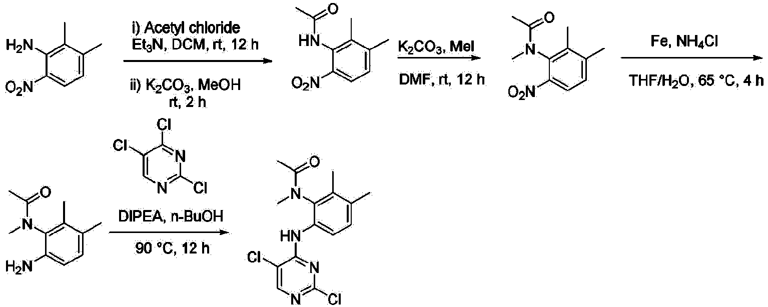

### <Preparation Example 83> Preparation of N-(2,3-dimethyl-6-nitrophenyl)acetamide

NaH (0.8 g, 36.3 mmol) and acetic anhydride (1.7 mL, 18.1 mmol) were slowly added at 0°C to a stirred solution of 2,3-dimethyl-6-nitroaniline (2.0 g, 12.1 mmol) in DMF. The resulting mixture was warmed to room temperature and stirred for 3 hours. The reaction mixture was immersed in ice water, and the precipitated solid was filtered, washed with water, trituration with diethyl ether, and dried to obtain Preparation Example 83. LCMS: 208.4 [M+H⁺].

### <Preparation Example 84> Preparation of N-(2,3-dimethyl-6-nitrophenyl)-N-methylacetamide

Methyl iodide (0.9 mL, 14.0 mmol) and potassium carbonate (580.0 mg, 4.20 mmol) were added at 0°C to a stirred solution of Preparation Example 83 (290 mg, 1.40 mmol) dissolved in DMF. The resulting mixture was warmed to room temperature and stirred for 4 hours. The reaction mixture was immersed in water and extracted with ethyl acetate. The mixed extracted organic layer was washed with water and brine, dried over MgSO₄, and evaporated under reduced pressure to obtain Preparation Example 84. LCMS: 222.3 [M+H⁺].

### <Preparation Example 85> Preparation of N-(6-amino-2,3-dimethylphenyl)-N-methylacetamide

Iron powder (1.3 g, 24.2 mmol) and NH₄Cl (1.3 g, 24.2 mmol) were added at room temperature to a stirred solution of Preparation Example 84 (1.08 g, 4.84 mmol) dissolved in 20 mL of THF/H₂O (1:1). The resulting mixture was heated to 65°C and stirred for 14 hours. The reaction mixture was filtered through concentrated celite under reduced pressure. The obtained material was diluted with ethyl acetate and washed with water. The organic phase was separated, washed with brine, dried over MgSO₄, filtered and concentrated under reduced pressure to obtain Preparation Example 85. LCMS: 192.4 [M+H⁺].

### <Preparation Example 86> Preparation of N-(6-((2,5-dichloropyrimidin-4-yl)amino)-2,3-dimethylphenyl)-N-methylacetamide

Diisopropylethylamine (1.9 mL, 11.4 mmol) and 2,4,5-trichloropyrimidine (1.2 g, 6.82 mmol) were added at room temperature to a stirred solution of Preparation Example 85 (1.00 g, 5.67 mmol) dissolved in n-BuOH (5 mL). The resulting mixture was heated to 90°C for 14 hours. The reaction mixture was cooled to room temperature and concentrated under reduced pressure. The obtained material was filtered and washed with n-BuOH to obtain Preparation Example 86. LCMS: 340.0 [M+H⁺].

### <Preparation Examples 94 to 96> Prepared according to the scheme below.

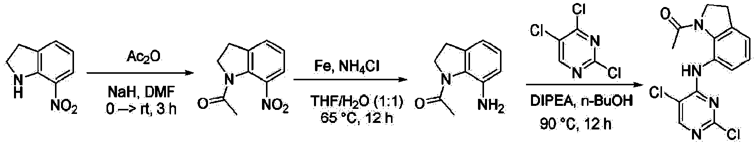

### <Preparation Example 94> Preparation of 1-(7-nitroindolin-1-yl)ethan-1-on

NaH (0.8 g, 36.3 mmol) and acetic anhydride (1.7 mL, 18.1 mmol) were slowly added at 0°C to a stirred solution of 7-nitroindoline (2.0 g, 12.1 mmol) dissolved in DMF. The resulting mixture was heated at room temperature and stirred for 3 hours. The reaction mixture was cooled in ice water, and the precipitated solid was filtered, washed with water, and purified with diethyl ether to obtain Preparation Example 94 as a yellow solid (71%). LCMS: 207.2 [M+H⁺].

### <Preparation Example 95> Preparation of 1-(7-aminoindolin-1-yl)ethan-1-on

Iron powder (1.3 g, 24.24 mmol) and NH₄Cl (1.3 g, 24.24 mmol) were added at room temperature to a stirred solution of Preparation Example 94 (1.0 g, 4.84 mmol) dissolved in 20 ml of THF/H₂O (1:1). The resulting mixture was heated to 65°C and stirred for 14 hours. The reaction mixture was filtered through concentrated celite under reduced pressure. The obtained material was diluted with ethyl acetate and washed with water. The organic phase was separated, washed with brine, dried over MgSO₄, and concentrated under reduced pressure to obtain Preparation Example 95 as a brown solid (90%). LCMS: 177.8 [M+H⁺].

### <Preparation Example 96> Preparation of 1-(7-((2,5-dichloropyrimidin-4-yl)amino)indolin-1-yl)ethan-1-on

Diisopropylethylamine (1.9 mL, 11.35 mmol) and 2,4,5-trichloropyrimidine (1.2 g, 6.82 mmol) were added at room temperature to a stirred solution of Preparation Example 95 (1.0 g, 5.67 mmol) dissolved in n-BuOH (5 mL). The resulting mixture was heated to 90°C for 14 hours. The reaction mixture was cooled to room temperature and concentrated under reduced pressure. The obtained material was filtered and washed with n-BuOH to obtain Preparation Example 96 as a dark green solid (80%). LCMS: 324.8 [M+H⁺].

Table B below summarizes Preparation Examples 83 to 104 prepared based on the Preparation Examples above.

**[Table B]**

| Prepar ation Examp le | Structure | Name | LCMS | Referre d-to Prepar ation Examp le |
|---|---|---|---|---|
| 83 | 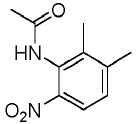 | *N*-(2,3-dimethyl-6-nitrophenyl)acetamide | 208.4 [M+H⁺] | - |
| 84 | 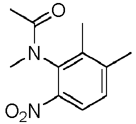 | *N*-(2,3-dimethyl-6-nitrophenyl)-N methylacetamide | 222.3 [M+H⁺] | - |
| 85 | 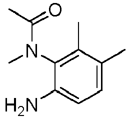 | *N*-(6-amino-2,3-dimethylphenyl)-*N*-methylacetamide | 192.4 [M+H⁺] | - |
| 86 | 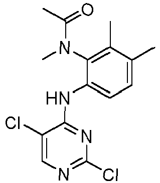 | *N*-(6-((2,5-dichloropyrimidin-4-yl)amino)-2,3-dimethylphenyl)-*N-*methylacetamide | 340.0 [M+H⁺] | - |
| 87 | 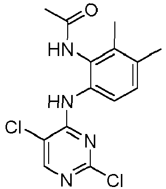 | *N*-(6-((2,5-dichloropyrimidin-4-yl)amino)-2,3-dimethylphenyl)acetamide | 325.8 [M+H⁺] | 86 |
| 88 | 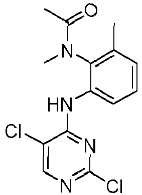 | *N*-(2-((2,5-dichloropyrimidin-4-yl)amino)-6-methylphenyl)-*N* methylacetamide | 326.8 [M+H⁺] | 86 |
| 89 | 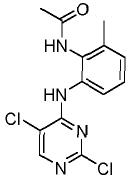 | *N*-(2-((2,5-dichloropyrimidin-4-yl)amino)-6-methylphenyl)acetamide | 311.4 [M+H⁺] | 86 |
| 90 | 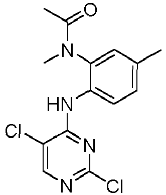 | *N*-(2-((2,5-dichloropyrimidin-4-yl)amino)-S-methylphenyl)-N-methylacetamide | 325.8 [M+H⁺] | 86 |
| 91 | 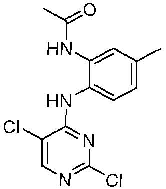 | *N*-(2-((2,5-dichloropyrimidin-4-yl)amino)-5-methylphenyl)acetamide | 311.8 [M+H⁺] | 86 |
| 92 | 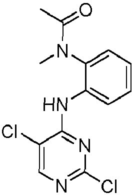 | *N*-(2-((2,S-dichloropyrimidin-4-yl)amino)phenyl)-N-methylacetamide | 311.2 [M+H⁺] | 86 |
| 93 | 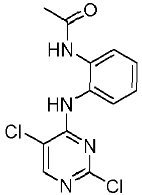 | *N*-(2-((2,5-dichloropyrimidin-4-yl)amino)phenyl)acetamide | 297.2 [M+H⁺] | 86 |
| 94 | 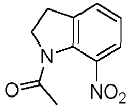 | 1-(7-nitroindolin-1-yl)ethan-1-on | 207.2 [M+H⁺] | - |
| 95 | 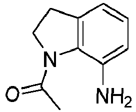 | 1-(7-aminoindolin-1-yl)ethan-1- on | 177.8 [M+H⁺] | - |
| 96 | 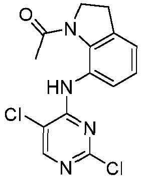 | 1-(7-((2,5-dichloropyrimidin-4-yl)amino)indolin-1-yl)ethan-1-on | 324.8 [M+H⁺] | - |
| 97 | 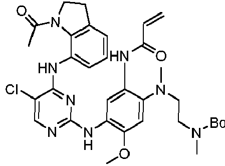 | *tert*-butyl(2-((4-((4-((1-acetylindolin-7-yl)amino)-5-chloropyrimidin-2-yl)amino)-2-acrylamido-5-methoxyphenyl)(methyl)amino)et hyl)(methyl)carbamate | 665.2 [M+H⁺] | 7 |
| 98 | 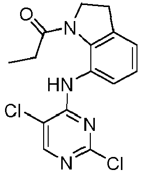 | 1-(7-((2,5-dichloropyrimidin-4-yl)amino)indolin-1-yl)propan-1- on | 337.8 [M+H⁺] | 96 |
| 99 | 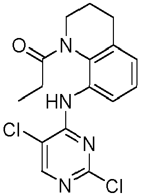 | 1-(8-((2,5-dichloropyrimidin-4-yl)amino)-3,4-dihydroquinolin- 1(2H)-yl)propan-1-on | 351.8 [M+H⁺] | 96 |
| 100 | 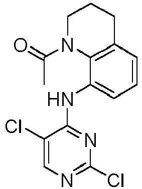 | 1-(8-((2,5-dichloropyrimidin-4-yl)amino)-3,4-dihydroquinolin-1(2H)-yl)ethan-1-on | 337.8 [M+H⁺] | 96 |
| 101 | 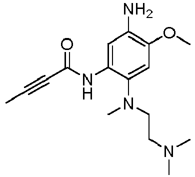 | *N*-(5-amino-2-((2-(dimethylamino)ethyl)(methyl)am ino)-4-methoxyphenyl)but-2-inamide | 304.8 [M+H⁺] | 8 |
| 102 | 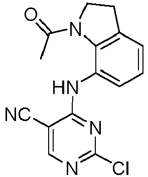 | 4-((1-acetylindolin-7-yl)amino)-2-chloropyrimidin-5-carbonitrile | 313.8 [M+H⁺] | 96 |
| 103 | 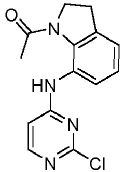 | 1-(7-((2-chloropyrimidin-4-yl)amino)indolin-1-yl)ethan-1-on | 288.8 [M+H⁺] | 96 |
| 104 | 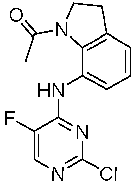 | 1-(7-((2-chloro-5-fluoropyrimidin-4-yl)amino)indolin-1-yl)ethan-1-on | 306.8 [M+H⁺] | 96 |

### < Preparation of Compounds of Examples>

### <Example 1> Preparation of N (5-((5-chloro-4-((2-(propylsulfonamido)phenyl)amino)pyrimidin-2-yl)amino)-2-((2-(dimethylamino)ethyl)(methyl)amino)-4-methoxyphenyl)acrylamide

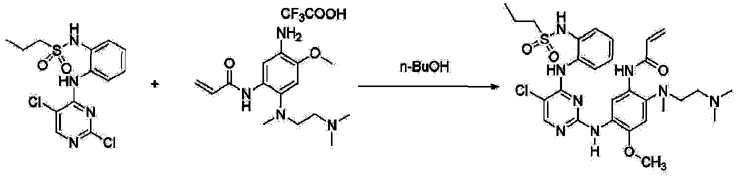

Preparation Example 8 (71.5 mg, 0.3 mmol) was added at room temperature to a stirred solution of Preparation Example 1 (100 mg, 0.3 mmol) in 1 N TFA dissolved in *n-*butanol (30 mL). The reaction tube was sealed with a Teflon-lined cap and the reaction mixture was stirred at 90°C overnight. The mixture was concentrated under reduced pressure. The residue was dissolved in DCM, washed with saturated NaHCO₃, water, and brine, dried over Na₂SO₄, and evaporated under reduced pressure. The obtained crude material was purified by column chromatography (DCM: MeOH/9: 1-8.5: 1.5) to obtain pure Example 1 as an off-white solid (31%).

### <Example 24> Preparation of N-(5-((5-chloro-4-((2-(methylsulfonamido)phenyl)amino)pyrimidin-2-yl)amino)-2-((2-(isopropylamino)ethyl)(methyl)amino)-4-methoxyphenyl)acrylamide

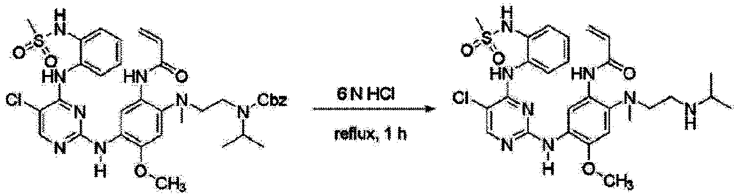

Preparation Example 33 (50.0 mg, 0.06 mmol) dissolved in 6 N HCl (1.0 mL) was heated under reflux for 1 hour. The resulting mixture was cooled to room temperature and concentrated under reduced pressure. The obtained crude material was purified by HPLC (0.1% TFA in H₂O and 0.1% TFA in ACN as buffer) to obtain the desired product Example 24 as an off-white solid (40%).

### <Example 26> Preparation of (E)-N-(5-((5-chloro-4-((2-(methylsulfonamido)phenyl)amino)pyrimidin-2-yl)amino)-4-methoxy-2-morpholinophenyl)-4-(dimethylamino)buten-2-amide

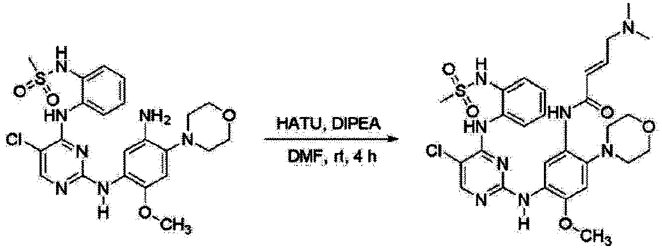

DIPEA (0.1 mL, 0.60 mmol), (2E)-4-(dimethylamino)but-2-enoic acid hydrochloride salt (38.2 mg, 0.23 mmol), and HATU (114.0 mg, 0.3 mmol) were added to a stirred solution of Preparation Example 36 (100 mg, 0.20 mmol) dissolved in DMF (5 mL). The resulting mixture was stirred for 14 hours. The reaction mixture was quenched with water and extracted with ethyl acetate. The organic layer was separated, washed with water and brine, dried over MgSO₄, and evaporated under reduced pressure. The crude material obtained was purified via column chromatography (5-9% MeOH in DCM as an eluent) to obtain pure Example 26 as an off-white solid (35%).

### <Example 48> Preparation of N (5-((5-chloro-4-((1-(methylsulfonyl)-1,2,3,4-tetrahydroquinolin-8-yl)amino)pyrimidin-2-yl)amino)-4-methoxy-2-(methyl(2-(methylamino)ethyl)amino)phenyl)acrylamide

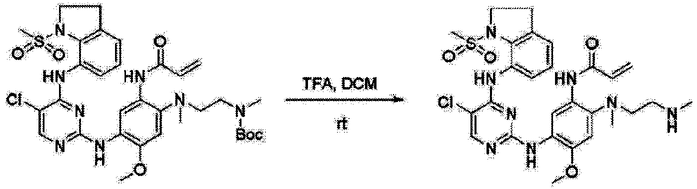

TFA (0.1 mL) was added at room temperature to Preparation Example 58 (50 mg, 0.07 mmol) dissolved in DCM, and the mixture was stirred for 12 hours. The reaction mixture was concentrated under vacuum and purified using HPLC (0.1% TFA in H₂O and 0.1% TFA in ACN as buffer) to obtain pure Example 48 as an off-white solid (46%).

### <Example 57> Preparation of N-(5-((5-chloro-4-((1-(methylsulfonyl)indolin-7-yl)amino)pyrimidin-2-yl)amino)-4-methoxy-2-(methyl(2-(methylamino)ethyl)amino)phenyl)acrylamide

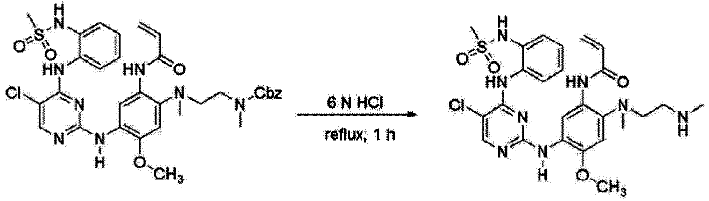

TFA (0.1 mL) was added to a stirred solution of Preparation Example 67 (50 mg 0.07 mmol) dissolved in DCM, and the resulting mixture was stirred for 12 hours. The reaction mixture was concentrated under vacuum and purified using HPLC (0.1% TFA in H₂O and 0.1% TFA in ACN as buffer) to obtain pure Example 57 as an off-white solid (39%).

### <Example 91> Preparation of N-(5-((4-((1-acetylindolin-7-yl)amino)-5-chloropyrimidin-2-yl)amino)-2-((2-(dimethylamino)ethyl)(methyl)amino)-4-methoxyphenyl)acrylamide

*N-*(5-amino-2-((2-(dimethylamino)ethyl)(methyl)amino)-4-methoxyphenyl)acrylamide (a TFA salt) (0.90 g, 3.10 mmol) was added at room temperature to a stirred solution of (1-(7-((2,5-dichloropyrimidin-4-yl)amino)indolin-1-yl)ethan-1-on (1.0 g, 3.10 mmol) dissolved in 1 N TFA in *n*-BuOH (15 mL). The resulting mixture was heated to 90°C for 16 hours. The reaction mixture was cooled and concentrated under reduced pressure. The residue was diluted with CH₂Cl₂ and neutralized by adding saturated sodium bicarbonate. The organic phase was separated and the aqueous phase was extracted with CH₂Cl₂. The organic layers were mixed, dried over MgSO₄, filtered, and concentrated under reduced pressure. The obtained material was purified using silica gel column chromatography in 1-10% methanol with DCM as an eluent. Additional purification was performed by trituration with methanol to obtain pure Example 91 (30%).

Table C below summarizes the structural formulas of the compounds of Examples 1 to 101 prepared according to the present invention.

**[Table C]**

| Example/Name | Structure | NMR; LCMC |
|---|---|---|
| 1/ *N-*(5-((5-chloro-4-((2-(propylsulfonami do)phenyl) amino) pyrimidin-2-yl)amino)-2-((2-(dimethylamino)e thyl)(methyl)amin o)-4-methoxyphenyl)a crylamide | 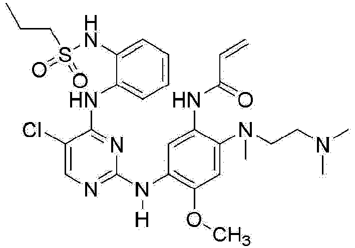 | ¹H NMR (400 MHz, Chloroform-*d*) δ10.14 (s, 1 H), 8.98 (s, 1 H), 8.17 (s, 1 H), 7.67 - 7.58 (d, *J* = 7.9 Hz, 1 H), 7.48 (s, 1 H), 7.44 - 7.39 (d, *J* = 7.8 Hz, 1 H), 7.27 - 7.21 (m, 2 H), 7.21 - 7.14 (t, *J* = 7.6 Hz, 1 H), 6.74 (s, 1 H), 6.46 - 6.36 (d, *J* = 16.5 Hz, 1 H), 6.30 (s, 1 H), 5.75 - 5.68 (d, *J* = 10.7 Hz, 1 H), 3.83 (s, 3 H), 3.04 - 2.97 (m, 2 H), 2.88 (bs, 2 H), 2.70 (s, 3 H), 2.31 (bs, 8 H), 1.89 - 1.77 (m, 2H), 1.04 - 0.96 (t, J = 7.4 Hz, 3 H) ; LCMS: 517.2 [M+H⁺]. |
| 2/ *N-*(5-((5-chloro-4-((2-(phenylsulfonami do)phenyl) amino) pyrimidin-2-yl)amino)-2-((2-(dimethylamino)e thyl)(methyl)amin o)-4-methoxyphenyl)a crylamide | 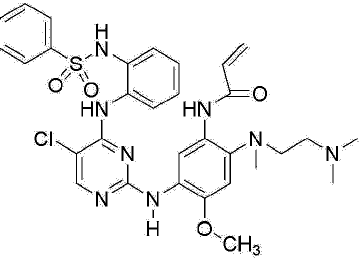 | ¹H NMR (400 MHz, Methanol-*d*₄) δ8.09 (s, 1 H), 7.72 (s, 1 H), 7.70 - 7.66 (d, *J* = 8.0 Hz, 1 H), 7.64 - 7.58 (d, *J* = 7.8 Hz, 2 H), 7.47 - 7.40 (t, *J* = 7.4 Hz, 1 H), 7.33 - 7.22 (m, 3 H), 7.16 - 7.06 (m, 2 H), 6.95 (s, 1 H), 6.57 - 6.49 (m, 2 H), 5.98 - 5.91 (dd, *J* = 7.7, 4.2 Hz, 1 H), 4.00 (s, 3 H), 3.51 - 3.44 (t, *J* = 5.8 Hz, 2 H), 3.28 - 2.23 (t, *J =* 5.8 Hz, 2 H), 2.86 (s, 6 H), 2.70 (s, 3 H); LCMS: 651.2 [M+H⁺]. |
| 3/ *N-*(5-((5-chloro-4-((2-((4-methylphenyl)sulf onamido)phenyl)a mino)pyrimidin-2-yl)amino)-2-((2-(dimethylamino)e thyl)(methyl)amin o)-4-methoxyphenyl)a crylamide | 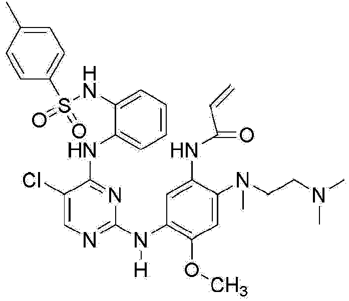 | ¹H NMR (400 MHz, Methanol-*d*₄) δ8.09 (s, 1 H), 7.70 (s, 1 H), 7.66 - 7.61 (d, *J* = 8.1 Hz, 1 H), 7.48 - 7.43 (d, *J* = 7.9 Hz, 2 H), 7.28 - 7.22 (d, *J* = 7.9 Hz, 1 H), 7.22 - 7.12 (m, 2 H), 7.06 - 6.99 (d, *J =* 7.9 Hz, 2 H), 6.95 (s, 1 H), 6.56 - 6.50 (m, 2 H), 5.99 - 5.93 (m, 1 H), 4.00 (s, 3 H), 3.51 - 3.45 (t, *J* = 5.7 Hz, 2 H), 3.28 - 3.22 (d, *J* = 5.8 Hz, 2 H), 2.86 (s, 6 H), 2.71 (s, 3 H); LCMS: 665.8 [M+H⁺]. |
| 4/ *N-*(5-((5-chloro-4-((2-(ethylsulfonamido )phenyl)amino)py rimidin-2-yl)amino)-2-((2-(dimethylamino)e thyl)(methyl)amin o)-4-methoxyphenyl)a crylamide | 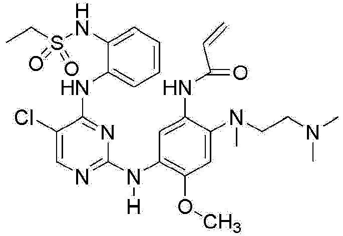 | ¹H NMR (400 MHz, Chloroform-*d)* δ10.11 (s, 1 H), 8.95 (s, 1 H), 8.16 (s, 1 H), 7.63 - 7.57 (d, *J* = 7.8 Hz, 1 H), 7.48 (s, 1 H), 7.45 - 7.40 (d, *J* = 7.8 Hz, 1 H), 7.27 - 7.20 (m, 2 H), 7.20 - 7.14 (t, *J* = 7.7 Hz, 1 H), 6.73 (s, 1 H), 6.51 - 6.15 (m, 2 H), 5.76 - 5.68 (d, *J* = 11.6 Hz, 1 H), 3.83 (s, 3 H), 3.11 - 3.01 (q, *J* = 7.4 Hz, 2 H), 2.91 (bs, 2 H), 2.69 (s, 3 H), 2.34 (bs, 8 H), 1.38 - 1.30 (t, *J* = 7.4 Hz, 3 H); LCMS: 604.8 [M+H⁺]. |
| 5/ *N*-(5-((5-chloro-4-((2-((1-methylethyl)sulfo namido)phenyl)a mino)pyrimidin-2-yl)amino)-2-((2-(dimethylamino)e thyl)(methyl)amin o)-4-methoxyphenyl)a crylamide | 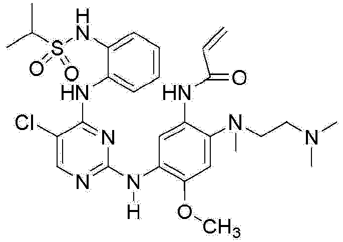 | ¹H NMR (400 MHz, Methanol-*d*₄) δ8.15 (s, 1 H), 7.79 (s, 1 H), 7.75 - 7.69 (dd, *J* = 7.9, 2.0 Hz, 1 H), 7.51 - 7.44 (m, 1 H), 7.33 - 7.20 (m, 2 H), 6.92 (s, 1 H), 6.59 - 6.45 (m, 2 H), 5.99 - 5.91 (dd, *J* = 7.9, 4.0 Hz, 1 H), 3.96 (s, 3 H), 3.50 - 3.43 (t, *J* = 5.6 Hz, 2 H), 3.30 - 3.17 (m, 4 H), 2.86 (s, 6 H), 2.68 (s, 3 H), 1.38 - 1.28 (d, *J* = 6.8 Hz, 6 H); LCMS: 618.2 [M+H⁺]. |
| 6/ *N*-(2-((2-(dimethylamino)e thyl)(methyl)amin o)-4-methoxy-5-((4-((2-(phenylsulfonami do)phenyl) amino) pyrimidin-2-yl)amino)phenyl) acrylamide | 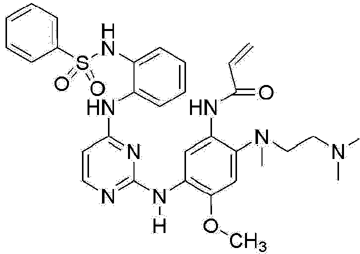 | ¹H NMR (400 MHz, Chloroform-*d)* δ7.86 - 7.81 (d, *J* = 5.8 Hz, 1 H), 7.69 - 7.60 (d, *J* = 7.6 Hz, 3 H), 7.43 (s, 1 H), 7.28 - 7.18 (m, 3 H), 7.18 - 7.11 (t, *J* = 7.7 Hz, 2 H), 6.80 (s, 1 H), 6.56 - 6.47 (d, *J* = 16.9 Hz, 1 H), 6.46 - 6.33 (m, 1 H), 5.82 - 6.75 (d, *J* = 10.2 Hz, 1 H), 5.66 - 5.58 (d, *J* = 5.8 Hz, 1 H), 3.90 (s, 3 H), 2.93 (bs, 2 H), 2.75 (s, 3 H), 2.33 (s, 6 H), 2.30 - 2.22 (m, 2 H); LCMS: 617.8 [M+H⁺]. |
| 7/ N-(2-((2-(dimethylamino)e thyl)(methyl)amin o)-4-methoxy-5-((4-((2-(methylsulfonami do)phenyl) amino) pyrimidin-2-yl)amino)phenyl) acrylamide | 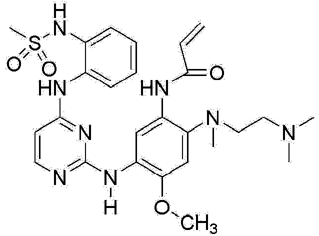 | ¹H NMR (400 MHz, Chloroform-*d)* δ10.34 (s, 1 H), 9.80 (s, 1 H), 8.12 - 8.05 (d, *J* = 5.7 Hz, 1 H), 7.66 - 6.59 (m, 1 H), 7.53 - 7.44 (m, 2 H), 7.40 (bs, 1 H), 7.27 - 7.22 (m, 2 H), 6.78 (s, 1 H), 6.45 - 6.23(m, 1 H), 6.10 - 6.05 (d, *J* = 5.8 Hz, 1 H), 5.75 - 5.69 (d, *J* = 11.3 Hz, 1 H), 3.88 (s, 3 H), 2.96 (s, 3 H), 2.91 (bs, 2 H), 2.73 (s, 3 H), 2.30 (s, 8 H); LCMS: 555.2 [M+H⁺]. |
| 8/ *N*-(2-((2-(dimethylamino)e thyl)(methyl)amin o)-4-methoxy-5-((4-((2-(propylsulfonami do)phenyl)amino) pyrimidin-2-yl)amino)phenyl) acrylamide | 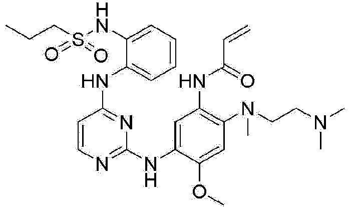 | ¹H NMR (300 MHz, Chloroform-*d)* δ10.35 (s, 1H), 9.70 (s, 1H), 8.03 (d, *J* = 5.8 Hz, 1H), 7.62 - 7.53 (m, 1H), 7.46 - 7.36 (m, 2H), 7.24 - 7.15 (m, 2H), 6.75 (s, 1H), 6.40 (dd, *J* = 16.9, 2.1 Hz, 1H), 6.27 (dd, *J* = 29.3, 7.5 Hz, 1H), 6.01 (d, *J* = 5.8 Hz, 1H), 5.73 - 5.62 (m, 1H), 3.84 (s, 3H), 2.99 - 2.91 (m, 2H), 2.86 (t, *J* = 6.0 Hz, 2H), 2.70 (s, 3H), 2.27 (d, *J* = 2.6 Hz, 8H), 1.85 - 1.67 (m, 2H), 0.83 (t, *J* = 7.4 Hz, 3H); LCMS: 583.8 [M+H⁺]. |
| 9/ *N*-(2-((2-(dimethylamino)e thyl)(methyl)amin o)-4-methoxy-5-((4-((2-((4-methylphenyl)sulf onamido)phenyl)a mino)pyrimidin-2-yl)amino)phenyl) acrylamide | 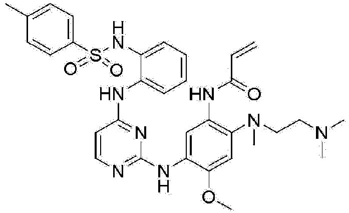 | ¹H NMR (300 MHz, Chloroform-*d*) δ10.45 (s, 1H), 9.83 (s, 1H), 7.82 (d, *J* = 5.8 Hz, 1H), 7.61 - 7.52 (m, 1H), 7.47 (d, *J* = 8.3 Hz, 2H), 7.38 (d, *J* = 11.2 Hz, 2H), 7.23 - 7.09 (m, 3H), 6.88 (d, *J* = 8.0 Hz, 2H), 6.77 (s, 1H), 6.50 (dd, *J* = 17.0, 1.6 Hz, 1H), 6.34 (dd, *J* = 17.1, 10.1 Hz, 1H), 5.75 (dd, *J* = 10.0, 1.6 Hz, 1H), 5.61 (d, *J* = 5.8 Hz, 1H), 3.87 (s, 3H), 2.89 (t, *J* = 5.5 Hz, 2H), 2.73 (s, 3H), 2.29 (s, 8H), 2.19 (s, 3H); LCMS: 631.8 [M+H⁺]. |
| 10/ *N*-(2-((2-(dimethylamino)e thyl)(methyl)amin o)-5-((4-((2-(ethylsulfonamido )phenyl)amino)py rimidin-2-yl)amino)-4-methoxyphenyl)a crylamide | 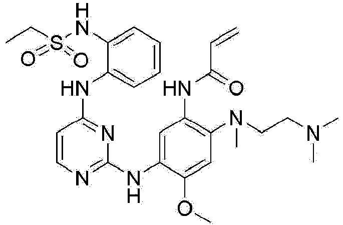 | ¹H NMR (400 MHz, Chloroform-*d*) δ10.29 (s, 1H), 9.77 (s, 1H), 8.06 (d, *J* = 5.7 Hz, 1H), 7.63 (d, 1H), 7.47 (s, 1H), 7.42 (dd, *J* = 7.6, 1.9 Hz, 1H), 7.24 - 7.16 (m, 2H), 6.76 (s, 1H), 6.38 (d, 1H), 6.28 (dd, *J* = 16.9, 9.9 Hz, 1H), 6.00 (d, *J* = 5.7 Hz, 1H), 5.69 (d, *J* = 10.1 Hz, 1H), 3.86 (s, 3H), 3.06 (q, *J* = 7.4 Hz, 2H), 2.87 (t, *J* = 5.5 Hz, 2H), 2.70 (s, 3H), 2.26 (s, 8H), 1.28 (t, *J* = 7.7 Hz, 5H); LCMS: 569.7 [M+H⁺]. |
| 11/ *N*-(2-((2-(dimethylamino)e thyl)(methyl)amin o)-5-((5-fluoro-4-((2-(methylsulfonami do)phenyl) amino) pyrimidin-2-yl)amino)-4-methoxyphenyl)a crylamide | 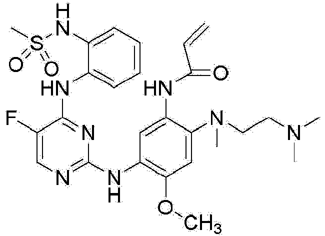 | ¹H NMR (400 MHz, Methanol-*d*₄) δ8.10 - 8.05 (d, *J* = 4.3 Hz, 1 H), 7.79 (s, 1 H), 7.75 - 7.68 (m, 1 H), 7.53 - 7.46(dd, *J* = 6.0, 3.6 Hz, 1 H), 7.33 - 7.25 (m, 2 H), 6.92 (s, 1 H), 6.58 - 6.47 (m, 2 H), 5.99 - 5.91 (dd, *J* = 8.0, 3.9 Hz, 1 H), 3.96 (s, 3 H), 3.49 - 3.43 (t, *J* = 5.8 Hz, 2 H), 3.27 - 3.20 (t, *J* = 5.7 Hz, 2 H), 2.96 (s, 3 H), 2.86 (s, 6 H), 2.68 (s, 3 H). LCMS: 573.2 [M+H⁺]. |
| 12/ *N*-(2-((2-(dimethylamino)e thyl)(methyl)amin o)-5-((4-((2-(ethylsulfonamido )phenyl)amino)-5-fluoropyrimidin-2-yl)amino)-4-methoxyphenyl)a crylamide | 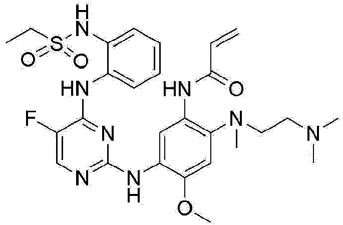 | ¹H NMR (400 MHz, Chloroform-*d*) δ10.16 (s, 1H), 9.10 (s, 1H), 8.04 (d, *J* = 2.9 Hz, 1H), 7.65 (d, *J* = 7.9 Hz, 1H), 7.41 (d, *J* = 7.9 Hz, 1H), 7.35 (s, 1H), 7.26 - 7.17 (m, 3H), 7.14 (t, *J* = 7.6 Hz, 1H), 6.72 (s, 1H), 6.38 (d, *J* = 16.6 Hz, 1H), 6.28 (s, 1H), 5.69 (d, *J* = 10.4 Hz, 1H), 3.82 (s, 3H), 3.05 (q, *J* = 7.4 Hz, 2H), 2.85 (s, 2H), 2.67 (s, 3H), 2.32 - 2.20 (m, 8H), 1.33 (t, *J* = 7.4 Hz, 3H); LCMS: 587.8 [M+H⁺]. |
| 13/ *N*-(2-((2-(dimethylamino)e thyl)(methyl)amin o)-5-((5-fluoro-4-((2-(propylsulfonami do)phenyl) amino) pyrimidin-2-yl)amino)-4-methoxyphenyl)a crylamide | 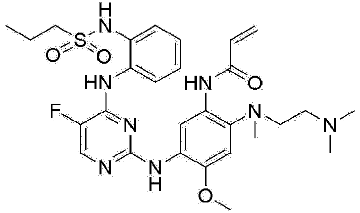 | ¹H NMR (300 MHz, Chloroform-*d*) δ10.22 (s, 1H), 9.08 (s, 1H), 8.00 (d, *J* = 3.0 Hz, 1H), 7.70 (dd, *J* = 8.0, 1.6 Hz, 1H), 7.56 (d, *J* = 2.8 Hz, 1H), 7.39 (dd, *J* = 7.9, 1.6 Hz, 1H), 7.25 - 7.14 (m, 2H), 7.10 (td, *J* = 7.7, 1.6 Hz, 1H), 6.71 (s, 1H), 6.38 (dd, *J* = 16.9, 1.8 Hz, 1H), 6.26 (dd, *J* = 17.1, 9.7 Hz, 1H), 5.69 (dd, *J* = 9.7, 2.1 Hz, 1H), 3.81 (s, 3H), 3.02 - 2.94 (m, 2H), 2.85 (t, *J* = 5.6 Hz, 2H), 2.67 (s, 3H), 2.27 (s, 8H), 1.87 - 1.73 (m, 2H), 0.95 (t, *J* = 7.4 Hz, 3H); LCMS: 601.8 [M+H⁺]. |
| 14/ *N*-(2-((2-(dimethylamino)e thyl)(methyl)amin o)-5-((5-fluoro-4-((2-(phenylsulfonami do)phenyl) amino) pyrimidin-2-yl)amino)-4-methoxyphenyl)a crylamide | 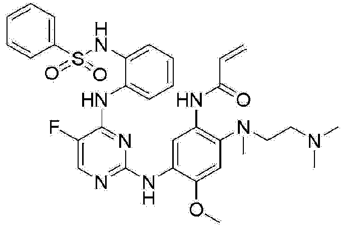 | ¹H NMR (300 MHz, Chloroform-*d*) δ10.19 (s, 1H), 9.11 (s, 1H), 7.93 (d, *J* = 3.1 Hz, 1H), 7.63 - 7.56 (m, 2H), 7.52 (d, *J* = 7.9 Hz, 1H), 7.50 - 7.43 (m, 1H), 7.37 - 7.29 (m, 2H), 7.23 - 7.12 (m, 3H), 7.09 - 7.00 (m, 1H), 6.89 (d, *J* = 2.2 Hz, 1H), 6.72 (s, 1H), 6.39 (d, *J* = 16.7 Hz, 1H), 5.67 (dd, *J* = 11.8, 8.0 Hz, 1H), 3.82 (s, 3H), 2.87 (s, 2H), 2.68 (s, 3H), 2.26 (d, *J* = 14.3 Hz, 8H); LCMS: 635.9 [M+H⁺]. |
| 15/ *N*-(2-((2-(dimethylamino)e thyl)(methyl)amin o)-5-((5-fluoro-4-((2-((4-methylphenyl)sulf onamido)phenyl)a mino)pyrimidin-2-yl)amino)-4-methoxyphenyl)a crylamide | 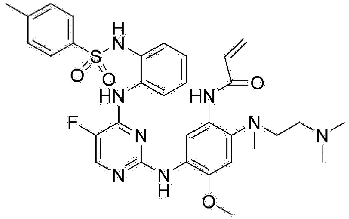 | ¹H NMR (300 MHz, Chloroform-*d*) δ10.17 (s, 1H), 9.12 (s, 1H), 7.93 (d, *J* = 3.1 Hz, 1H), 7.58 (d, 1H), 7.51 - 7.45 (m, 2H), 7.21 - 7.16 (m, 2H), 7.15 - 7.09 (m, 3H), 7.06 - 6.97 (m, 2H), 6.72 (s, 1H), 6.39 (d, *J* = 16.3 Hz, 1H), 6.30 (s, 1H), 5.73 - 5.62 (m, 1H), 3.82 (s, 3H), 2.87 (s, 2H), 2.68 (s, 3H), 2.31 (d, *J* = 15.2 Hz, 11H); LCMS: 649.9 [M+H⁺]. |
| 16/ *N*-(2-((2-(dimethylamino)e thyl)(methyl)amin o)-4-methoxy-5-((5-methyl-4-((2-(methylsulfonami do)phenyl) amino) pyrimidin-2-yl)amino)phenyl) acrylamide | 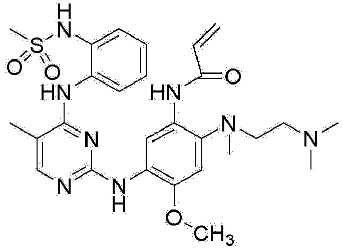 | ¹H NMR (400 MHz, Chloroform-*d*) δ10.11 (s, 1 H), 9.05 (s, 1 H), 7.97 (s, 1 H), 7.79 - 7.73 (d, *J* = 8.1 Hz, 1 H), 7.34 - 7.29 (d, *J* = 7.9 Hz, 1 H), 7.26 - 7.21 (t, *J* = 7.8 Hz, 1 H), 7.20 (s, 1 H), 7.15 (s, 1 H), 7.11 - 7.05 (t, *J* = 7.7 Hz, 1 H), 6.73 (s, 1 H), 6.43 - 6.22 (m, 2 H), 5.73 - 5.66 (m, 1 H), 3.84 (s, 3 H), 2.93 (s, 3 H), 2.90 (bs, 2 H), 2.69 (s, 3 H), 2.32 (s, 8 H), 2.14 (s, 3 H). LCMS: 569.2 [M+H⁺]. |
| 17/ *N*-(2-((2-(dimethylamino)e thyl)(methyl)amin o)-4-methoxy-5-((5-methyl-4-((2-((4-methylphenyl)sulfonamido)phenyl)a mino)pyrimidin-2-yl)amino)phenyl) acrylamide | 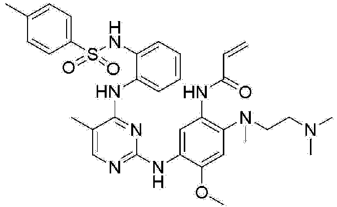 | ¹H NMR (300 MHz, Chloroform-*d*) δ10.09 (s, 1H), 9.09 (s, 1H), 7.90 (s, 1H), 7.64 (d, *J* = 8.1 Hz, 1H), 7.48 (s, 1H), 7.45 (s, 1H), 7.21 - 7.08 (m, 4H), 7.01 - 6.90 (m, 2H), 6.71 (d, *J* = 3.7 Hz, 2H), 6.39 - 6.26 (m, 2H), 5.70 - 5.60 (m, 1H), 3.82 (s, 3H), 2.87 (s, 2H), 2.67 (s, 3H), 2.36 (s, 3H), 2.26 (d, *J* = 15.8 Hz, 8H), 1.95 (s, 3H); LCMS: 645.8 [M+H⁺]. |
| 18/ *N*-(2-((2-(dimethylamino)e thyl)(methyl)amin o)-4-methoxy-5-((5-methyl-4-((2-(propylsulfonami do)phenyl) amino) pyrimidin-2-yl)amino)phenyl) acrylamide | 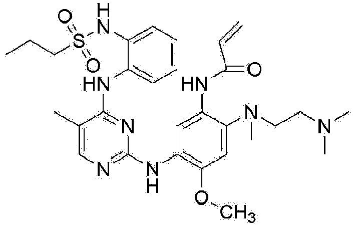 | ¹H NMR (400 MHz, Chloroform-*d*) δ10.17 (s, 1 H), 9.05 (s, 1 H), 7.92 (s, 1 H), 7.80 - 7.74 (d, *J* = 8.1 Hz, 1 H), 7.36 (s, 1 H), 7.26 (s, 1 H), 7.23 - 7.15 (m, 2 H), 7.08 - 6.99 (t, *J* = 7.6 Hz, 1 H), 6.72 (s, 1 H), 6.40 - 6.24 (m, 2 H), 5.73 - 5.64 (m, 1 H), 3.83 (s, 3 H), 3.01 - 2.95 (m, 2 H), 2.92 - 2.84 (t, *J* = 5.6 Hz, 2 H), 2.69 (s, 3 H), 2.31 (s, 8 H), 2.11 (s, 3 H), 1.85 - 1.74 (m, 2 H), 1.00 - 0.91 (t, *J* = 7.4 Hz, 3 H). LCMS: 597.8 [M+H⁺]. |
| 19/ *N*-(2-((2-(dimethylamino)e thyl)(methyl)amin o)-5-((4-((2-(ethylsulfonamido )phenyl)amino)-5-methylpyrimidin-2-yl)amino)-4-methoxyphenyl)a crylamide | 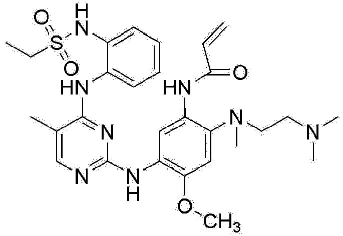 | ¹H NMR (400 MHz, Chloroform-*d*) δ10.16 (s, 1 H), 9.05 (s, 1 H), 7.96 (s, 1 H), 7.75 - 7.69 (d, *J* = 8.0 Hz, 1 H), 7.35 - 7.30 (d, *J* = 7.9 Hz, 1 H), 7.24 - 7.16 (m, 3 H), 7.10 - 7.04 (t, *J* = 7.7 Hz, 1 H), 6.73 (s, 1 H), 6.41 - 6.23 (m, 1 H), 5.73 - 5.66 (m, 1 H), 3.83 (s, 3 H), 3.09 - 2.99 (q, *J* = 7.4 Hz, 2 H), 2.91 - 2.83 (m, 2 H), 2.69 (s, 3 H), 2.30 (s, 8 H), 2.14 (s, 3 H), 1.36 - 1.29 (t, *J* = 7.4 Hz, 3 H). LCMS: 583.4 [M+H⁺]. |
| 20/ *N*-(2-((2-(dimethylamino)e thyl)(methyl)amin o)-4-methoxy-5-((5-methyl-4-((2-(phenylsulfonami do)phenyl)amino) pyrimidin-2-yl)amino)phenyl) acrylamide | 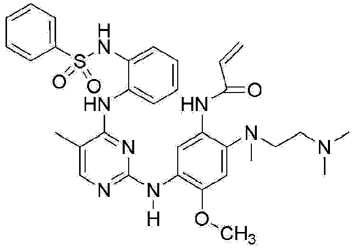 | ¹H NMR (400 MHz, Chloroform-*d*) δ10.20 (s, 1 H), 9.05 (s, 1 H), 7.86 (s, 1 H), 7.65 - 7.61 (d, *J* = 8.0 Hz, 1 H), 7.61 - 7.55 (d, *J* = 7.8 Hz, 2 H), 7.50 - 7.43 (t, *J* = 7.4 Hz, 1 H), 7.37 - 7.29 (t, *J* = 7.6 Hz, 2 H), 7.18 (s, 1 H), 7.16 - 7.11 (d, *J* = 7.7 Hz, 1 H), 7.02 - 6.90 (m, 2 H), 6.76 (s, 1 H), 6.73 (s, 1 H), 6.40 - 6.24 (m, 2 H), 5.72 - 5.64 (m, 1 H), 3.84 (s, 3 H), 2.92 - 2.85 (m, 2 H), 2.69 (s, 3 H), 2.31 (s, 8 H), 1.93 (s, 3 H). LCMS: 631.2 [M+H⁺]. |
| 21/ *N-*(5-((5-chloro-4-((2-(methylsulfonami do)phenyl)amino) pyrimidin-2-yl)amino)-4-methoxy-2-(methyl(2-(pyrrolidin-1-yl)ethyl)amino)ph enyl)acrylamide | 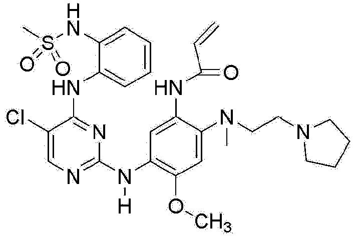 | ¹H NMR (400 MHz, Chloroform-*d*) δ 9.77 (s, 1 H), 8.92 (s, 1 H), 8.15 (s, 1 H), 7.69 - 7.64 (d, *J* = 8.0 Hz, 1 H), 7.53 (s, 1 H), 7.43 - 7.37 (d, *J* = 7.9 Hz, 1 H), 7.27 (s, 1 H), 7.25 - 7.21 (d, *J* = 7.8 Hz, 1 H), 7.19 - 7.13 (t, *J* = 7.7 Hz, 1 H), 6.73 (s, 1 H), 6.47 - 6.26 (m, 2 H), 5.78 - 5.67 (m, 1 H), 3.83 (s, 3 H), 2.94 (bs, 5H), 2.68 (s, 3 H), 2.64 - 2.53 (bs, 4 H), 2.47 (bs, 2 H), 1.88 (bs, 4 H). LCMS: 615.0 [M+H⁺]. |
| 22/ *N-*(5-((5-chloro-4-((2-(methylsulfonami do)phenyl)amino) pyrimidin-2-yl)amino)-4-methoxy-2-(2-(pyrrolidin-1-yl)ethoxy)phenyl) acrylamide | 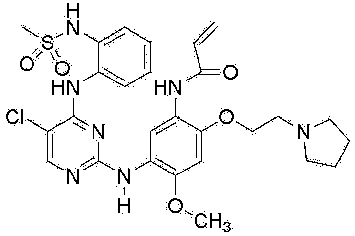 | ¹H NMR (500 MHz, Chloroform-*d*) δ 9.36 (s, 1 H), 8.74 (s, 1 H), 8.10 (s, 1 H), 7.70 - 7.65 (d, *J* = 8.0 Hz, 1 H), 7.60 (s, 1 H), 7.40 - 7.34 (d, *J* = 7.9 Hz, 1 H), 7.23 - 7.18 (t, *J* = 7.8 Hz, 1 H), 7.17 (s, 1 H), 7.15 - 7.09 (t, *J* = 7.6 Hz, 1 H), 6.55 (s, 1 H), 6.41 - 6.34 (d, *J* = 16.8 Hz, 1 H), 6.33 - 6.24 (m, 1 H), 5.74 - 5.70 (d, *J* = 10.2 Hz, 1 H), 4.19 - 4.13 (t, *J* = 5.2 Hz, 2 H), 3.82 (s, 3 H), 2.93 (s, 3 H), 2.78 (bs, 2 H), 2.70 (bs, 4 H), 1.96 - 1.85 (m, 4 H). LCMS: 603.2 [M+H⁺]. |
| 23/ *N-*(5-((5-chloro-4-((2-(methylsulfonami do)phenyl) amino) pyrimidin-2-yl)amino)-4-methoxy-2-(methyl(2-(piperidin-1-yl)ethyl)amino)ph enyl)acrylamide | 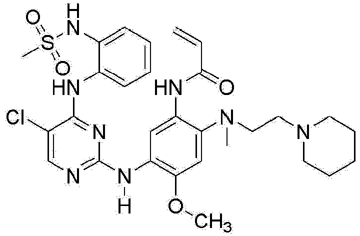 | ¹H NMR (400 MHz, Methanol-*d*₄) δ 8.16 (s, 1 H), 7.84 - 7.76 (m, 1 H), 7.73 (s, 1 H), 7.50 - 7.44 (m, 1 H), 7.33 - 7.24 (m, 2 H), 6.92 (s, 1 H), 6.61 - 6.51 (m, 1 H), 6.51 - 6.43 (m, 1 H), 5.99 - 5.92 (m, 1 H), 3.95 (s, 3 H), 3.52 - 3.47 (t, *J* = 5.6 Hz, 2 H), 3.46 - 3.41 (d, *J* = 12.2 Hz, 2 H), 3.23 - 3.17 (m, 2 H), 2.96 (s, 3 H), 2.93 - 2.83(m, 2 H), 2.68 (s, 3 H), 2.12 - 1.98 (m, 2 H), 1.96 - 1.85 (d, *J* = 12.5 Hz, 2 H), 1.63 - 1.47 (m, 2 H). LCMS: 630.8 [M+H⁺]. |
| 24/ *N*-(5-((5-chloro-4-((2-(methylsulfonami do)phenyl) amino) pyrimidin-2-yl)amino)-2-((2-(isopropylamino) ethyl)(methyl)ami no)-4-methoxyphenyl)a crylamide | 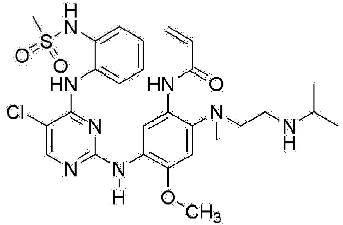 | ¹H NMR (400 MHz, Methanol-*d*₄) δ 8.16 (s, 1 H), 7.84 - 7.77 (d, *J* = 7.8 Hz, 1 H), 7.74 (s, 1 H), 7.49 - 7.43 (d, *J* = 7.5 Hz, 1 H), 7.33 - 7.22 (m, 2 H), 6.88 (s, 1 H), 6.60 - 6.38 (m, 2 H), 5.97 - 5.88 (d, *J* = 9.7 Hz, 1 H), 3.95 (s, 3 H), 3.44 - 3.39 (m, 3 H), 3.15 - 3.07 (m, 2 H), 2.97 (s, 3 H), 2.67 (s, 3 H), 1.37 - 1.30 (d, *J* = 6.7 Hz, 6 H). LCMS: 604.8 [M+2H⁺]. |
| 25/ *N-*(5-((5-chloro-4-((2-(methylsulfonami do)phenyl)amino) pyrimidin-2-yl)amino)-4-methoxy-2-(methyl(2-(methylamino)eth yl)amino)phenyl) acrylamide | 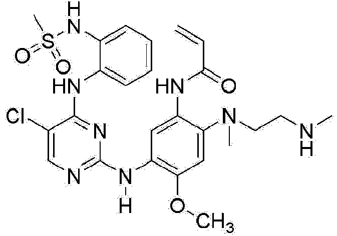 | ¹H NMR (400 MHz, Methanol-*d*₄) δ 8.15 (s, 1 H), 7.84 - 7.77 (m, 2 H), 7.48 - 7.43 (dd, *J* = 7.6, 1.9 Hz, 1 H), 7.34 - 7.25 (m, 2H), 6.90 (s, 1 H), 6.60 - 6.41 (m, 2 H), 5.96 - 5.89 (m, 1 H), 3.94 (s, 3 H), 3.42 - 3.36 (t, *J* = 5.6 Hz, 2 H), 3.16 - 3.10 (t, *J* = 5.5 Hz, 2 H), 2.98 (s, 3 H), 2.71 (s, 3 H), 2.67 (s, 3 H). LCMS: 576.2 [M+2H⁺]. |
| 26/ (E)-*N*-(5-((5-chloro-4-((2-(methylsulfonami do)phenyl) amino) pyrimidin-2-yl)amino)-4-methoxy-2-morpholinopheny l)-4-(dimethylamino)b uten-2-amide | 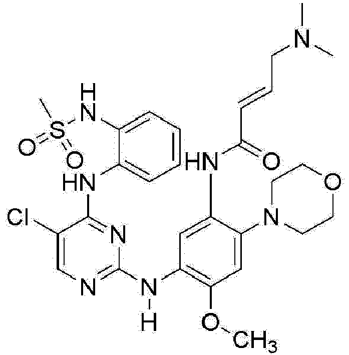 | ¹H NMR (400 MHz, Methanol-*d*₄) δ 8.26 (s, 1 H), 8.13 (d, *J* = 4.5 Hz, 1 H), 7.82 (t, *J* = 5.6 Hz, 1 H), 7.44 (t, *J* = 4.4 Hz, 1 H), 7.32 - 7.23 (m, 2 H), 6.91 (s, 1 H), 6.85 (d, *J* = 7.2 Hz, 1 H), 6.77 (d, *J* = 15.3 Hz, 1 H), 4.06 (d, *J* = 6.9 Hz, 2 H), 3.90 (s, 3 H), 3.62 (bs, 4 H), 3.23 (bs, 2 H), 3.17 (d, *J* = 12.0 Hz, 2H), 3.01 (d, *J* = 2.3 Hz, 3 H), 2.98 (s, 6 H). |
| 27/ (E)-*N*-(5-((5-chloro-4-((2-(methylsulfonami do)phenyl) amino) pyrimidin-2-yl)amino)-4-methoxy-2-(4-methylpiperazin-1-yl)phenyl)-4-(dimethylamino)b uten-2-amide | 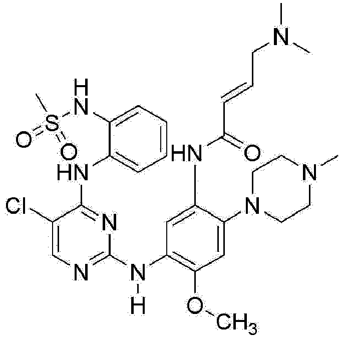 | ¹H NMR (400 MHz, Methanol-*d*₄) δ 8.11 (s, 1 H), 8.08 (s, 1 H), 7.83 - 7.77 (m, 1 H), 7.49 - 7.43 (d, *J* = 8.8 Hz, 1 H), 7.32 - 7.24 (m, 2 H), 6.92 (s, 1 H), 6.86 - 6.81 (m, 1 H), 6.72 (s, 1 H), 6.45 (s, 1 H), 4.07 - 4.02 (d, *J* = 7.0 Hz, 2 H), 3.89 (bs, 7 H), 3.04 - 2.90 (m, 13 H). LCMS: 645.0 [M+2H⁺]. |
| 28/ (E)-*N*-(5-((5-chloro-4-((2-(methylsulfonami do)phenyl)amino) pyrimidin-2-yl)amino)-2-((2-(dimethylamino)e thyl)(methyl)amin o)-4-methoxyphenyl)-4-(dimethylamino)b uten-2-amide | 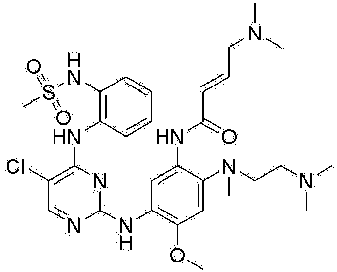 | ¹H NMR (300 MHz, Methanol-*d₄*) δ 8.10 (s, 1H), 7.92 (s, 1H), 7.82 - 7.77 (m, 1H), 7.45 - 7.40 (m, 1H), 7.28 - 7.21 (m, 2H), 6.98 - 6.92 (m, 1H), 6.90 (s, 1H), 6.69 (d, *J* = 15.2 Hz, 1H), 4.07 (d, *J* = 7.1 Hz, 2H), 3.92 (s, 3H), 3.43 (t, *J* = 6.2 Hz, 2H), 2.97 (s, 6H), 2.93 (s, 3H), 2.88 (s, 3H), 2.85 (s, 6H), 2.64 (s, 2H); LCMS: 646.6 [M+H⁺]. |
| 29/ *N-*(5-((5-chloro-4-((2-(*N-*methylmethylsulf onamido)phenyl)a mino)pyrimidin-2-yl)amino)-2-((2-(dimethylamino)e thyl)(methyl)amin o)-4-methoxyphenyl)a crylamide | 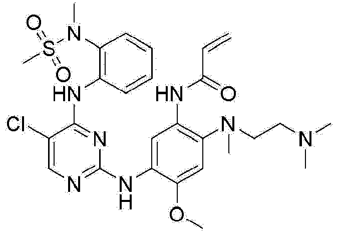 | ¹H NMR (300 MHz, Chloroform-*d*) δ 10.03 (s, 1H), 9.18 (s, 1H), 8.41 (d, *J* = 8.2 Hz, 1H), 8.31 (s, 1H), 8.16 (s, 1H), 7.29 (d, *J* = 1.6 Hz, 1H), 7.06 (t, *J* = 7.4 Hz, 1H), 6.74 (s, 1H), 6.36 - 6.30 (m, 2H), 5.69 - 5.64 (m, 1H), 3.84 (s, 3H), 3.29 (s, 3H), 2.98 (s, 3H), 2.87 (t, *J* = 5.4 Hz, 2H), 2.68 (s, 3H), 2.28 (s, 8H); LCMS: 603.7 [M+H⁺]. |
| 30/ *N-*(5-((5-chloro-4-((2-(*N-*methylethylsulfon amido)phenyl)am ino)pyrimidin-2-yl)amino)-2-((2-(dimethylamino)e thyl)(methyl)amin o)-4-methoxyphenyl)a crylamide | 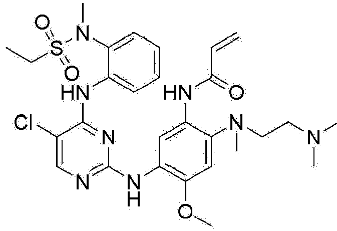 | ¹H NMR (300 MHz, Methanol-*d₄*) δ 8.15 (s, 1H), 7.98 (dd, *J* = 8.1, 1.6 Hz, 1H), 7.88 (s, 1H), 7.57 (dd, *J* = 7.8, 1.6 Hz, 1H), 7.40 - 7.32 (m, 1H), 7.28 (td, *J* = 7.6, 1.7 Hz, 1H), 6.93 (s, 1H), 6.53 - 6.47 (m, 2H), 5.96 - 5.89 (m, 1H), 3.96 (s, 3H), 3.47 (t, *J* = 5.7 Hz, 2H), 3.26 (s, 3H), 3.22 (d, *J* = 7.5 Hz, 2H), 2.85 (s, 6H), 2.68 (s, 3H), 1.36 (t, *J* = 7.4 Hz, 3H); LCMS: 617.6 [M+H⁺]. |
| 31/ *N-*(5-((5-chloro-4-((2-(*N-*ethylmethylsulfon amido)phenyl)am ino)pyrimidin-2-yl)amino)-2-((2-(dimethylamino)e thyl)(methyl)amin o)-4-methoxyphenyl)a crylamide | 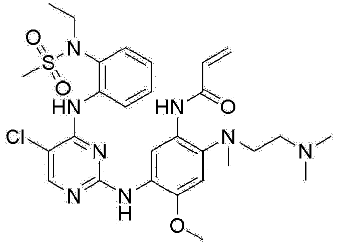 | ¹H NMR (300 MHz, Methanol-*d₄*) δ 8.19 - 8.13 (m, 2H), 7.95 (s, 1H), 7.56 (dd, *J* = 7.9, 1.6 Hz, 1H), 7.37 (t, 1H), 7.27 (t, 1H), 6.94 (s, 1H), 6.50 (d, *J* = 6.3 Hz, 2H), 5.95 - 5.89 (m, 1H), 3.96 (s, 3H), 3.48 (t, *J* = 5.7 Hz, 2H), 3.25 (t, *J* = 5.7 Hz, 2H), 3.04 (s, 3H), 2.86 (s, 6H), 2.69 (s, 3H), 1.01 (t, *J* = 7.1 Hz, 3H); LCMS: 617.6 [M+H⁺]. |
| 32/ *N-*(5-((5-chloro-4-((2-(*N-*isopropylmethyls ulfonamido)phen yl)amino)pyrimid in-2-yl)amino)-2-((2-(dimethylamino)e thyl)(methyl)amin o)-4-methoxyphenyl)a crylamide | 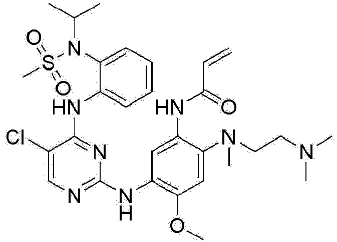 | ¹H NMR (300 MHz, Methanol-*d₄*) δ 8.17 (s, 1H), 8.13 (d, *J* = 8.2 Hz, 1H), 7.97 (s, 1H), 7.48 (dd, *J* = 7.9, 1.6 Hz, 1H), 7.43 (t, *J* = 7.9 Hz, 1H), 7.27 (td, *J* = 7.6, 1.5 Hz, 1H), 6.94 (s, 1H), 6.51 - 6.46 (m, 2H), 5.92 (dd, *J* = 6.6, 5.3 Hz, 1H), 4.55 (d, *J* = 6.7 Hz, 1H), 3.96 (s, 3H), 3.47 (d, *J* = 5.9 Hz, 2H), 3.26 (d, *J* = 5.7 Hz, 2H), 3.08 (s, 3H), 2.86 (s, 6H), 2.69 (s, 3H), 1.27 (d, *J* = 6.7 Hz, 3H), 0.96 (d, *J* = 6.7 Hz, 3H); LCMS: 631.6 [M+H⁺]. |
| 33/ *N-*(5-((5-chloro-4-((2-(*N-*ethylethylsulfona mido)phenyl)ami no)pyrimidin-2-yl)amino)-2-((2-(dimethylamino)e thyl)(methyl)amin o)-4-methoxyphenyl)a crylamide | 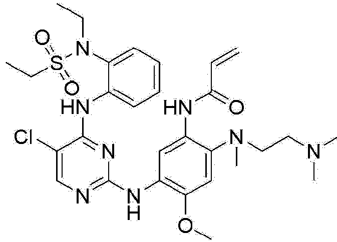 | ¹H NMR (300 MHz, Methanol-*d₄*) δ 8.16 (s, 1H), 8.09 (dd, *J* = 8.0, 1.5 Hz, 1H), 7.93 (s, 1H), 7.54 (dd, *J* = 7.9, 1.6 Hz, 1H), 7.38 (td, *J* = 7.8, 1.6 Hz, 1H), 7.28 (td, *J* = 7.6, 1.6 Hz, 1H), 6.94 (s, 1H), 6.51 (d, *J* = 1.1 Hz, 1H), 6.49 (s, 1H), 5.96 - 5.89 (m, 1H), 3.96 (s, 3H), 3.47 (t, *J* = 5.7 Hz, 2H), 3.27 - 3.16 (m, 4H), 2.85 (s, 6H), 2.69 (s, 3H), 1.39 (t, *J* = 7.4 Hz, 3H), 0.99 (t, *J* = 7.1 Hz, 3H); LCMS: 631.6 [M+H⁺]. |
| 34/ *N*-(5-((5-chloro-4-((3-chloro-2-(methylsulfonami do)phenyl)amino) pyrimidin-2-yl)amino)-2-((2-(dimethylamino)e thyl)(methyl)amin o)-4-methoxyphenyl)a crylamide | 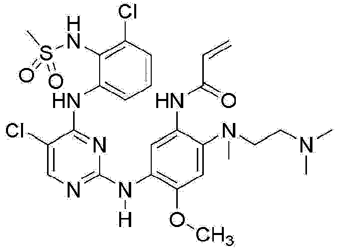 | ¹H NMR (400 MHz, Chloroform-*d*) δ 10.00 (s, 1 H), 9.05 (s, 1 H), 8.61 (s, 1 H), 8.28 - 8.09 (m, 2 H), 7.27 - 7.09 (m, 2 H), 6.73 (s, 1 H), 6.36 (d, *J* = 15.4 Hz, 2 H), 5.80 - 5.62 (m, 1 H), 3.84 (s, 3H), 3.11 (s, 3 H), 3.04 - 2.84 (m, 2 H), 2.70 (s, 3 H), 2.62 - 2.11 (bs, 8 H). LCMS: 624.0 [M+2H⁺]. |
| 35/ *N*-(5-((5-chloro-4-((3-chloro-2-(*N-*methylmethylsulf onamido)phenyl)a mino)pyrimidin-2-yl)amino)-2-((2-(dimethylamino)e thyl)(methyl)amin o)-4-methoxyphenyl)a crylamide | 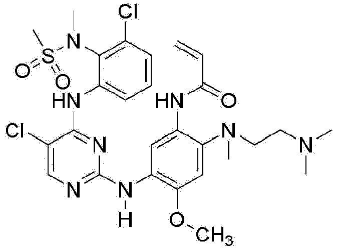 | ¹H NMR (400 MHz, Methanol-*d₄*) δ 8.20 (s, 1 H), 7.89 - 7.83 (d, *J* = 8.1 Hz, 1 H), 7.81 (s, 1 H), 7.47 - 7.32 (m, 2 H), 6.95 (s, 1 H), 6.60 - 6.44 (m, 2 H), 5.98 - 5.90 (d, *J* = 9.3 Hz, 1 H), 3.96 (s, 3 H), 3.48 (bs, 2 H), 3.30 - 3.24 (m, 5 H), 3.17 (s, 3 H), 2.88 (bs, 6 H), 2.70 (s, 3 H). LCMS: 638.0 [M+2H⁺]. |
| 36/ *N-*(5-((5-chloro-4-((3-methyl-2-(methylsulfonami do)phenyl)amino) pyrimidin-2-yl)amino)-2-((2-(dimethylamino)e thyl)(methyl)amin o)-4-methoxyphenyl)a crylamide | 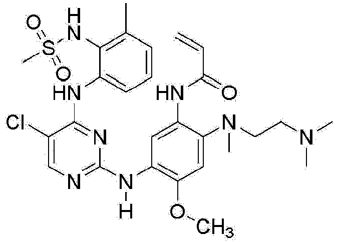 | ¹H NMR (400 MHz, Methanol-*d*₄) δ 8.17 (s, 1 H), 7.84 (s, 1 H), 7.70 - 7.65 (d, *J* = 7.9 Hz, 1 H), 7.28 - 7.21 (t, *J* = 7.8 Hz, 1 H), 7.20 - 7.15 (d, *J* = 7.6 Hz, 1 H), 6.94 (s, 1 H), 6.59 - 6.46 (m, 2 H), 5.99 - 5.91 (m, 1 H), 3.97 (s, 3 H), 3.51 - 3.44 (t, *J* = 5.8 Hz, 2 H), 3.28 - 3.23 (t, *J* = 5.7 Hz, 2 H), 3.05 (s, 3 H), 2.87 (s, 6 H), 2.69 (s, 3 H), 2.46 (s, 3 H). LCMS: 604.2 [M+2H⁺]. |
| 37/ *N-*(5-((5-chloro-4-((3 -isopropyl-2-(*N-*methylmethylsulf onamido)phenyl)a mino)pyrimidin-2-yl)amino)-2-((2-(dimethylamino)e thyl)(methyl)amin o)-4-methoxyphenyl)a crylamide | 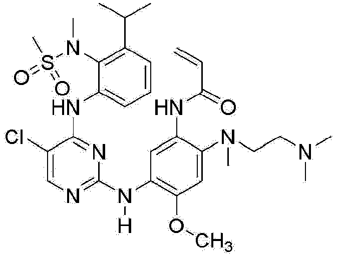 | ¹H NMR (400 MHz, Methanol-*d₄*) δ 8.19 - 8.12 (m, 1 H), 7.84 (s, 1 H), 7.73 - 7.65 (d, *J* = 8.1 Hz, 1 H), 7.44 - 7.35 (t, *J* = 7.4 Hz, 1 H), 7.33 - 7.26 (d, *J* = 8.1 Hz, 1 H), 6.94 (s, 1 H), 6.60 - 6.46 (m, 2 H), 6.00 - 5.90 (m, 1 H), 4.01 (s, 3 H), 3.54 - 3.40 (m, 2 H), 3.30 - 3.22 (m, 3 H), 3.22 - 3.17 (m, 3 H), 3.17 - 3.12 (m, 3 H), 2.86 (s, 6 H), 2.72 - 2.63 (m, 3 H), 1.37 - 1.23 (m, 6 H). LCMS: 645.8 [M+H⁺]. |
| 38/ *N-*(5-((5-chloro-4-((3-methyl-2-*(N-*methylmethylsulf onamido)phenyl)a mino)pyrimidin-2-yl)amino)-2-((2-(dimethylamino)e thyl)(methyl)amin o)-4-methoxyphenyl)a crylamide | 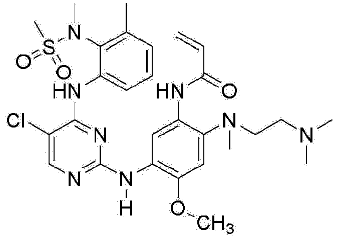 | ¹H NMR (400 MHz, Chloroform-*d*) δ 10.07 (s, 1 H), 9.22 (s, 1 H), 8.18 (s, 1 H), 8.12 - 8.07 (d, *J* = 8.3 Hz, 1 H), 7.96 (s, 1 H), 7.27 (s, 1 H), 7.23 - 7.17 (t, *J* = 7.7 Hz, 1 H), 6.99 - 6.95 (d, *J* = 7.7 Hz, 1 H), 6.77 (s, 1 H), 6.41 - 6.33 (m, 1 H), 6.33 - 6.23 (m, 1 H), 5.72 - 5.66 (d, *J* = 9.8 Hz, 1 H), 3.86 (s, 3 H), 3.27 (s, 3 H), 3.11 (s, 3 H), 2.88 (bs, 2 H), 2.71 (s, 3 H), 2.41 (s, 3 H), 2.30 (s, 8 H). LCMS: 618.2 [M+2H⁺]. |
| 39/ *N-*(5-((5-chloro-4-((2-(*N-*ethylmethylsulfon amido)-3-methylphenyl)ami no)pyrimidin-2-yl)amino)-2-((2-(dimethylamino)e thyl)(methyl)amin o)-4-methoxyphenyl)a crylamide | 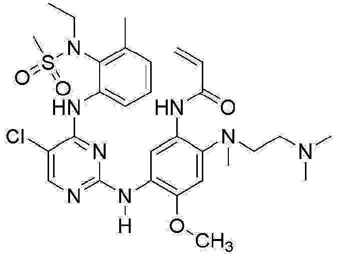 | ¹H NMR (400 MHz, Chloroform-*d*) δ 10.06 (s, 1 H), 9.21 (s, 1 H), 8.31 - 8.15 (m, 3 H), 8.08 (s, 1 H), 7.27 - 7.19 (m, 1 H), 7.01 (d, *J* = 7.4 Hz, 1 H), 6.76 (s, 1 H), 6.37 (m, 2 H), 5.69 (d, *J* = 9.3 Hz, 1H), 3.99 - 3.81 (m, 5H), 3.55 - 3.41 (m, 2 H), 3.08 (d, *J* = 3.4 Hz, 3 H), 2.90 (s, 2 H), 2.71 (s, 3 H), 2.39 (s, 3H), 2.32 (s, 8 H), 1.18 (t, *J* = 3.7 Hz, 3 H). LCMS: 632.0 [M+2H⁺]. |
| 40/ *N-*(5-((5-chloro-4-((3-ethyl-2-(methylsulfonami do)phenyl)amino) pyrimidin-2-yl)amino)-2-((2-(dimethylamino)e thyl)(methyl)amin o)-4-methoxyphenyl)a crylamide | 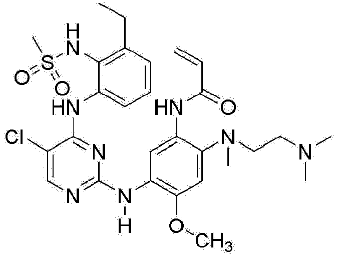 | ¹H NMR (500 MHz, Chloroform-*d*) δ 10.10 (s, 1 H), 9.15 (s, 1 H), 8.16 (s, 1 H), 7.88 (s, 1 H), 7.74 - 7.69 (d, *J* = 8.0 Hz, 1 H), 7.28 - 7.23 (m, 1 H), 7.22 (s, 1 H), 7.13 - 7.08 (d, *J* = 7.7 Hz, 1 H), 6.75 (s, 1 H), 6.43 - 6.36 (d, *J* = 16.8 Hz, 1 H), 6.29 (bs, 1 H), 5.73 - 5.67 (d, *J* = 10.2 Hz, 1 H), 3.84 (s, 3H), 3.01 (s, 3 H), 2.92 - 2.81 (m, 4 H), 2.70 (s, 3 H), 2.29 (s, 8 H), 1.28 - 1.21 (t, *J* = 7.5 Hz, 3 H). LCMS: 617.8 [M+H⁺]. |
| 41/ *N-*(5-((5-chloro-4-((3-ethyl-2-(*N-*methylmethylsulf onamido)phenyl)a mino)pyrimidin-2-yl)amino)-2-((2-(dimethylamino)e thyl)(methyl)amin o)-4-methoxyphenyl)a crylamide | 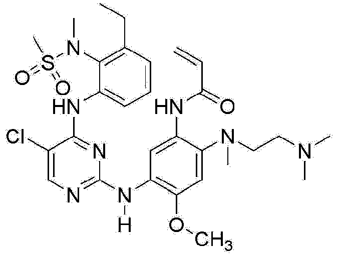 | ¹H NMR (400 MHz, Methanol-*d*₄) δ 8.16 (s, 1 H), 7.79 (s, 1 H), 7.66 - 7.59 (d, *J* = 7.9 Hz, 1 H), 7.41 - 7.33 (m, 1 H), 7.30 - 7.25 (d, *J* = 7.9 Hz, 1 H), 6.93 (s, 1H), 6.62 - 6.42 (m, 2 H), 5.98 - 5.91 (d, *J* = 8.8 Hz, 1 H), 3.96 (s, 3 H),3.55 - 3.39 (m, 2 H), 3.26 (bs, 2 H), 3.16 (s, 3 H), 3.12 (s, 3 H), 2.86 (s, 6 H), 2.82 - 2.70 (m, 2 H), 2.68 (s, 3 H), 1.34 - 1.27 (td, *J* = 7.6, 3.9 Hz, 3 H). LCMS: 631.8 [M+H⁺]. |
| 42/ *N*-(5-((5-chloro-4-((3-ethyl-2-(ethylsulfonamido )phenyl)amino)py rimidin-2-yl)amino)-2-((2-(dimethylamino)e thyl)(methyl)amin o)-4-methoxyphenyl)a crylamide | 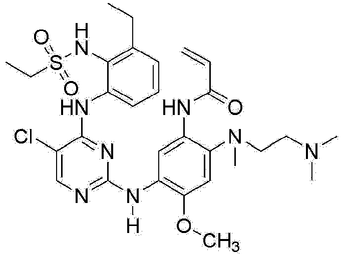 | ¹H NMR (500 MHz, Chloroform-*d*) δ 10.10 (s, 1 H), 9.13 (s, 1 H), 8.16 (s, 1 H), 7.88 (s, 1 H), 7.69 - 7.65 (dd, *J* = 8.0, 1.4 Hz, 1 H), 7.28 - 7.24 (m, 1 H), 7.22 (s, 1 H), 7.14 - 7.10 (d, *J* = 7.8 Hz, 1 H), 6.73 (s, 2 H), 6.46 - 6.21 (m, 2 H), 5.74 - 5.67 (d, *J* =11.4 Hz, 1 H), 3.84 (s, 3 H), 3.18 - 3.11 (q, *J* = 7.4 Hz, 2 H), 2.99 - 2.81 (m, 4 H), 2.70 (s, 3 H), 2.31 (s, 8 H) 1.42 - 1.37 (t, *J* = 7.4 Hz, 3 H), 1.29 - 1.23 (t, *J* = 7.5 Hz, 3 H). LCMS: 631.9 [M+2H⁺]. |
| 43/ *N-*(5-((5-chloro-4-((3,4-dimethyl-2-(*N-*methylmethylsulf onamido)phenyl)a mino)pyrimidin-2-yl)amino)-2-((2-(dimethylamino)e thyl)(methyl)amin o)-4-methoxyphenyl)a crylamide | 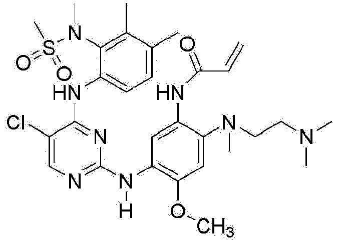 | ¹H NMR (400 MHz, Chloroform-*d*) δ 10.08 (s, 1 H), 9.26 (s, 1 H), 8.17 (s, 1 H), 8.01 - 7.94 (d, *J* = 8.5 Hz, 1 H), 7.90 (s, 1 H), 7.26 (s, 1 H), 7.17 - 7.09 (d, *J* = 8.4 Hz, 1 H), 6.76 (s, 1 H), 6.44 - 6.35 (d, *J* = 16.6 Hz, 1 H), 6.35 - 6.24 (m, 1 H), 5.73 - 7.66 (d, *J* = 9.9 Hz, 1 H), 3.85 (s, 3 H), 3.26 (s, 3 H), 3.09 (s, 3 H), 2.88 (bs, 2 H), 2.71 (s, 3 H), 2.29 (s, 8 H), 2.26 (s, 3 H). LCMS: 632.0 [M+2H⁺]. |
| 44/ *N-*(5-((5-chloro-4-((3,4-dimethyl-2-(methylsulfonami do)phenyl)amino) pyrimidin-2-yl)amino)-2-((2-(dimethylamino)e thyl)(methyl)amin o)-4-methoxyphenyl)a crylamide | 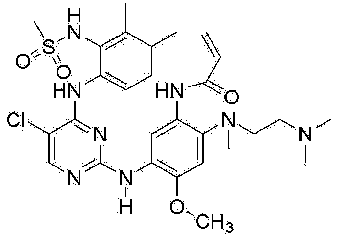 | ¹H NMR (500 MHz, Chloroform-*d*) δ 10.11 (s, 1 H), 9.11 (s, 1 H), 8.14 (s, 1 H), 7.74 (s, 1 H), 7.50 - 7.47 (d, *J* = 7.9 Hz, 1 H), 7.22 (s, 1 H), 7.15 - 7.10 (d, *J* = 8.2 Hz, 1 H), 6.72 (s, 1 H), 6.43 - 6.23 (m, 2 H), 5.74 - 5.68 (m, 1 H), 3.84 (s, 3 H), 2.95 (s, 5 H), 2.70 (s, 3 H), 2.33 (s, 8 H), 2.27 (s, 6 H). LCMS: 618.2 [M+2H⁺]. |
| 45/ *N-*(5-((5-chloro-4-((4-methyl-2-(*N-*methylmethylsulf onamido)phenyl)a mino)pyrimidin-2-yl)amino)-2-((2-(dimethylamino)e thyl)(methyl)amin o)-4-methoxyphenyl)a crylamide | 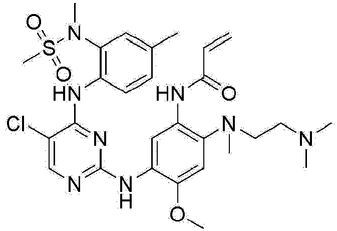 | ¹H NMR (400 MHz, Methanol-*d₄*) δ 8.12 (s, 1H), 7.98 - 7.92 (m, 2H), 7.42 (d, *J* = 2.0 Hz, 1H), 7.17 (dd, 1H), 6.95 (s, 1H), 6.51 (d, *J* = 2.3 Hz, 1H), 6.50 (s, 1H), 5.92 (dd, *J* = 7.0, 4.8 Hz, 1H), 3.96 (s, 3H), 3.48 (t, *J* = 5.7 Hz, 2H), 3.28 - 3.24 (m, 5H), 3.03 (d, *J* = 1.3 Hz, 3H), 2.86 (s, 6H), 2.69 (s, 3H), 2.37 (s, 3H); LCMS: 617.6 [M+H⁺]. |
| 46/ *N-*(5-((5-chloro-4-((4-methyl-2-(methylsulfonami do)phenyl) amino) pyrimidin-2-yl)amino)-2-((2-(dimethylamino)e thyl)(methyl)amin o)-4-methoxyphenyl)a crylamide | 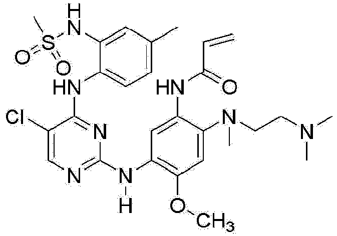 | ¹H NMR (400 MHz, Chloroform-*d*) δ 10.16 (s, 1 H), 9.03 (s, 1 H), 8.14 (s, 1 H), 7.53 - 7.44(d, *J* = 8.3 Hz, 1 H), 7.39 (s, 1 H), 7.26 - 7.21 (d, *J* = 3.9 Hz, 2 H), 7.10 - 7.04 (d, *J* = 8.3 Hz, 1 H), 6.72 (s, 1 H), 6.47 - 6.20 (m, 2 H), 5.75 - 5.67 (m, 1 H), 3.83 (s, 3 H), 2.92 (s, 5 H), 2.70 (s, 3 H), 2.34 (m, 11 H). LCMS: 603.8 [M+H⁺]. |
| 47/ *N-*(5-((5-chloro-4-((1-(methylsulfonyl)-1,2,3,4-tetrahydroquinoli n-8-yl)amino)pyrimid in-2-yl)amino)-2-((2-(dimethylamino)e thyl)(methyl)amin o)-4-methoxyphenyl)a crylamide | 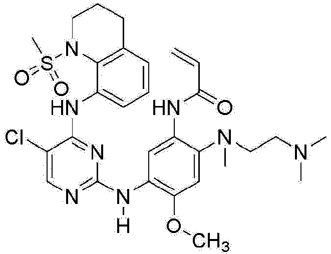 | ¹H NMR (500 MHz, Chloroform-*d*) δ 10.08 (s, 1 H), 9.16 (s, 1 H), 8.53 (s, 1 H), 8.16 (s, 1 H), 7.99 - 7.94 (d, *J* = 8.2 Hz, 1 H), 7.23 (s, 1 H), 7.20 - 7.15 (t, *J* = 7.9 Hz, 1 H), 6.94 - 6.89 (d, *J* = 7.5 Hz, 1 H), 6.75 (s, 1 H), 6.41 - 6.35 (d, *J* = 16.8 Hz, 1 H), 6.34 - 6.35 (m, 1 H), 5.71 - 5.65 (m, 1 H), 3.84 (s, 3 H), 3.73 (bs, 2 H), 3.02 (s, 3 H), 2.88 (bs, 2 H), 2.86 - 2.81 (t, *J* = 7.2 Hz, 2 H), 2.70 (s, 3 H), 2.30 (bs, 8 H), 2.21 - 2.12 (m, 2 H). LCMS: 629.8 [M+H⁺]. |
| 48/ *N-*(5-((5-chloro-4-((1-(methylsulfonyl)-1,2,3,4-tetrahydroquinolin-8-yl)amino)pyrimid in-2-yl)amino)-4-methoxy-2-(methyl(2-(methylamino)eth yl)amino)phenyl) acrylamide | 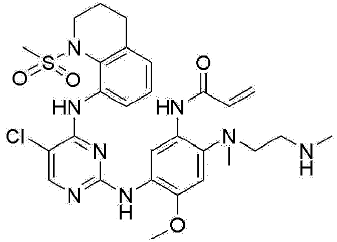 | ¹H NMR (500 MHz, Chloroform-*d*) δ 8.70 (s, 1 H), 8.53 (s, 1 H), 8.10 (s, 1 H), 7.92 (d, *J* = 8.2 Hz, 1 H), 7.31 (s, 1 H), 7.22 (t, *J* = 7.8 Hz, 1 H), 6.97 (d, *J* = 7.5 Hz, 1 H),6.70 - 6.64 (m 1 H) 6.62 (s, 1 H), 6.38 (dd, *J* = 16.9, 1.7 Hz, 1 H), 5.75 (d, *J* = 10.1 Hz, 1 H), 3.86 (s, 3 H), 3.76 - 3.66 (m, 2 H), 3.19 (bs, 2 H), 3.02 (s, 3 H), 2.92 - 2.81 (m, 4 H), 2.63 (s, 3 H), 2.56 (s, 3 H), 2.22 - 2.11 (m, 2 H). LCMS: 615.2 [M+H⁺]. |
| 49/ *N-*(5-((5-chloro-4-((1-(methylsulfonyl)-1,2,3,4-tetrahydroquinoli n-8-yl)amino)pyrimid in-2-yl)amino)-2-(4-(dimethylamino)p iperidin-1-yl)-4-methoxyphenyl)a crylamide | 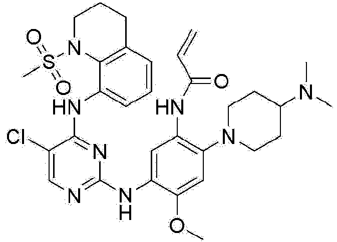 | ¹H NMR (300 MHz, Chloroform-*d*) δ 9.03 (s, 1H), 8.51 (s, 1H), 8.24 (s, 1H), 8.11 (s, 1H), 7.90 (d, *J* = 8.2 Hz, 1H), 7.22 (s, 1H), 7.15 (t, *J* = 7.8 Hz, 1H), 6.91 (d, *J* = 7.5 Hz, 1H), 6.65 (s, 1H), 6.32 - 6.25 (m, 2H), 5.76 - 5.70 (m, 1H), 3.82 (s, 3H), 3.70 (s, 2H), 3.06 (d, *J* = 12.0 Hz, 2H), 2.99 (s, 3H), 2.82 (t, *J* = 7.2 Hz, 2H), 2.70 (t, *J* = 11.7 Hz, 2H), 2.56 (s, 7H), 2.13 (d, *J* = 7.3 Hz, 4H), 1.84 (d, *J* = 12.3 Hz, 2H); LCMS: 655.6 [M+H⁺]. |
| 50/ *N*-(5-((5-chloro-4-((1-(methylsulfonyl)-1,2,3,4-tetrahydroquinoli n-8-yl)amino)pyrimid in-2-yl)amino)-4-methoxy-2-(4-(4-methylpiperazin-1-yl)piperidin-1-yl)phenyl)acryla mide | 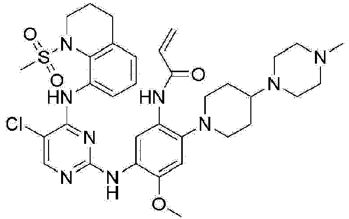 | ¹H NMR (300 MHz, Methanol-*d₄*) δ 8.09 (s, 1H), 7.97 (s, 1H), 7.56 (d, *J* = 7.9 Hz, 1H), 7.20 (t, *J* = 7.7 Hz, 1H), 7.13 (d, *J* = 7.4 Hz, 1H), 6.89 (s, 1H), 6.55 (dd, *J* = 17.0, 10.1 Hz, 1H), 6.34 (d, *J* = 16.4 Hz, 1H), 5.84 (d, *J* = 11.5 Hz, 1H), 3.87 (s, 3H), 3.63 (s, 2H), 3.20 (d, *J* = 12.6 Hz, 6H), 3.00 (s, 3H), 2.84 (d, *J* = 11.1 Hz, 8H), 2.08 (d, *J* = 12.3 Hz, 4H), 1.96 - 1.79 (m, 2H); LCMS: 710.7 [M+H⁺]. |
| 51/ methyl 2-((5-acrylamido-4-((2-(dimethylamino)e thyl)(methyl)amin o)-2-methoxyphenyl)a mino)-4-((1-(methylsulfonyl)-1,2,3,4-tetrahydroquinoli n-8-yl)amino)pyrimid in-5-carboxylate | 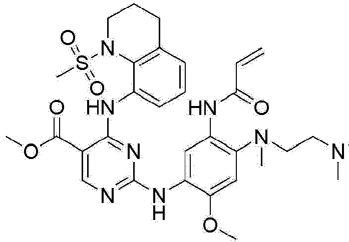 | ¹H NMR (300 MHz, Chloroform-*d*) δ 10.35 (s, 1H), 9.97 (s, 1H), 9.05 (s, 1H), 8.85 (s, 1H), 7.94 (d, *J* = 8.2 Hz, 1H), 7.45 (s, 1H), 7.08 (t, *J* = 8.2 Hz, 1H), 6.88 (d, *J* = 7.6 Hz, 1H), 6.71 (s, 1H), 6.35 (d, *J* = 13.8 Hz, 2H), 5.72 - 5.62 (m, 1H), 3.89 (s, 3H), 3.82 (s, 3H), 3.66 (s, 2H), 3.11 (s, 3H), 2.90 (s, 2H), 2.80 (t, *J* = 7.1 Hz, 2H), 2.68 (s, 3H), 2.58 - 2.05 (m, 10H); LCMS: 653.6 [M+H⁺]. |
| 52/ isopropyl 2-((5-acrylamido-4-((2-(dimethylamino)e thyl)(methyl)amin o)-2-methoxyphenyl)a mino)-4-((1-(methylsulfonyl)-1,2,3,4-tetrahydroquinoli n-8-yl)amino)pyrimid in-5-carboxylate | 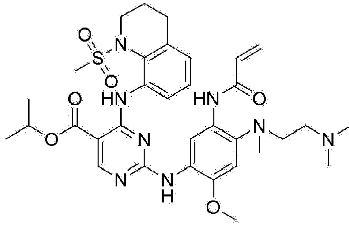 | ¹H NMR (300 MHz, Chloroform-*d*) δ 10.39 (s, 1H), 10.04 (s, 1H), 9.21 (s, 1H), 8.89 (s, 1H), 7.97 (d, *J* = 8.1 Hz, 1H), 7.46 (s, 1H), 7.12 (s, 1H), 6.89 (d, *J* = 7.6 Hz, 1H), 6.73 (s, 1H), 6.38 (d, *J* = 15.6 Hz, 2H), 5.68 (d, *J* = 11.8 Hz, 1H), 5.23 (d, *J* = 6.2 Hz, 1H), 3.83 (s, 3H), 3.66 (s, 2H), 3.14 (s, 3H), 2.92 - 2.77 (m, 4H), 2.69 (s, 3H), 2.49 - 2.06 (m, 10H), 1.35 (d, *J* = 6.3 Hz, 6H); LCMS: 681.8 [M+H⁺]. |
| 53/ *N-*(5-((5-chloro-4-((1-(methylsulfonyl)-1,2,3,4-tetrahydroquinoli n-8-yl)amino)pyrimid in-2-yl)amino)-4-methoxy-2-(methyl(2-morpholinoethyl) amino)phenyl)acr ylamide | 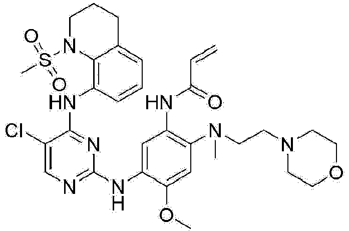 | ¹H NMR (400 MHz, Methanol-d4) δ 8.12 (s, 1H), 7.76 (s, 1H), 7.60 (dd, *J* = 8.1, 1.5 Hz, 1H), 7.23 (t, *J* = 7.8 Hz, 1H), 7.10 (dd, *J* = 7.5, 1.4 Hz, 1H), 6.91 (s, 1H), 6.59 - 6.45 (m, 2H), 5.99 (dd, *J* = 9.0, 2.7 Hz, 1H), 4.10 - 3.99 (m, 4H), 3.96 (s, 3H), 3.61 (s, 2H), 3.53 - 3.47 (m, 2H), 3.29 - 3.10 (m, 6H), 3.02 (s, 3H), 2.82 (t, *J* = 7.0 Hz, 2H), 2.66 (s, 3H), 2.05 (s, 2H). |
| 54/ *N-*(5-((5-chloro-4-((1-(methylsulfonyl)-1,2,3,4-tetrahydroquinoli n-8-yl)amino)pyrimid in-2-yl)amino)-2-((2-(dimethylamino)e thyl)(methyl)amin o)-4-methoxyphenyl)-2-fluoroacrylamide | 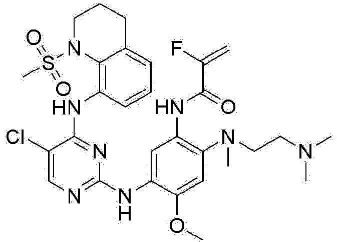 | ¹H NMR (300 MHz, Methanol-*d₄*) δ 8.15 (s, 1H), 7.80 (s, 1H), 7.60 - 7.51 (m, 1H), 7.26 (t, *J* = 7.8 Hz, 1H), 7.12 (d, *J* = 7.6 Hz, 1H), 6.92 (s, 1H), 5.90 (dd, *J* = 46.7, 3.5 Hz, 1H), 5.51 (dd, *J* = 15.1, 3.5 Hz, 1H), 3.96 (s, 3H), 3.61 (s, 2H), 3.45 (t, *J* = 5.8 Hz, 2H), 3.24 (t, *J* = 5.7 Hz, 2H), 3.02 (s, 3H), 2.90 - 2.77 (m, 8H), 2.67 (s, 3H), 2.06 (s, 2H); LCMS: 647.7 [M+H⁺]. |
| 55/ *N-*(5-((5-chloro-4-((1-(methylsulfonyl)i ndolin-7-yl)amino)pyrimid in-2-yl)amino)-2-((2-(dimethylamino)e thyl)(methyl)amin o)-4-methoxyphenyl)a crylamide | 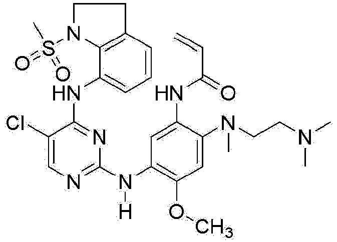 | ¹H NMR (400 MHz, Chloroform-*d*) δ 10.07 (s, 1 H), 9.17 (s, 1 H), 8.88 (s, 1 H), 8.15 (s, 1 H), 8.07 - 8.02 (d, *J* = 8.3 Hz, 1 H), 7.22 (s, 1 H), 7.16 - 7.10 (t, *J* = 7.8 Hz, 1 H), 7.02 - 6.96 (d, *J* = 7.2 Hz, 1 H), 6.75 (s, 1 H), 6.42 - 6.34 (d, *J* = 2.2 Hz, 1 H), 6.34 - 6.26(m, 1 H), 5.72 - 5.65 (m, 1 H), 4.18 - 4.10 (t, *J* = 7.5 Hz, 2 H), 3.84 (s, 3 H), 3.12 - 3.04 (t, *J* = 7.5 Hz, 2 H), 2.94 (s, 3 H), 2.88 (bs, 2 H), 2.70 (s, 3 H), 2.30 (bs, 8 H). LCMS: 615.8 [M+H⁺]. |
| 56/ *N-*(5-((5-chloro-4-((1-(ethylsulfonyl)ind olin-7-yl)amino)pyrimid in-2-yl)amino)-2-((2-(dimethylamino)e thyl)(methyl)amin o)-4-methoxyphenyl)a crylamide | 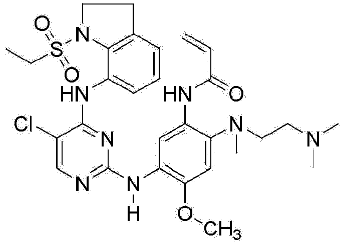 | ¹H NMR (400 MHz, Chloroform-*d*) δ 10.06 (s, 1 H), 9.12 (s, 1 H), 8.83 (s, 1 H), 8.14 (s, 1 H), 7.95 - 7.89 (d, *J* = 8.3 Hz, 1 H), 7.22 (s, 1 H), 7.15 - 7.09 (t, *J* = 7.8 Hz, 1 H), 7.04 - 7.97 (d, *J* = 7.5 Hz, 1 H), 6.72 (s, 1 H), 6.48 - 6.24 (m, 1 H), 5.74 - 5.66 (d, *J* = 11.5 Hz, 1 H), 4.15 - 7.07 (t, *J* = 7.4 Hz, 2 H), 3.84 (s, 3 H), 3.18 - 3.05 (m, 4 H), 3.01 - 2.83 (m, 2 H), 2.71 (s, 3 H), 2.33 (bs, 8 H). LCMS: 629.8 [M+H⁺]. |
| 57/ *N*-(5-((5-chloro-4-((1-(methylsulfonyl)i ndolin-7-yl)amino)pyrimidin-2-yl)amino)-4-methoxy-2-(methyl(2-(methylamino)eth yl)amino)phenyl) acrylamide | 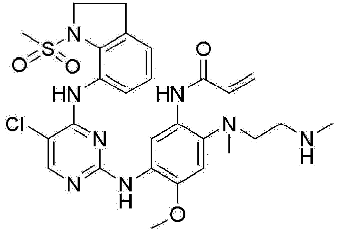 | ¹H NMR (400 MHz, Methanol-*d*₄) δ 8.15 (s, 1 H), 7.92 (s, 1 H), 7.72 (dd, *J* = 5.9, 3.5 Hz, 1 H), 7.23 - 7.17 (m, 2 H), 6.92 (s, 1 H), 6.55 (dd, *J* = 17.0, 9.8 Hz, 1 H), 6.45 (dd, *J* = 17.0, 2.1 Hz, 1H), 5.93 (dd, *J* = 9.9, 2.1 Hz, 1 H), 4.13 (t, *J* = 7.5 Hz, 2 H), 3.96 (s, 3 H), 3.41 (t, *J* = 5.6 Hz, 2 H), 3.22 - 3.11 (m, 4 H), 2.98 (s, 3 H), 2.72 (s, 3 H), 2.68 (s, 3 H). LCMS: 603.8 [M+2H⁺]. |
| 58/ *N-*(5-((5-chloro-4-((1-(methylsulfonyl)i ndolin-7-yl)amino)pyrimid in-2-yl)amino)-2-(4-(dimethylamino)p iperidin-1-yl)-4-methoxyphenyl)a crylamide | 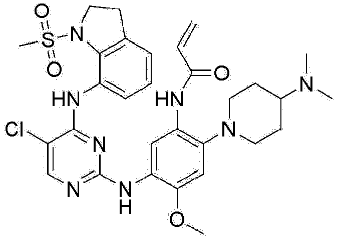 | ¹H NMR (300 MHz, Chloroform-*d*) δ 8.90 (s, 1H), 8.07 (s, 1H), 7.92 (d, *J* = 8.2 Hz, 1H), 7.09 (t, *J* = 7.8 Hz, 1H), 6.98 (d, *J* = 7.3 Hz, 1H), 6.60 (s, 1H), 6.42 - 6.29 (m, 2H), 5.73 (d, *J* = 9.3 Hz, 1H), 4.11 (t, *J* = 7.5 Hz, 2H), 3.85 - 3.77 (m, 3H), 3.07 (q, *J* = 10.4, 7.5 Hz, 4H), 2.90 (s, 3H), 2.83 - 2.63 (m, 9H), 2.17 (d, *J* = 12.0 Hz, 2H), 1.98 (d, *J* = 11.4 Hz, 2H); LCMS: 641.6 [M+H⁺]. |
| 59/ *N-*(5-((5-chloro-4-((1-(methylsulfonyl)i ndolin-7-yl)amino)pyrimid in-2-yl)amino)-4-methoxy-2-(4-(4-methylpiperazin-1-yl)piperidin-1-yl)phenyl)acryla mide | 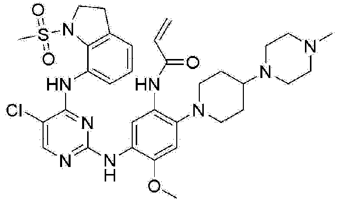 | ¹H NMR (300 MHz, Methanol-*d₄*) δ 8.09 (s, 1H), 8.07 (s, 1H), 7.69 (d, *J* = 7.9 Hz, 1H), 7.21 (d, *J* = 7.2 Hz, 1H), 7.15 (t, *J* = 7.7 Hz, 1H), 6.91 (s, 1H), 6.54 (dd, *J* = 17.0, 10.1 Hz, 1H), 6.33 (d, *J* = 17.0 Hz, 1H), 5.83 (d, *J* = 11.1 Hz, 1H), 4.11 (t, *J* = 7.5 Hz, 2H), 3.89 (s, 3H), 3.29 - 2.99 (m, 10H), 2.96 (s, 3H), 2.85 (s, 6H), 2.08 (d, *J* = 12.5 Hz, 2H), 1.95 - 1.77 (m, 2H); LCMS: 696.7 [M+H⁺]. |
| 60/ methyl 2-((5-acrylamido-4-((2-(dimethylamino)e thyl)(methyl)amin o)-2-methoxyphenyl)a mino)-4-((1-(methylsulfonyl)i ndolin-7-yl)amino)pyrimid in-5-carboxylate | 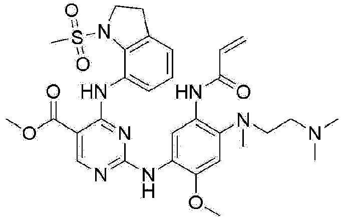 | ¹H NMR (300 MHz, Chloroform-*d*) δ 10.36 (s, 1H), 9.96 (s, 1H), 9.02 (s, 1H), 8.84 (s, 1H), 7.88 (d, *J* = 7.4 Hz, 1H), 7.48 (s, 1H), 7.10 - 6.90 (m, 2H), 6.71 (s, 1H), 6.31 (s, 2H), 5.71 - 5.60 (m, 1H), 4.09 (t, *J* = 7.3 Hz, 2H), 3.89 (s, 3H), 3.82 (s, 3H), 3.05 (s, 3H), 2.92 (d, *J* = 14.0 Hz, 2H), 2.68 (s, 3H), 2.34 (s, 8H), 1.69 (s, 2H); LCMS: 639.6 [M+H⁺]. |
| 61/ isopropyl 2-((5-acrylamido-4-((2-(dimethylamino)e thyl)(methyl)amin o)-2-methoxyphenyl)a mino)-4-((1-(methylsulfonyl)i ndolin-7-yl)amino)pyrimid in-5-carboxylate | 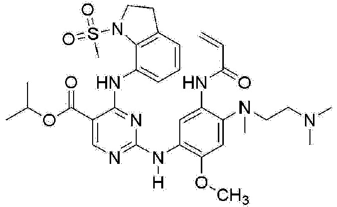 | ¹H NMR (400 MHz, Methanol-*d*₄) δ 8.73 (s, 1 H), 7.90 (s, 1 H), 7.72 (bs, 1 H), 7.23 - 7.11 (m, 2 H), 6.98 (s, 1 H), 6.60 - 6.45 (m, 2 H), 5.95 (dd, *J* = 8.5, 3.2 Hz, 1 H), 5.36 - 5.24 (m, 1 H), 4.09 (t, *J* = 7.4 Hz, 2 H), 3.99 (s, 3 H), 3.49 (t, *J* = 5.7 Hz, 2 H), 3.27 (t, *J* = 5.8 Hz, 2 H), 3.15 (t, *J* = 7.3 Hz, 2 H), 3.04 (s, 3 H), 2.88 (s, 6 H), 2.71 (s, 3 H), 1.42 (d, *J* = 6.2 Hz, 6 H). LCMS: 667.8 [M+H⁺]. |
| 62/ *N-*(5-((5-cyano-4-((1-(methylsulfonyl)i ndolin-7-yl)amino)pyrimid in-2-yl)amino)-2-((2-(dimethylamino)ethyl)(methyl)amin o)-4-methoxyphenyl)a crylamide | 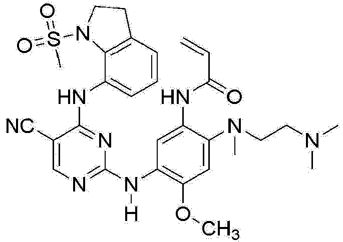 | ¹H NMR (500 MHz, DMSO-*d*₆) δ 10.08 (s, 1 H), 9.12 (bs, 1 H), 8.96 (s, 1 H), 8.48 (s, 1 H), 8.27 (s, 1 H), 7.70 (bs, 1 H), 7.10 - 7.01 (bs, 1 H), 7.00 (s, 1 H), 6.94 - 6.77 (bs, 1 H), 6.40 (dd, *J* = 16.9, 10.1 Hz, 1 H), 6.20 (dd, *J* = 16.9, 2.0 Hz, 1 H), 5.75 (dd, *J* = 10.1, 2.0 Hz, 1 H), 4.09 - 3.97 (m, 2 H), 3.75 (s, 3 H), 3.04 (s, 5 H), 2.87 (t, *J* = 5.8 Hz, 2 H), 2.72 (s, 3 H), 2.32 (t, *J* = 5.8 Hz, 2 H), 2.22 (s, 6 H). LCMS: 606.8 [M+H⁺]. |
| 63/ *N-*(5-((5-chloro-4-((1-(methylsulfonyl)i ndolin-7-yl)amino)pyrimid in-2-yl)amino)-2-(2-(dimethylamino)e thoxy)-4-methoxyphenyl)a crylamide | 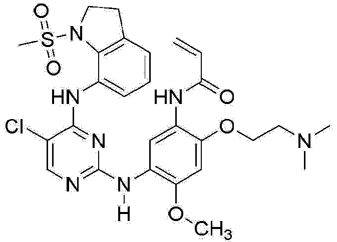 | ¹H NMR (400 MHz, Methanol-*d*₄) δ 8.10 (s, 1 H), 8.01 (s, 1 H), 7.77 - 7.69 (m, 1 H), 7.31 - 7.23 (m, 1 H), 7.16 (d, *J* = 6.8 Hz, 2 H), 6.88 (s, 1 H), 6.53 (dd, *J* = 17.0, 9.9 Hz, 1 H), 6.42 (dd, *J* = 17.0, 1.9 Hz, 1 H), 5.89 (dd, *J* = 9.9, 1.9 Hz, 1 H), 4.56 - 4.48 (m, 2 H), 4.14 (d, *J* = 9.9 Hz, 2 H), 3.96 (s, 3 H), 3.64 - 3.58 (m, 2 H), 3.15 (t, *J* = 7.5 Hz, 2 H), 3.02 (s, 6 H), 2.98 (ds, 3H). LCMS: 603.8 [M+2H⁺]. |
| 64/ *N-*(5-((5-chloro-4-((1-(methylsulfonyl)i ndolin-7-yl)amino)pyrimid in-2-yl)amino)-4-methoxy-2-(methyl(2-morpholinoethyl) amino)phenyl)acr ylamide | 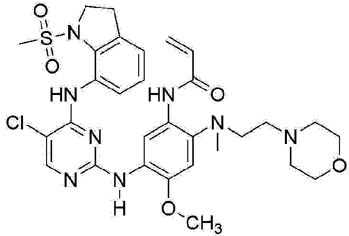 | ¹H NMR (400 MHz, Methanol-*d*₄) δ 8.15 (s, 1 H), 7.88 (s, 1 H), 7.74 (dd, *J* = 6.1, 3.3 Hz, 1 H), 7.24 - 7.17 (m, 2 H), 6.96 (s, 1 H), 6.62 - 6.51 (m, 2 H), 6.00 (dd, *J* = 9.5, 2.2 Hz, 1 H), 4.13 (t, *J* = 7.5 Hz, 2 H), 4.06 (t, *J* = 4.9 Hz, 4 H), 3.98 (s, 3 H), 3.52 (t, *J* = 5.6 Hz, 2 H), 3.32 - 3.22 (t, *J* = 5.6 Hz, 6 H), 3.21 - 3.14 (m, 3 H), 2.98 (s, 3 H), 2.69 (s, 3 H). LCMS: 657.8 [M+H⁺]. |
| 65/ *N*-(2-((2-(dimethylamino)e thyl)(methyl)amin o)-4-methoxy-5-((4-((1-(methylsulfonyl)i ndolin-7-yl)amino)-5-(trifluoromethyl)p yrimidin-2-yl)amino)phenyl) acrylamide | 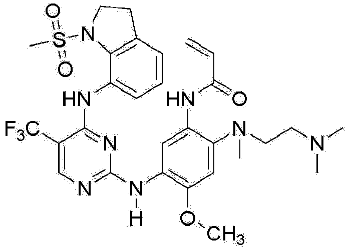 | ¹H NMR (400 MHz, Methanol-*d₄*) δ 8.40 (s, 1 H), 7.80 (dd, *J* = 4.6, 2.3 Hz, 1 H), 7.59 (s, 1 H ), 7.29 - 7.13 (m, 2 H), 7.01 - 6.90 (m, 1 H), 6.60 - 6.44 (m, 2 H), 5.96 (d, *J* = 8.1 Hz, 1 H), 4.16 - 4.00 (m, 2 H), 3.87 (m, 3 H), 3.47 (d, *J* = 5.9 Hz, 2 H), 3.25 (s, 2 H), 3.15 (d, *J* = 5.8 Hz, 3 H), 3.20 - 3.10 (m, 2 H), 2.86 (s, 6 H), 2.69 (s, 3 H). LCMS: 649.8 [M+H⁺]. |
| 66/ *N-*(5-((5-chloro-4-((1-(methylsulfonyl)i ndolin-7-yl)amino)pyrimid in-2-yl)amino)-2-((2-(dimethylamino)e thyl)(methyl)amin o)-4-methoxyphenyl)-2-fluoroacrylamide | 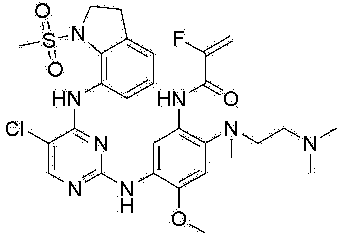 | ¹H NMR (300 MHz, Methanol-*d₄*) δ 8.14 (s, 1H), 7.92 (s, 1H), 7.67 (dd, *J* = 6.9, 2.4 Hz, 1H), 7.28 - 7.15 (m, 2H), 6.94 (s, 1H), 5.88 (dd, *J* = 46.7, 3.5 Hz, 1H), 5.50 (dd, *J* = 15.1, 3.5 Hz, 1H), 4.12 (t, *J* = 7.5 Hz, 2H), 3.97 (s, 3H), 3.46 (t, *J* = 5.8 Hz, 2H), 3.25 (t, *J* = 5.8 Hz, 2H), 3.16 (t, *J* = 7.5 Hz, 2H), 2.96 (s, 3H), 2.86 (s, 7H), 2.68 (s, 3H); LCMS: 633.7 [M+H⁺]. |
| 67/ *N-*(5-((5-chloro-4-((1-(methylsulfonyl)-1H-indol-7-yl)amino)pyrimidin-2-yl)amino)-2-((2-(dimethylamino)e thyl)(methyl)amin o)-4-methoxyphenyl)a crylamide | 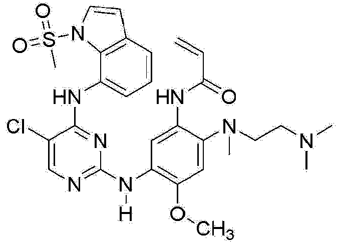 | ¹H NMR (400 MHz, Methanol-*d₄*) δ 8.19 (s, 1 H), 7.64 (s, 1 H), 7.62 (d, *J* = 3.7 Hz, 1 H), 7.60 (dd, *J* = 7.8, 1.2 Hz, 1 H), 7.52 (dd, *J* = 7.8, 1.1 Hz, 1 H), 7.34 (t, *J* = 7.8 Hz, 1 H), 6.88 (s, 1 H), 6.81 (d, *J* = 3.8 Hz, 1 H), 6.57 - 6.47 (m, 2 H), 5.98 (dd, *J* = 8.6, 3.2 Hz, 1 H), 3.94 (s, 3H ), 3.43 (t, *J* = 5.7 Hz, 2H), 3.21 (t, *J* = 5.7 Hz, 2 H), 2.83 (s, 6 H), 2.64 (s, 3 H). LCMS: 613.8 [M+H⁺]. |
| 68/ *N-*(5-((5-chloro-4-((5-(N-methylmethylsulf onamido)quinoxal in-6-yl)amino)pyrimid in-2-yl)amino)-2-((2-(dimethylamino)e thyl)(methyl)amin o)-4-methoxyphenyl)a crylamide | 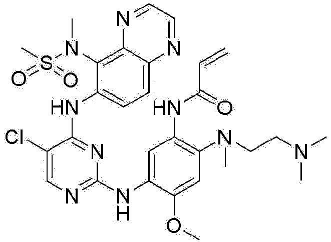 | ¹H NMR (500 MHz, Chloroform-*d*) δ 9.23 (s, 1H), 8.86 (d, *J* = 9.4 Hz, 1H), 8.81 (s, 1H), 8.76 (d, *J* = 1.8 Hz, 1H), 8.75 (d, *J* = 1.9 Hz, 1H), 8.22 (s, 1H), 7.95 (d, *J* = 9.4 Hz, 1H), 7.36 (s, 1H), 6.73 (s, 1H), 5.91 (d, *J* = 16.7 Hz, 1H), 5.39 (d, *J* = 10.4 Hz, 1H), 3.86 (s, 3H), 3.54 (s, 3H), 3.15 (s, 3H), 2.69 (s, 3H), 2.35 (s, 6H); LCMS: 655.5 [M+H⁺]. |
| 69/ *N-*(6-((5-chloro-2-((4-((2-(dimethylamino)e thyl)(methyl)amin o)-2-methoxyphenyl)a mino)pyrimidin-4-yl)amino)quinoxa lin-5-yl)methanesulfonamide | 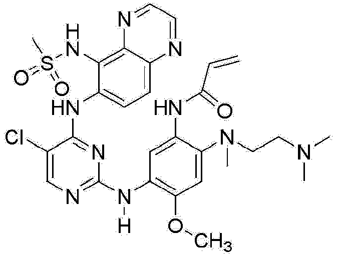 | ¹H NMR (500 MHz, Chloroform-*d*) δ 10.01 (s, 1 H), 9.18 (s, 1 H), 9.09 (s, 1 H), 8.83 (s, 1 H), 8.82 - 8.69 (m, 2 H), 8.23 (s, 1 H), 7.97 - 7.88 (d, *J* = 9.5 Hz, 1 H), 7.34 (s, 1 H), 6.73 (s, 1 H), 6.25 - 5.85 (m, 2 H), 5.50 - 5.42 (d, *J* = 10.2 Hz, 1 H), 3.86 (s, 3 H), 2.97 (s, 3 H), 2.72 (s, 5 H), 2.26 (s, 8 H). LCMS: 642.2 [M+2H⁺]. |
| 70/ *N-*(5-((5-chloro-4-((2-(1,1-dioxidoisothiazoli din-2-yl)phenyl)amino) pyrimidin-2-yl)amino)-2-((2-(dimethylamino)e thyl)(methyl)amin o)-4-methoxyphenyl)a crylamide | 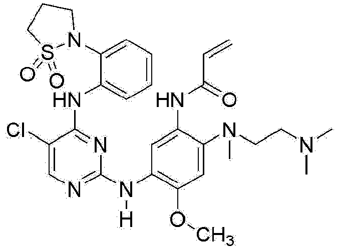 | ¹H NMR (400 MHz, Chloroform-*d*) δ 10.08 (s, 1 H), 9.20 (s, 1 H), 8.40 - 8.34 (d, *J* = 8.3 Hz, 1 H), 8.33 (s, 1 H), 8.17 (s, 1 H), 7.46 - 7.39 (dd, *J* = 7.9, 1.6 Hz, 1 H), 7.37 - 7.29 (t, *J* = 8.5 Hz, 1 H), 7.14 - 7.06 (t, *J* = 7.7 Hz, 1 H), 6.75 (s, 1 H), 6.49 - 6.26 (m, 1 H), 5.74 - 5.66 (m, 1 H), 3.87 (s, 3 H), 3.76 - 3.70 (t, *J* = 6.7 Hz, 2 H), 3.48 - 3.42 (m, 2 H), 3.04 - 2.84 (m, 2 H), 2.64 - 2.54 (m, 2 H), 2.34 (bs, 8 H). LCMS: 615.8 [M+H⁺]. |
| 71/ *N-*(5-((5-chloro-4-((2-(1,1-dioxido-1,2-thiazinan-2-yl)phenyl)amino) pyrimidin-2-yl)amino)-2-((2-(dimethylamino)e thyl)(methyl)amin o)-4-methoxyphenyl)a crylamide | 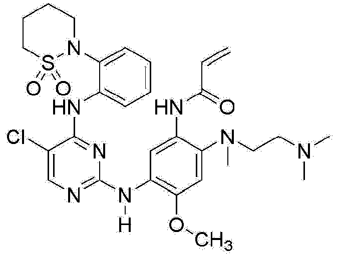 | ¹H NMR (400 MHz, Chloroform-*d*) δ 10.09 (s, 1 H), 9.23 (s, 1 H), 8.48 - 8.42 (d, *J* = 8.2 Hz, 1 H), 8.22 (s, 1 H), 8.18 (s, 1 H), 7.46 - 7.41 (dd, *J* = 7.9, 1.5 Hz, 1 H), 7.34 - 7.39 (m, 1 H), 7.10 - 7.03 (t, *J* = 7.7 Hz, 1 H), 6.76 (s, 1 H), 6.49 - 6.23 (m, 1 H), 5.75 - 5.65 (m, 1 H), 3.95 - 3.83 (m, 4 H), 3.49 - 3.23 (m, 3 H), 2.92 (m, 2 H), 2.72 (s, 3 H), 2.53 - 2.19 (m, 10 H), 2.03 - 1.89 (m, 2 H). LCMS: 629.8 [M+H⁺]. |
| 72/ *N-*(5-((5-chloro-4-((1-(methylsulfonyl)i ndolin-7-yl)amino)pyrimid in-2-yl)amino)-4-methoxy-2-(methyl(2-(piperidin-1-yl)ethyl)amino)ph enyl)acrylamide | 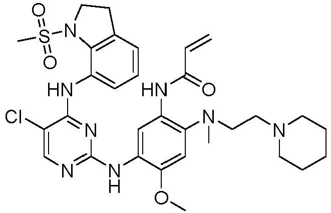 | ¹H NMR (400 MHz, DMSO-*d*₆) δ 9.34 (s, 1 H), 8.92 (s, 1 H), 8.22 (s, 1 H), 8.17 - 8.06 (m, 2 H), 7.89 - 7.79 (m, 1 H), 7.08 - 6.95 (m, 2 H), 6.92 (s, 1 H), 6.58 (dd, *J* = 17.0, 10.2 Hz, 1 H), 6.17 (dd, *J* = 16.9, 2.0 Hz, 1 H), 5.79 - 5.67 (m, 1 H), 4.03 (t, *J* = 7.4 Hz, 2 H), 3.77 (s, 3 H), 3.12 - 2.98 (m, 5 H), 2.94 (t, *J* = 6.6 Hz, 2 H), 2.67 (s, 3 H), 2.39 - 2.23 (m, 6 H), 1.57 - 1.43 (m, 4 H), 1.43 - 1.33 (m, 2 H); LCMS: 655.5 [M+H⁺]. |
| 73/ *N-*(5-((5-chloro-4-((1-(methylsulfonyl)i ndolin-7-yl)amino)pyrimid in-2-yl)amino)-4-methoxy-2-(methyl(2-(4-methylpiperazin-1-yl)ethyl)amino)ph enyl)acrylamide | 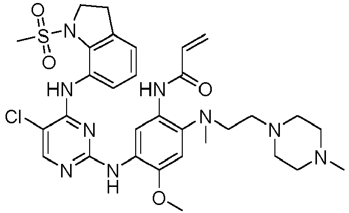 | ¹H NMR (300 MHz, Methanol-*d*₄) δ 8.14 (s, 1 H), 7.99 (s, 1 H), 7.72 (d, *J* = 7.7 Hz, 1 H), 7.25 - 7.12 (m, 2 H), 7.01 (s, 1 H), 6.58 (dd, *J* = 17.0, 9.8 Hz, 1 H), 6.45 (dd, *J* = 17.0, 1.9 Hz, 1 H), 5.97 (dd, *J* = 9.8, 1.9 Hz, 1 H), 4.12 (t, *J* = 7.5 Hz, 2 H), 3.95 (s, 3 H), 3.45 (t, *J* = 5.9 Hz, 2 H), 3.40 - 3.33 (m, 4 H), 3.24 - 3.09 (m, 6 H), 3.09 - 3.01 (m, 2 H), 2.97 (s, 3 H), 2.86 (s, 3 H), 2.81 (s, 3 H); LCMS: 670.5 [M+H⁺]. |
| 74/ *N-*(5-((5-chloro-4-((1-(methylsulfonyl)i ndolin-7-yl)amino)pyrimidin-2-yl)amino)-4-methoxy-2-(methyl(2-(pyrrolidin-1-yl)ethyl)amino)ph enyl)acrylamide | 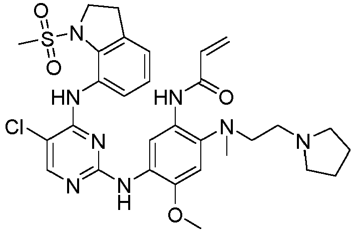 | ¹H NMR (300 MHz, Methanol-*d*₄) δ 8.15 (s, 1 H), 7.81 (s, 1 H), 7.74 - 7.65 (m, 1 H), 7.23 - 7.14 (m, 2 H), 6.92 (s, 1 H), 6.53 (dd, *J* = 16.9, 9.7 Hz, 1 H), 6.42 (dd, *J* = 16.9, 2.2 Hz, 1 H), 5.94 (dd, *J* = 9.6, 2.2 Hz, 1 H), 4.11 (t, *J* = 7.5 Hz, 2 H), 3.96 (s, 3 H), 3.61 - 3.49 (m, 2 H), 3.46 (t, *J* = 5.6 Hz, 2 H), 3.15 (t, *J* = 7.5 Hz, 2 H), 3.11 - 2.98 (m, 2 H), 2.96 (s, 3 H), 2.68 (s, 3 H), 2.24 - 2.09 (m, 4 H); LCMS: 641.5 [M+H⁺]. |
| 75/ *N-*(5-((5-chloro-4-((1-(ethylsulfonyl)ind olin-7-yl)amino)pyrimid in-2-yl)amino)-4-methoxy-2-(methyl(2-(pyrrolidin-1-yl)ethyl)amino)ph enyl)acrylamide | 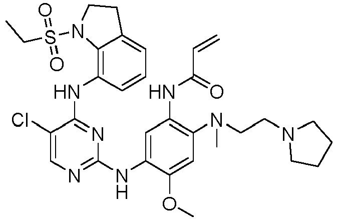 | ¹H NMR (300 MHz, Methanol-*d₄*) δ 8.14 (s, 1 H), 7.83 (s, 1 H), 7.68 - 7.61 (m, 1 H), 7.20 - 7.13 (m, 2 H), 6.91 (s, 1 H), 6.53 (dd, *J* = 16.9, 9.5 Hz, 1 H), 6.42 (dd, *J* = 17.0, 2.3 Hz, 1 H), 5.94 (dd, *J* = 9.6, 2.3 Hz, 1 H), 4.07 (t, *J* = 7.5 Hz, 2 H), 3.96 (s, 3 H), 3.59 - 3.48 (m, 2 H), 3.45 (t, *J* = 5.5 Hz, 2 H), 3.25 - 3.10 (m, 4 H), 3.10 - 2.97 (m, 2 H), 2.67 (s, 3 H), 2.22 - 2.09 (m, 4 H), 1.30 (t, *J* = 7.4 Hz, 3 H); LCMS: 655.7 [M+H⁺]. |
| 76/ *N-*(5-((5-chloro-4-((4-methyl-2-(*N-*methylmethylsulf onamido)phenyl)a mino)pyrimidin-2-yl)amino)-4-methoxy-2-(methyl(2-(pyrrolidin-1-yl)ethyl)amino)ph enyl)acrylamide | 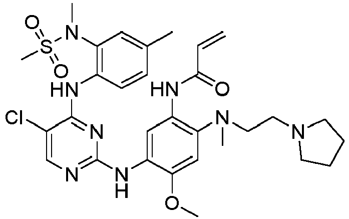 | ¹H NMR (400 MHz, DMSO-*d*₆) δ 9.77 (s, 1 H), 8.38 (s, 1 H), 8.24 (s, 1 H), 8.21 (s, 1 H), 8.13 (s, 1 H), 8.08 (d, *J* = 8.4 Hz, 1 H), 7.37 (s, 1 H), 7.03 - 6.90 (m, 2 H), 6.43 (d, *J* = 14.3 Hz, 1 H), 6.19 (d, *J* = 16.9 Hz, 1 H), 5.74 (d, *J* = 10.2 Hz, 1 H), 3.77 (s, 3 H), 3.16 (s, 3 H), 3.09 (s, 3 H), 3.01 - 2.89 (m, 2 H), 2.70 (s, 3 H), 2.26 (s, 3 H), 1.83 - 1.62 (m, 4 H); LCMS: 643.6 [M+H⁺]. |
| 77/ *N-*(5-((5-chloro-4-((1-(methylsulfonyl)i ndolin-7-yl)amino)pyrimid in-2-yl)amino)-4-methoxy-2-(2-morpholinoethox y)phenyl)acrylam ide | 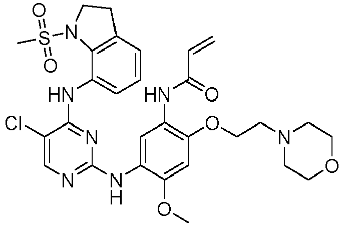 | 1H NMR (300 MHz, DMSO-d6) δ 9.14 (s, 1 H), 8.92 (s, 1 H), 8.10 (s, 2 H), 8.03 (s, 1 H), 7.90 - 7.80 (m, 1 H), 7.10 - 6.99 (m, 2 H), 6.84 (s, 1 H), 6.58 (dd, J = 17.0, 10.2 Hz, 1 H), 6.17 (dd, J = 17.0, 2.1 Hz, 1 H), 5.71 (dd, J = 10.1, 2.1 Hz, 1 H), 4.20 (t, J = 5.8 Hz, 2 H), 4.04 (t, J = 7.4 Hz, 2 H), 3.80 (s, 3 H), 3.63 - 3.54 (m, 4 H), 3.17 - 2.96 (m, 5 H), 2.79 - 2.66 (m, 2 H), 2.55 - 2.45 (m, 4 H). LCMS: 648.7 [M+H⁺]. |
| 78/ *N-*(5-((5-chloro-4-((1-(methylsulfonyl)i ndolin-7-yl)amino)pyrimid in-2-yl)amino)-4-methoxy-2-(2-(piperidin-1-yl)ethoxy)phenyl) acrylamide | 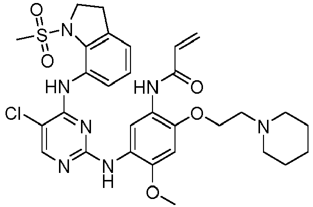 | 1H NMR (300 MHz, DMSO-d6) δ 9.17 (s, 1 H), 8.91 (s, 1 H), 8.10 (s, 2 H), 8.06 (s, 1 H), 7.89 - 7.77 (m, 1 H), 7.03 (d, J = 4.3 Hz, 2 H), 6.85 (s, 1 H), 6.58 (dd, J = 17.0, 10.2 Hz, 1 H), 6.17 (dd, J = 17.0, 2.1 Hz, 1 H), 5.71 (dd, J = 10.2, 2.1 Hz, 1 H), 4.18 (t, J = 5.9 Hz, 2 H), 4.04 (t, J = 7.0 Hz, 2 H), 3.79 (s, 3 H), 3.13 - 2.98 (m, 5 H), 2.66 (t, J = 5.9 Hz, 2 H), 2.48 - 2.33 (m, 4 H), 1.51 (d, J = 4.9 Hz, 4 H), 1.46 - 1.31 (m, 2 H). LCMS: 643.2 [M+H⁺]. |
| 79/ *N-*(5-((5-chloro-4-((1-(methylsulfonyl)i ndolin-7-yl)amino)pyrimidin-2-yl)amino)-4-methoxy-2-(2-(pyrrolidin-1-yl)ethoxy)phenyl) acrylamide | 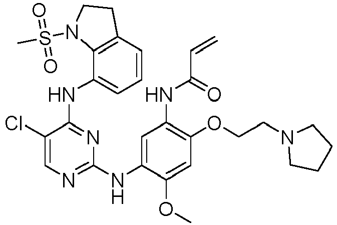 | 1H NMR (300 MHz, DMSO-d6) δ 9.40 (s, 1 H), 8.92 (s, 1 H), 8.16 - 8.07 (m, 3 H), 7.84 (d, J = 7.4 Hz, 1 H), 7.08 - 6.96 (m, 2 H), 6.87 (s, 1 H), 6.49 (dd, J = 16.9, 10.1 Hz, 1 H), 6.17 (dd, J = 17.0, 2.1 Hz, 1 H), 5.72 (dd, J = 10.1, 2.1 Hz, 1 H), 4.19 (t, J = 5.7 Hz, 2 H), 4.04 (t, J = 7.4 Hz, 2 H), 3.78 (s, 3 H), 3.12 - 2.98 (m, 5 H), 2.82 - 2.71 (m, 2 H), 2.63 - 2.53 (m, 4 H), 1.79 - 1.68 (m, 4 H). LCMS: 629.2 [M+H⁺]. |
| 80/ *N*-(2-((2-(azetidin-1-yl)ethyl)(methyl)a mino)-5-((5-chloro-4-((1-(methylsulfonyl)i ndolin-7-yl)amino)pyrimid in-2-yl)amino)-4-methoxyphenyl)a crylamide | 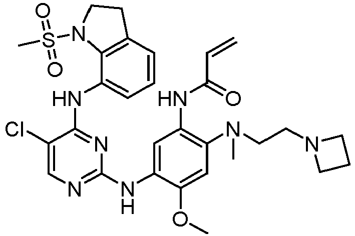 | 1H NMR (400 MHz, Methanol-d4) δ 13.11 (s, 1 H), 10.06 (s, 1 H), 9.57 (s, 1 H), 9.20 (s, 1 H), 8.70 (s, 1 H), 7.90 (s, 1 H), 7.73 (d, J = 8.2 Hz, 1 H), 7.13 - 6.90 (m, 3 H), 6.69 (s, 1 H), 6.31 (dd, J = 16.9, 2.1 Hz, 1 H), 5.70 (dd, J = 10.1, 2.1 Hz, 1 H), 4.36 (bs, 2 H), 4.09 (t, J = 7.5 Hz, 2 H), 3.81 (s, 3 H), 3.78 - 3.66 (m, 2 H), 3.21 (bs, 2 H), 3.17 - 2.99 (m, 4 H), 2.88 (s, 3 H), 2.67 (q, J = 9.7 Hz, 1 H), 2.55 (s, 3 H), 2.32 (bs, 1 H). LCMS: 628.0 [M+H⁺]. |
| 81/ *N*-(2-((2-(azetidin-1-yl)ethyl)(methyl)a mino)-5-((5-chloro-4-((1-(ethylsulfonyl)ind olin-7-yl)amino)pyrimid in-2-yl)amino)-4-methoxyphenyl)a crylamide | 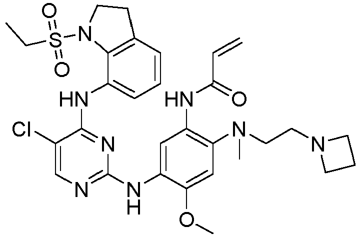 | 1H NMR (400 MHz, Methanol-d4) δ 13.11 (s, 1 H), 10.26 (s, 1 H), 9.62 (s, 1 H), 9.19 (s, 1 H), 8.66 (s, 1 H), 7.88 (s, 1 H), 7.65 (d, J = 8.2 Hz, 1 H), 7.14 - 6.87 (m, 3 H), 6.69 (s, 1 H), 6.32 (dd, J = 16.8, 2.1 Hz, 1 H), 5.71 (dd, J = 10.1, 2.1 Hz, 1 H), 4.36 (bs, 2 H), 4.05 (t, J = 7.5 Hz, 2 H), 3.81 (s, 3 H), 3.77 - 3.65 (m, 2 H), 3.22 (s, 2 H), 3.15 - 2.99 (m, 6 H), 2.67 (q, J = 9.8, 9.2 Hz, 1 H), 2.55 (s, 3 H), 2.39 - 2.25 (m, 1 H), 1.36 (t, J = 7.4 Hz, 3 H). LCMS: 641.7 [M+H⁺]. |
| 82/ *N*-(2-((2-(azetidin-1-yl)ethyl)(methyl)a mino)-5-((5-chloro-4-((4-methyl-2-(*N-*methylmethylsulf onamido)phenyl)a mino)pyrimidin-2-yl)amino)-4-methoxyphenyl)a crylamide | 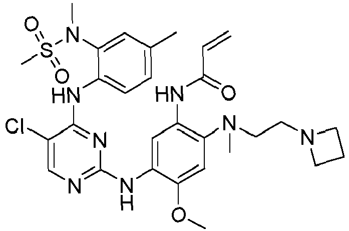 | 1H NMR (400 MHz, DMSO-*d*₆) δ 10.20 (s, 1 H), 8.48 (s, 1 H), 8.28 (s, 1 H), 8.20 (s, 1 H), 8.12 (s, 1 H), 8.08 (d, J = 8.4 Hz, 1 H), 7.37 (d, J = 1.9 Hz, 1 H), 6.99 - 6.89 (m, 2 H), 6.66 (dd, J = 17.0, 10.2 Hz, 1 H), 6.25 (dd, J = 16.7, 2.0 Hz, 1 H), 5.81 (dd, J = 10.0, 2.0 Hz, 1 H), 3.76 (s, 3 H), 3.21 (t, *J* = 7.0 Hz, 4 H), 3.17 (s, 3 H), 3.10 (s, 3 H),, 2.73 (t, *J* = 5.8 Hz, 2 H), 2.69 (s, 3 H),, 2.51 - 2.45 (m, 2 H), 2.26 (s, 3 H), 2.04 (t, J = 7.0 Hz, 2 H). LCMS: 629.7 [M+H⁺]. |
| 83/ *N-*(5-((5-chloro-4-((3,4-dimethyl-2-(*N-*methylacetamido) phenyl)amino)pyr imidin-2-yl)amino)-2-((2-(dimethylamino)e thyl)(methyl)amin o)-4-methoxyphenyl)a crylamide | 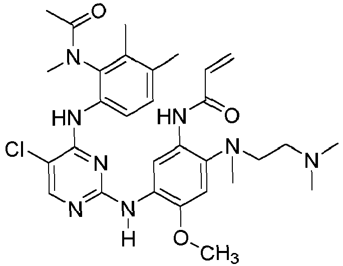 | ¹H NMR (500 MHz, DMSO-d6) δ 10.06 (s, 1 H), 8.42 (s, 1 H), 8.09 (s, 1 H), 8.04 (s, 1 H), 7.85 (s, 1 H), 7.72 (d, J = 8.3 Hz, 1 H), 7.06 (d, J = 8.4 Hz, 1 H), 6.95 (s, 1 H), 6.39 (dd, J = 16.8, 10.2 Hz, 1 H), 6.23 - 6.15 (m, 1 H), 5.80 - 5.71 (m, 1 H), 3.75 (s, 3 H), 2.99 (d, J = 2.5 Hz, 3 H), 2.87 (s, 2 H), 2.70 (s, 3 H), 2.31 (s, 2 H), 2.26 - 2.15 (m, 11 H), 2.04 (s, 3 H), 1.63 (s, 3 H). LCMS: 596.2 [M+H⁺]. |
| 84/ *N*-(5-((4-((2-acetamido-3,4-dimethylphenyl)a mino)-5-chloropyrimidin-2-yl)amino)-2-((2-(dimethylamino)e thyl)(methyl)amin o)-4-methoxyphenyl)a crylamide | 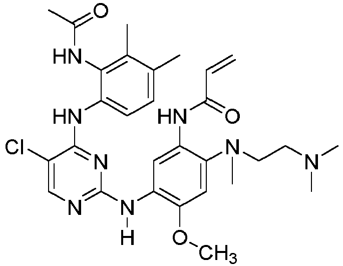 | ¹H NMR (300 MHz, DMSO-d6) δ 10.09 (s, 1 H), 9.59 (s, 1 H), 8.46 (s, 1 H), 8.08 (s, 1 H), 7.99 (s, 1 H), 7.87 (s, 1 H), 7.62 (d, J = 8.3 Hz, 1 H), 6.96 (s, 1 H), 6.93 (d, J = 8.3 Hz, 1 H), 6.41 (dd, J = 16.9, 10.0 Hz, 1 H), 6.21 (dd, J = 17.2, 2.2 Hz, 1 H), 5.80 - 5.71 (m, 1 H), 3.77 (s, 3 H), 2.86 (s, 2 H), 2.69 (s, 3 H), 2.31 (s, 2 H), 2.22 (s, 6 H), 2.18 (s, 3 H), 2.11 (s, 3 H), 2.08 (s, 3 H). LCMS: 382.8 [M+H⁺]. |
| 85/ *N-*(5-((5-chloro-4-((3-methyl-2-(*N-*methylacetamido) phenyl)amino)pyr imidin-2-yl)amino)-2-((2-(dimethylamino)e thyl)(methyl)amin o)-4-methoxyphenyl)a crylamide | 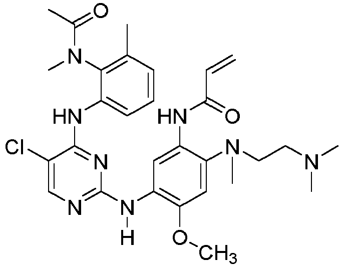 | ¹H NMR (500 MHz, DMSO-d6) δ 10.05 (s, 1 H), 8.36 (s, 1 H), 8.14 (s, 1 H), 8.11 (d, J = 2.2 Hz, 1 H), 7.90 (s, 1 H), 7.85 (d, J = 8.0 Hz, 1 H), 7.14 (d, J = 8.2 Hz, 1 H), 7.09 (d, J = 7.7 Hz, 1 H), 6.95 (s, 1 H), 6.43 - 6.34 (m, 1 H), 6.20 (d, J = 16.8 Hz, 1 H), 5.75 (t, J = 9.4 Hz, 1 H), 3.75 (s, 3 H), 3.01 (s, 3 H), 2.87 (s, 3 H), 2.70 (s, 3 H), 2.31 (s, 2 H), 2.22 (s, 6 H), 2.15 (s, 3 H), 1.64 (s, 3 H). LCMS: 581.8 [M+H⁺]. |
| 86/ *N*-(5-((4-((2-acetamido-3-methylphenyl)ami no)-5-chloropyrimidin-2-yl)amino)-2-((2-(dimethylamino) ethyl)(methyl)ami no)-4-methoxyphenyl)a crylamide | 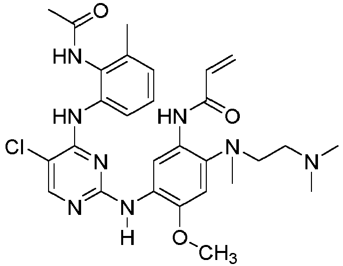 | ¹H NMR (400 MHz, DMSO-d6) δ 10.02 (s, 1 H), 9.60 (s, 1 H), 8.39 (s, 1H), 8.10 (s, 1 H), 8.03 (s, 1 H), 7.98 (s, 1 H), 7.72 (d, J = 8.0 Hz, 1 H), 7.01 (t, J = 7.9 Hz, 1 H), 6.97 - 6.90 (m, 2 H), 6.44 (s, 1 H), 6.22 (d, J = 16.3 Hz, 1H), 5.75 (d, J = 8.6 Hz, 2 H), 3.77 (s, 3 H), 2.90 (s, 2 H), 2.67 (s, 3 H), 2.27 (s, 6 H), 2.20 (s, 3 H), 2.12 (s, 3 H). LCMS: 568.0 [M+H⁺]. |
| 87/ *N-*(5-((5-chloro-4-((4-methyl-2-(*N-*methylacetamido) phenyl)amino)pyr imidin-2-yl)amino)-2-((2-(dimethylamino)e thyl)(methyl)amin o)-4-methoxyphenyl)a crylamide | 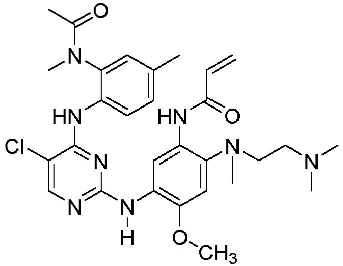 | ¹H NMR (300 MHz, DMSO-d6) δ 10.04 (s, 1 H), 8.46 (s, 1 H), 8.21 (s, 1 H), 8.07 (s, 1 H), 7.85 (s, 1 H), 7.61 (d, J = 8.2 Hz, 1 H), 7.15 (s, 1 H), 7.07 (d, J = 8.4 Hz, 1 H), 6.92 (s, 1 H), 6.36 (d, J = 9.9 Hz, 1 H), 6.26 - 6.15 (m, 1 H), 5.74 (d, J = 11.0 Hz, 1 H), 3.75 (s, 3 H), 2.99 (d, J = 3.0 Hz, 3 H), 2.85 (s, 2 H), 2.69 (s, 3 H), 2.29 (s, 5 H), 2.21 (s, 6 H), 1.73 (s, 3 H). LCMS: 581.8 [M+H⁺]. |
| 88/ *N*-(5-((4-((2-acetamido-4-methylphenyl)ami no)-5-chloropyrimidin-2-yl)amino)-2-((2-(dimethylamino) ethyl)(methyl)ami no)-4-methoxyphenyl)a crylamide | 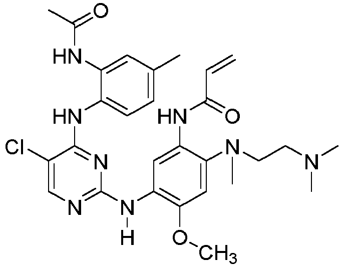 | ¹H NMR (400 MHz, DMSO-d6) δ 10.07 (s, 1 H), 9.92 (s, 1 H), 8.45 (s, 1 H), 8.35 (s, 1 H), 8.06 (s, 1 H), 7.89 (s, 1 H), 7.61 (d, J = 8.3 Hz, 1 H), 7.01 (s, 1 H), 6.94 (s, 1 H), 6.88 (d, J = 8.5 Hz, 1 H), 6.40 (dd, J = 16.9, 10.1 Hz, 1 H), 6.22 (dd, J = 17.0, 2.1 Hz, 1 H), 5.77 - 5.72 (m, 1 H), 3.76 (s, 3 H), 2.86 (s, 2 H), 2.68 (s, 3 H), 2.32 (d, J = 8.8 Hz, 2 H), 2.23 (s, 9 H), 2.07 (s, 3 H). LCMS: 568.0 [M+H⁺]. |
| 89/ *N-*(5-((5-chloro-4-((2-(*N-*methylacetamido) phenyl)amino)pyr imidin-2-yl)amino)-2-((2-(dimethylamino) ethyl)(methyl)ami no)-4-methoxyphenyl)a crylamide | 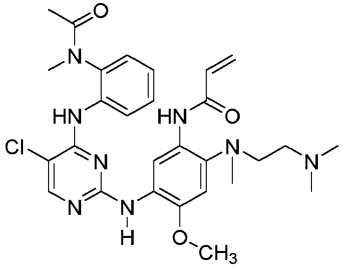 | ¹H NMR (400 MHz, DMSO-d6) δ 10.03 (s, 1 H), 8.39 (s, 1 H), 8.25 (s, 1 H), 8.09 (s, 1 H), 7.94 (s, 1 H), 7.78 (d, J = 7.9 Hz, 1 H), 7.33 (dd, J = 7.4, 1.8 Hz, 1H), 7.29 - 7.18 (m, 2 H), 6.92 (s, 1 H), 6.38 (dd, J = 17.0, 10.0 Hz, 1 H), 6.25 - 6.17 (m, 1 H), 5.79 - 5.71 (m, 1 H), 3.75 (s, 3 H), 3.00 (s, 3 H), 2.89 - 2.82 (m, 2 H), 2.68 (d, J = 4.4 Hz, 3 H), 2.38 - 2.17 (m, 8 H), 1.72 (s, 3 H). LCMS: 567.8 [M+H⁺]. |
| 90/ *N*-(5-((4-((2-acetamidophenyl) amino)-5-chloropyrimidin-2-yl)amino)-2-((2-(dimethylamino) ethyl)(methyl)ami no)-4-methoxyphenyl)a crylamide | 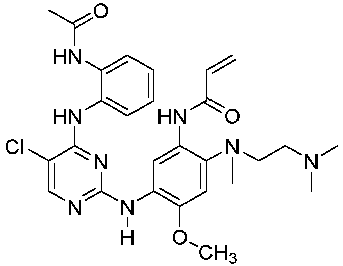 | ¹H NMR (400 MHz, DMSO-d6) δ 10.08 (s, 1 H), 10.00 (s, 1 H), 8.43 (s, 1 H), 8.41 (s, 1 H), 8.08 (s, 1 H), 7.96 (s, 1 H), 7.74 (dd, J = 6.0, 3.7 Hz, 1 H), 7.20 (dd, J = 5.9, 3.6 Hz, 1 H), 7.13 - 7.04 (m, 2 H), 6.93 (s, 1 H), 6.40 (dd, J = 17.0, 10.1 Hz, 1 H), 6.22 (dd, J = 17.0, 2.1 Hz, 1 H), 5.75 (d, J = 9.3 Hz, 1 H), 3.75 (s, 3 H), 2.84 (d, J = 6.6 Hz, 2 H), 2.68 (s, 3 H), 2.29 (s, 2 H), 2.22 (s, 6 H), 2.09 (s, 3 H). LCMS: 554.2 [M+H⁺]. |
| 91/ *N-*(5-((4-((1-acetylindolin-7-yl)amino)-5-chloropyrimidin-2-yl)amino)-2-((2-(dimethylamino)e thyl)(methyl)amin o)-4-methoxyphenyl)a crylamide | 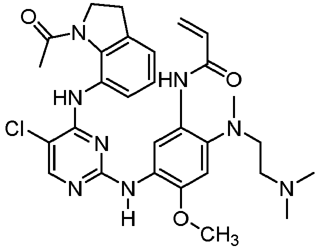 | ¹H NMR (400 MHz, DMSO-d6) δ 10.12 (s, 1 H), 9.77 (s, 1 H), 8.40 (s, 1 H), 8.06 (s, 1 H), 8.00 (s, 1 H), 7.68 - 7.61 (m, 1 H), 6.95 (s, 1 H), 6.92 (d, J = 4.7 Hz, 2 H), 6.38 (dd, J = 16.9, 10.1 Hz, 1 H), 6.19 (dd, J = 17.0, 2.1 Hz, 1H), 5.74 (dd, J = 10.0, 2.1 Hz, 1 H), 4.10 (t, J = 7.7 Hz, 2 H), 3.76 (s, 3 H), 3.01 (t, J = 7.7 Hz, 2 H), 2.84 (t, J = 5.8 Hz, 2 H), 2.69 (s, 3 H), 2.35 - 2.25 (m, 5 H), 2.21 (s, 6 H). LCMS: 579.4 [M+H⁺]. |
| 92/ *N-*(5-((4-((1-acetylindolin-7-yl)amino)-5-chloropyrimidin-2-yl)amino)-4-methoxy-2-(methyl(2-(methylamino)eth yl)amino)phenyl) acrylamide | 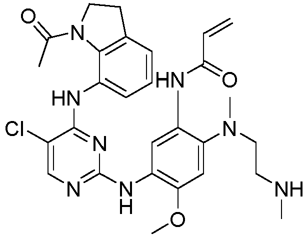 | ¹H NMR (300 MHz, DMSO-d6) δ 10.17 (s, 1 H), 9.38 (s, 1 H), 8.44 (s, 2 H), 8.33 (s, 1 H), 8.16 (s, 1 H), 8.11 (s, 1H), 7.64 (dd, J = 6.6, 2.9 Hz, 1 H), 7.06 - 6.96 (m, 2 H), 6.92 (s, 1 H), 6.64 (dd, J = 16.9, 10.1 Hz, 1H ), 6.27 (dd, J = 17.0, 2.1 Hz, 1H ), 5.81 (dd, J = 10.1, 2.1 Hz, 1 H), 4.13 (t, J = 7.7 Hz, 2 H), 3.83 (s, 3 H), 3.25 - 3.17 (m, 2 H), 3.17 - 2.98 (m, 4 H), 2.61 (t, J = 5.3 Hz, 2 H), 2.56 (s, 3 H), 2.32 (s, 3 H). LCMS: 565.8 [M+H⁺]. |
| 93/ *N-*(5-((5-chloro-4-((1-propionylindolin-7-yl)amino)pyrimid in-2-yl)amino)-2-((2-(dimethylamino) ethyl)(methyl)ami no)-4-methoxyphenyl)acrylamide | 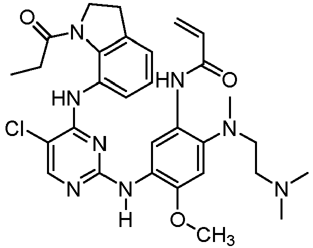 | ¹H NMR (400 MHz, DMSO-d6) δ 10.09 (s, 1 H), 9.62 (s, 1 H), 8.41 (s, 1 H), 8.06 (s, 1 H), 7.97 (s, ¹H ), 7.72 - 7.61 (m, 1 H), 6.95 (s, 1 H), 6.94 - 6.90 (m, 2 H), 6.39 (dd, J = 16.9, 10.1 Hz, 1 H), 6.20 (dd, J = 16.9, 2.2 Hz, 1 H), 5.80 - 5.70 (m, 1 H), 4.10 (t, J = 7.6 Hz, 2 H), 3.77 (s, 3 H), 3.02 (t, J = 7.6 Hz, 2 H), 2.85 (t, J = 5.8 Hz, 2 H), 2.69 (s, 3 H), 2.61 (dd, J = 9.2, 5.5 Hz, 2 H), 2.29 (t, J = 5.8 Hz, 2 H), 2.22 (s, 6 H), 1.12 (t, J = 7.3 Hz, 3 H). LCMS: 593.8 [M+H⁺]. |
| 94/ *N*-(5-((5-chloro-4-((1-propionyl-1,2,3,4-tetrahydroquinoli n-8-yl)amino)pyrimid in-2-yl)amino)-2-((2-(dimethylamino)e thyl)(methyl)amin o)-4-methoxyphenyl)a crylamide | 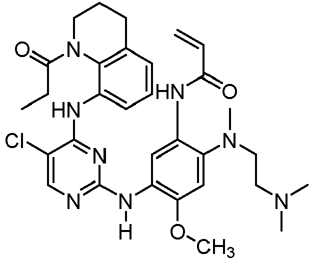 | ¹H NMR (500 MHz, DMSO-d6) δ 9.62 (s, 1 H), 8.46 (s, 1 H), 8.05 (s, 1 H), 7.97 (s, 1 H), 7.68 - 7.56 (d, J = 4.8 Hz, 2 H), 7.06 (d, J = 8.6 Hz, 1 H), 6.96 (s, 1 H), 6.92 (s, 1 H), 6.40 (dd, J = 16.9, 10.4 Hz, 1 H), 6.20 (d, J = 16.9 Hz, 1 H), 5.72 (d, J = 10.7 Hz, 1 H), 3.78 (s, 3 H), 2.90 (s, 2 H), 2.69 (d, J = 9.0 Hz, 5 H), 2.33 (s, 2 H), 2.21 (s, 6 H), 1.94 (s, 2 H), 1.08 (s, 2 H). LCMS: 607.2 [M+H⁺]. |
| 95/ *N-*(5-((4-((1-acetyl-1,2,3,4-tetrahydroquinoli n-8-yl)amino)-5-chloropyrimidin-2-yl)amino)-2-((2-(dimethylamino)e thyl)(methyl)amin o)-4-methoxyphenyl)a crylamide | 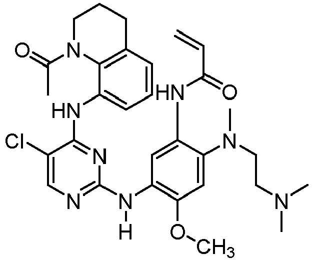 | ¹H NMR (500 MHz, DMSO-d6) δ 9.60 (s, 1 H), 8.46 (s, 1 H), 8.06 (s, 2 H), 7.62 (s, 1 H), 7.06 (d, J = 8.0 Hz, 1 H), 6.96 (d, J = 7.1 Hz, 1 H), 6.92 (s, 1 H), 6.40 (t, J = 13.9 Hz, 1 H), 6.24 - 6.13 (m, 1 H), 5.72 (d, J = 10.3 Hz, 1 H), 3.79 (d, J = 3.5 Hz, 3 H), 2.91 (t, J = 5.9 Hz, 2 H), 2.69 (q, J = 6.5, 3.5 Hz, 4 H), 2.34 (d, J = 6.8 Hz, 2 H), 2.23 (s, 9 H), 1.97 (s, 2 H). LCMS: 593.8 [M+H⁺]. |
| 96/ *N-*(5-((5-chloro-4-((1-(methylsulfonyl)i ndolin-7-yl)amino)pyrimid in-2-yl)amino)-2-((2-(dimethylamino)e thyl)(methyl)amin o)-4-methoxyphenyl)b ut-2-inamide | 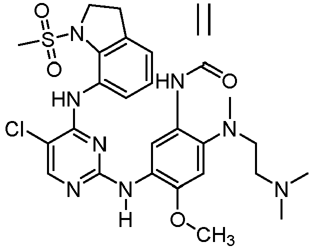 | ¹H NMR (400 MHz, DMSO-d6) δ 11.02 (s, 1 H), 8.92 (s, 1 H), 8.27 (s, 1 H), 8.16 (s, 1 H), 8.11 (s, 1 H), 7.80 (d, J = 8.0 Hz, 1 H), 7.10 - 7.01 (m, 2 H), 6.99 (s, 1 H), 4.05 (t, J = 7.4 Hz, 2 H), 3.76 (s, 3 H), 3.08 (t, J = 7.4 Hz, 2 H), 3.05 (s, 3 H), 2.83 (t, J = 5.5 Hz, 2 H), 2.70 (s, 3 H), 2.28 (s, 8 H), 2.05 (s, 3 H). LCMS: 627.4 [M+H⁺]. |
| 97/ *N-*(5-((4-((1-acetylindolin-7-yl)amino)-5-chloropyrimidin-2-yl)amino)-2-((2-(dimethylamino) ethyl)(methyl)ami no)-4-methoxyphenyl) but-2-inamide | 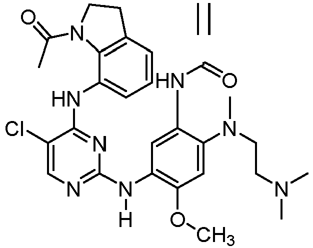 | ¹H NMR (400 MHz, DMSO-d6) δ 11.02 (s, 1 H), 9.76 (s, 1 H), 8.28 (s, 1 H), 8.06 (s, 1 H), 7.95 (s, 1 H), 7.65 - 7.56 (m, 1 H), 6.99 - 6.95 (m, 2 H), 4.11 (t, J = 7.7 Hz, 2 H), 3.76 (s, 3 H), 3.05 (t, J = 7.7 Hz, 2 H), 2.81 (d, J = 5.8 Hz, 2 H), 2.69 (s, 3 H), 2.31 (s, 3 H), 2.28 (s, 6 H), 2.26 (d, J = 5.2 Hz, 2 H), 2.06 (s, 3 H). LCMS: 591.8 [M+H⁺]. |
| 98/ *N-*(5-((5-chloro-4-((4-methyl-2-(*N-*methylmethylsulf onamido)phenyl)a mino)pyrimidin-2-yl)amino)-2-((2-(dimethylamino)e thyl)(methyl)amin o)-4-methoxyphenyl) but-2-inamide | 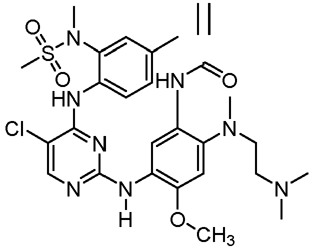 | ¹H NMR (400 MHz, DMSO-d6) δ 11.04 (s, 1 H), 8.33 (s, 1 H), 8.23 (d, J = 10.1 Hz, 2 H), 8.12 (s, 1 H), 8.07 (d, J = 8.4 Hz, 1 H), 7.40 (s, 1 H), 7.06 - 6.99 (m, 2 H), 3.76 (s, 3 H), 3.17 (s, 3 H), 3.10 (s, 3 H), 2.85 (d, J = 5.7 Hz, 2 H), 2.72 (s, 3 H), 2.29 (s, 11 H), 2.05 (s, 3 H). LCMS: 629.2 [M+H⁺]. |
| 99/ *N-*(5-((4-((1-acetylindolin-7-yl)amino)-5-cyanopyrimidin-2-yl)amino)-2-((2-(dimethylamino) ethyl)(methyl)ami no)-4-methoxyphenyl)a crylamide | 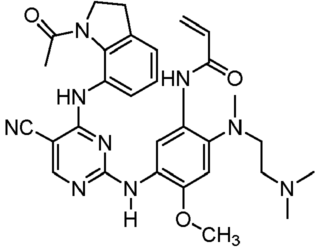 | ¹H NMR (400 MHz, DMSO-d6) δ 10.07 (s, 2 H), 8.42 (s, 1 H), 8.24 (s, 1 H), 7.62 (s, 1 H), 7.55 (s, 1 H), 6.97 (s, 1 H), 6.93 (s, 1 H), 6.79 (s, 1 H), 6.41 (dd, J = 16.7, 10.2 Hz, 1 H), 6.20 (ddd, J = 16.9, 7.8, 2.1 Hz, 1 H), 5.75 - 5.68 (m, 1 H), 4.09 (d, J = 7.9 Hz, 2 H), 3.75 (s, 3 H), 3.00 (d, J = 8.5 Hz, 3 H), 2.87 (s, 2 H), 2.70 (s, 3 H), 2.34 (s, 2 H), 2.29 (s, 3 H), 2.24 (s, 6 H). LCMS: 569.8 [M+H⁺]. |
| 100/ *N*-(5-((4-((1-acetylindolin-7-yl)amino)pyrimid in-2-yl)amino)-2-((2-(dimethylamino)e thyl) (methyl)amino)-4-methoxyphenyl)a crylamide | 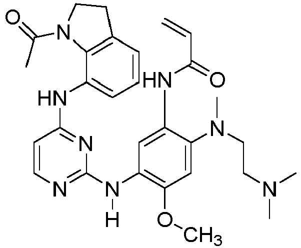 | ¹H NMR (500 MHz, DMSO-d6) δ 10.08 (s, 1 H), 9.42 (s, 1 H), 8.65 (s, 1 H), 7.97 - 7.93 (m, 1 H), 7.68 (s, 1 H), 7.65 (d, J = 8.0 Hz, 1 H), 7.03 (d, J = 7.8 Hz, 1 H), 6.96 (s, 2 H), 6.40 (dd, J = 17.0, 10.2 Hz, 1 H), 6.22 (d, J = 16.9 Hz, 1 H), 6.07 (d, J = 5.7 Hz, 1 H), 5.79 - 5.71 (m, 1 H), 4.13 (t, J = 7.9 Hz, 2 H), 3.82 (s, 3 H), 3.05 (t, J = 7.8 Hz, 2 H), 2.88 (s, 2 H), 2.69 (s, 3 H), 2.31 (s, 5 H), 2.24 (s, 6 H). LCMS: 544.8 [M+H⁺]. |
| | | |
| 101/ *N-*(5-((4-((1-acetylindolin-7-yl)amino)-5-fluoropyrimidin-2-yl)amino)-2-((2-(dimethylamino)e thyl) (methyl)amino)-4-methoxyphenyl)a crylamide | 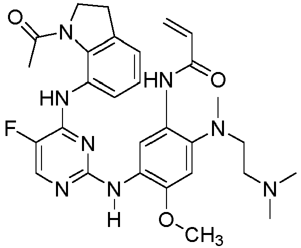 | ¹H NMR (500 MHz, DMSO-d6) δ 10.09 (s, 2 H), 8.52 (s, 1 H), 8.04 (d, J = 3.2 Hz, 1 H), 7.83 (d, J = 8.1 Hz, 1 H), 7.76 (s, 1 H), 7.03 - 6.91 (m, 3 H), 6.45 - 6.33 (m, 1 H), 6.21 (d, J = 16.9 Hz, 1 H), 5.79 - 5.74 (m, 1 H), 4.12 (t, J = 8.0 Hz, 2 H), 3.79 (s, 3 H), 3.04 (t, J = 7.9 Hz, 2 H), 2.86 (s, 2 H), 2.69 (s, 3 H), 2.32 (s, 5 H), 2.23 (s, 6 H). LCMS: 562.8 [M+H⁺]. |

### <Experimental Example 1> Measurement of inhibitory activity of compounds represented by Formula 1 according to the present invention against EGFR mutant enzyme

In order to confirm the inhibitory activity of the compounds represented by Formula 1 according to the present invention against EGFR mutant enzyme, the following experiment was performed. The results are shown in Table 1 below.

The activity of the compounds of the present invention against EGFR mutant enzymes was measured using the HTRF system purchased from Cisbio as follows. For an EGFR mutant enzyme, the EGFR del19 enzyme was a recombinant protein purchased from Carna Biosciences, and the EGFR A763_Y764insFHEA enzyme was a recombinant protein purchased from SignalChem, respectively.

The composition of the assay buffer used for activity measurement was 50 mM tris-HCl pH 7.5, 100 mM NaCl, 7.5 mM MgCl₂, 3 mM KCl, 0.01% tween 20, 0.1% BSA, and 1 mM DTT. An enzymatic reaction was performed using a peptide substrate labeled with ATP at a concentration of 50 mM and biotin at a concentration of 0.5 mM. The analysis of the inhibitory effect of the compounds on EGFR activity was performed according to the following method.
Component 1: 4 µL of EGFR mutant enzyme
Component 2: 2 µL of compound solution
Component 3: 4 µL of ATP and a biotin labeled peptide

The enzyme reaction begins by first mixing component 1 and component 2 and then adding component 3 thereto. After the reaction at 37°C for 2 hours, 10 mL of a measurement solution consisting of streptavidin-XL665 and a europium-labeled anti-phosphotyrosine antibody purchased from Cisbio was added to the enzyme reaction solution and reacted at room temperature for 1 hour. Finally, the ratio of fluorescence values at 615 nm and 665 nm was measured using Perkin-Elmer's Envision instrument to calculate enzyme activity and confirm the inhibitory ability of the compounds. The measured values at 7 compound concentrations were analyzed using the Prism program (version 5.01, Graphpad Software, Inc.), and the IC₅₀ (µM) values, which are measures of the compounds' inhibitory activity, were calculated.

**[Table 1]**

| Exam ple | EGFR del19 IC₅₀ (µM) | EGFR A763_Y764insFHEA IC₅₀ (µM) | Exam ple | EGFR del19 IC₅₀ (µM) | EGFR A763_Y764insFHEA IC₅₀ (µM) |
|---|---|---|---|---|---|
| 1 | <0.001 | 0.004 | 50 | <0.001 | 0.003 |
| 2 | <0.001 | 0.003 | 51 | <0.001 | <0.001 |
| 3 | <0.001 | 0.002 | 52 | <0.001 | <0.001 |
| 4 | <0.001 | 0.002 | 54 | <0.001 | <0.001 |
| 5 | <0.001 | <0.001 | 55 | <0.001 | <0.001 |
| 6 | 0.007 | 0.02 | 56 | <0.001 | <0.001 |
| 7 | <0.001 | 0.005 | 57 | <0.001 | <0.001 |
| 8 | 0.01 | 0.02 | 58 | <0.001 | 0.01 |
| 9 | 0.02 | 0.09 | 59 | <0.001 | <0.001 |
| 10 | <0.001 | 0.01 | 60 | <0.001 | <0.001 |
| 11 | <0.001 | 0.001 | 61 | <0.001 | <0.001 |
| 12 | <0.001 | 0.004 | 64 | <0.001 | <0.001 |
| 13 | 0.002 | 0.01 | 65 | <0.001 | <0.001 |
| 14 | 0.001 | 0.002 | 66 | <0.001 | <0.001 |
| 15 | 0.01 | 0.01 | 67 | <0.001 | <0.001 |
| 16 | <0.001 | 0.002 | 68 | <0.001 | <0.001 |
| 17 | 0.01 | 0.02 | 69 | <0.001 | <0.001 |
| 18 | <0.001 | 0.007 | 70 | <0.001 | 0.004 |
| 19 | <0.001 | 0.003 | 71 | <0.001 | 0.005 |
| 20 | 0.002 | 0.006 | 72 | <0.001 | <0.001 |
| 21 | 0.001 | 0.003 | 73 | <0.001 | <0.001 |
| 22 | <0.001 | 0.003 | 74 | <0.001 | <0.001 |
| 23 | <0.001 | 0.003 | 75 | <0.001 | <0.001 |
| 24 | <0.001 | 0.003 | 76 | <0.001 | <0.001 |
| 25 | <0.001 | <0.001 | 77 | <0.001 | <0.001 |
| 26 | 0.004 | 0.2 | 78 | <0.001 | <0.001 |
| 27 | 0.003 | 0.009 | 79 | <0.001 | <0.001 |
| 28 | <0.001 | 0.008 | 80 | <0.001 | <0.001 |
| 29 | <0.001 | <0.001 | 81 | <0.001 | <0.001 |
| 30 | <0.001 | <0.001 | 82 | <0.001 | <0.001 |
| 31 | <0.001 | <0.001 | 83 | 0.003 | 0.019 |
| 32 | <0.001 | 0.001 | 84 | 0.001 | 0.003 |
| 33 | <0.001 | <0.001 | 85 | 0.018 | 0.043 |
| 34 | <0.001 | 0.001 | 86 | 0.002 | 0.020 |
| 35 | <0.001 | 0.003 | 87 | 0.004 | 0.024 |
| 36 | <0.001 | 0.001 | 88 | < 0.001 | 0.002 |
| 37 | <0.001 | 0.002 | 89 | 0.020 | 0.077 |
| 38 | <0.001 | 0.002 | 90 | 0.001 | 0.007 |
| 39 | <0.001 | <0.001 | 91 | <0.001 | 0.001 |
| 40 | <0.001 | 0.001 | 92 | < 0.001 | 0.001 |
| 41 | <0.001 | 0.001 | 93 | 0.001 | 0.002 |
| 42 | <0.001 | <0.001 | 94 | 0.004 | 0.022 |
| 43 | <0.001 | <0.001 | 95 | 0.002 | 0.011 |
| 44 | <0.001 | <0.001 | 96 | 0.001 | 0.010 |
| 45 | <0.001 | <0.001 | 97 | 0.015 | 0.280 |
| 46 | <0.001 | <0.001 | 98 | < 0.001 | 0.009 |
| 47 | <0.001 | <0.001 | 99 | 0.001 | 0.003 |
| 48 | 0.003 | 0.007 | 100 | 0.003 | 0.009 |
| 49 | 0.0001 | 0.01 | 101 | 0.001 | 0.005 |

As shown in Table 1, it was confirmed the compounds of Examples according to the present invention inhibited activity against EGFR del19 and EGFR A763_Y764insFHEA mutations, which are EGFR mutations.

### <Experimental Example 2> Measurement of inhibitory activity of compounds represented by Formula 1 according to the present invention against HER2 mutation

In order to measure the inhibitory ability of the compounds represented by Formula 1 according to the present invention against HER2 mutation, the following experiment was performed. The results are shown in Table 2 below.

The activity of the compounds of the present invention against HER mutant enzymes was measured using the HTRF system purchased by Cisbio as follows. As a HER2 mutant enzyme, the HER2 A775_G776insYVMA mutant enzyme was used by purchasing a recombinant protein provided by Carna Biosciences.

The composition of the assay buffer used for activity measurement was 50 mM tris-HCl pH 7.5, 100 mM NaCl, 7.5 mM MgCl₂, 3 mM KCl, 0.01% tween 20, 0.1% BSA, and 1 mM DTT. In addition thereto, the enzymatic reaction was performed using a peptide substrate labeled with ATP at a concentration of 50 mM and biotin at a concentration of 0.5 mM. The analysis of the activity inhibitory effect of the compounds on HER2 A775_G776insYVMA mutation was performed according to the following method.
Component 1: 4 µL of HER2 A775_G776insYVMA mutant enzyme
Component 2: 2 µL of compound solution
Component 3: 4 µL of ATP and a biotin labeled peptide

The enzyme reaction begins by first mixing component 1 and component 2 and then adding component 3 thereto. After the reaction at 37°C for 2 hours, 10 mL of a measurement solution consisting of streptavidin-XL665 and a europium-labeled anti-phosphotyrosine antibody purchased from Cisbio was added to the enzyme reaction solution and reacted at room temperature for 1 hour. Finally, the ratio of fluorescence values at 615 nm and 665 nm was measured using Perkin-Elmer's Envision equipment to calculate enzyme activity and confirm the inhibitory ability of the compounds. The measured values at 7 compound concentrations were analyzed using the Prism program (version 5.01, Graphpad Software, Inc.), and the IC₅₀ (µM) values, which are measures of the compounds' inhibitory ability, were calculated.

**[Table 2]**

| Example | HER2 A775_G776insYVMA IC₅₀ (µM) | Example | HER2 A775_G776insYVMA IC₅₀ (µM) |
|---|---|---|---|
| 1 | 0.14 | 51 | 0.030 |
| 2 | 0.31 | 52 | 0.004 |
| 3 | 0.45 | 53 | 0.010 |
| 4 | 0.037 | 54 | 0.010 |
| 5 | 0.034 | 55 | <0.001 |
| 6 | 1.2 | 56 | <0.001 |
| 7 | 0.28 | 57 | <0.001 |
| 8 | 0.92 | 58 | 0.090 |
| 9 | 2.4 | 59 | 0.020 |
| 10 | 0.44 | 60 | 0.010 |
| 11 | 0.071 | 61 | 0.001 |
| 12 | 0.069 | 62 | 0.003 |
| 13 | 0.14 | 63 | <0.001 |
| 14 | 0.41 | 64 | 0.003 |
| 15 | 1.2 | 65 | 0.015 |
| 16 | 0.051 | 66 | 0.005 |
| 17 | 1.1 | 67 | 0.081 |
| 18 | 0.11 | 68 | 0.006 |
| 19 | 0.034 | 69 | 0.006 |
| 20 | 0.37 | 70 | 0.033 |
| 21 | 0.046 | 71 | 0.061 |
| 22 | 0.022 | 72 | 0.001 |
| 23 | 0.030 | 73 | 0.005 |
| 24 | 0.027 | 74 | <0.001 |
| 25 | 0.006 | 75 | 0.003 |
| 26 | 0.080 | 76 | 0.001 |
| 27 | 0.030 | 77 | 0.002 |
| 28 | 0.070 | 78 | 0.003 |
| 29 | 0.005 | 79 | 0.001 |
| 30 | 0.004 | 80 | 0.001 |
| 31 | 0.0003 | 81 | <0.001 |
| 32 | 0.008 | 82 | 0.002 |
| 33 | 0.007 | 83 | 0.374 |
| 34 | 0.020 | 84 | 0.052 |
| 35 | 0.027 | 85 | 0.871 |
| 36 | 0.010 | 86 | 0.157 |
| 37 | 0.026 | 87 | 0.107 |
| 38 | 0.014 | 88 | 0.014 |
| 39 | 0.008 | 89 | 0.479 |
| 40 | 0.004 | 90 | 0.037 |
| 41 | 0.009 | 91 | 0.006 |
| 42 | 0.006 | 92 | 0.005 |
| 43 | 0.008 | 93 | 0.011 |
| 44 | 0.006 | 94 | 0.292 |
| 45 | 0.008 | 95 | 0.054 |
| 46 | 0.002 | 96 | 0.087 |
| 47 | 0.006 | 97 | 0.258 |
| 48 | 0.020 | 98 | 0.053 |
| 49 | 0.060 | 99 | 0.019 |
| 50 | 0.060 | 100 | 0.225 |
| | | 101 | 0.089 |

As shown in Table 2, it was confirmed that the compounds of Examples according to the present invention inhibited the activity against the HER2 A775_G776insYVMA mutation.

### <Experimental Example 3> The Growth inhibition of Ba/F3 cell with EGFR mutations by compounds represented by Formula 1 according to the present invention.

In order to confirm the inhibitory ability of the compounds represented by Formula 1 according to the present invention on the growth of Ba/F3 EGFR mutant cells, the following experiment was performed. The results are shown in Table 3 below.

The activity of the compounds of the present invention against Ba/F3 EGFR V769_D770insASV and Ba/F3 EGFR D770_N771insSVD mutant cell lines was measured using the CellTiter-Glo system purchased from Promega as follows. CellTiter-Glo assay is a method to measure cell viability by monitoring ATP present in cells. Ba/F3 EGFR V769_D770insASV and Ba/F3 EGFR D770_N771insSVD mutant cell lines were purchased from Crown Biosciences. Ba/F3 EGFR V769_D770insASV and Ba/F3 EGFR D770_N771insSVD mutant cell lines were cultured in RPMI containing 10% FBS and 1% penicillin-streptomycin with 1 µg/mL of puromycin in a 5% COz incubator at 37°C.

The analysis of the ability of the compounds to inhibit the growth of EGFR mutant cells was performed according to the following method. 1,000 cells/100 µL were seeded in a 96-well cell culture plate, and 24 hours thereafter, the compounds represented by Formula 1 were treated with 0 µM, 0.01 µM, 0.03 µM, 0.1 µM, 0.3 µM, 1 µM, 3 µM, and 10 µM. After incubating for 72 hours, the plate treated with the compounds were left at room temperature for 30 minutes, 100 µL of a reagent was further added thereto, and then shaken at room temperature for 10 minutes. Finally, using equipment, the ratio of fluorescence values at 570 nm was measured to confirm the cell growth inhibition ability of the compounds. The measured values at 8 compound concentrations were analyzed using the Prism program (version 5.01, Graphpad Software, Inc.), and the IC₅₀ (µM) values, which are measures of the compounds' inhibitory ability, were calculated.

**[Table 3]**

| Exam ple | Ba/F3 EGFR V769_D770insAS V IC₅₀ (nM) | Ba/F3 EGFR D770_N771insSV D IC₅₀ (nM) | Exampl e | Ba/F3 EGFR V769_D770insAS V IC₅₀ (nM) | Ba/F3 EGFR D770_N771insSV D IC₅₀ (nM) |
|---|---|---|---|---|---|
| 4 | B | | 61 | B | B |
| 11 | E | | 62 | B | B |
| 16 | E | | 63 | B | B |
| 22 | E | E | 64 | C | C |
| 24 | E | E | 65 | B | B |
| 25 | E | E | 66 | B | B |
| 29 | B | B | 72 | B | A |
| 30 | A | C | 74 | A | A |
| 31 | A | C | 76 | A | A |
| 32 | C | C | 79 | B | A |
| 33 | A | C | 80 | A | A |
| 35 | C | C | 82 | A | A |
| 36 | B | B | 83 | C | C |
| 37 | D | D | 84 | C | C |
| 38 | C | C | 85 | C | C |
| 39 | A | C | 86 | C | C |
| 40 | C | C | 87 | C | C |
| 41 | C | C | 88 | B | B |
| 43 | A | D | 89 | C | C |
| 45 | A | A | 90 | C | C |
| 46 | C | A | 91 | A | A |
| 47 | A | A | 92 | B | B |
| 48 | D | D | 93 | B | B |
| 49 | D | D | 94 | B | B |
| 50 | D | D | 95 | B | B |
| 53 | B | B | 96 | A | A |
| 55 | A | A | 97 | B | B |
| 56 | A | A | 98 | B | B |
| 57 | D | D | 99 | B | B |
| 58 | D | D | 100 | B | B |
| 59 | D | D | 101 | B | C |

| | | | | | |
|---|---|---|---|---|---|
| * A < 10, 10< B < 30,30 < C < 50,50 < D < 100, 100 < E | | | | | |

As shown in Table 3, it was confirmed that the compounds of Examples according to the present invention inhibited the growth of Ba/F3 EGFR V769_D770insASV and Ba/F3 EGFR D770_N771insSVD mutant cells.

### <Experimental Example 4> The growth inhibition of Ba/F3 cell with HER2 mutations by compounds represented by Formula 1 according to the present invention.

In order to confirm the inhibitory ability of the compounds represented by Formula 1 according to the present invention on the growth of HER2 mutant cells in Ba/F3 cell line, the following experiment was performed. The results are shown in Table 4 below.

The activity of the compounds of the present invention against Ba/F3 HER2 A775_G776insYVMA and Ba/F3 HER2 G776delinsVC mutant cell lines was measured using the CellTiter-Glo system purchased from Promega as follows. CellTiter-Glo assay is a method to measure cell viability by monitoring ATP present in cells. Ba/F3 HER2 A775_G776insYVMA and Ba/F3 HER2 G776delinsVC mutant cell lines were purchased from Crown Biosciences. Ba/F3 HER2 A775_G776insYVMA and Ba/F3 HER2 G776delinsVC mutant cell lines were cultured in RPMI containing 10% FBS and 1% penicillin-streptomycin with 1 µg/mL of puromycin in a COz incubator at 37°C.

The analysis of the ability of the compounds to inhibit the growth of HER2 mutant cells was performed according to the following method. 1,000 cells/100 µL were seeded in a 96-well cell culture plate, and 24 hours thereafter, the compounds represented by Formula 1 were treated with 0 µM, 0.01 µM, 0.03 µM, 0.1 µM, 0.3 µM, 1 µM, 3 µM, and 10 µM. After reacting for 72 hours, the plate treated with the compounds were left at room temperature for 30 minutes, 100 µL of a reagent was added thereto, and then shaken at room temperature for 10 minutes. Finally, using equipment, the ratio of fluorescence values at 570 nm was measured to confirm the cell growth inhibition ability of the compounds. The measured values at 8 compound concentrations were analyzed using the Prism program (version 5.01, Graphpad Software, Inc.), and the IC₅₀ (µM) values, which are measures of the compounds' inhibitory ability, were calculated.

**[Table 4]**

| Exam ple | Ba/F3 HER2A775_G776i nsYVMA IC₅₀ (nM) | Ba/F3 HER2 G776delinsVC IC₅₀ (nM) | Exam ple | Ba/F3 HER2 A775_G776insY VMA IC₅₀ (nM) | Ba/F3 HER2 G776delinsVC IC₅₀ (nM) |
|---|---|---|---|---|---|
| 1 | C | | 53 | B | |
| 2 | C | | 55 | A | A |
| 3 | C | | 56 | A | A |
| 4 | E | B | 57 | D | D |
| 5 | C | | 58 | D | A |
| 6 | C | | 59 | D | D |
| 7 | C | | 61 | B | |
| 11 | E | | 62 | B | |
| 16 | E | | 63 | B | |
| 21 | C | | 64 | B | |
| 22 | E | B | 65 | B | |
| 24 | E | B | 66 | B | |
| 25 | E | E | 83 | E | A |
| 29 | D | B | 84 | E | A |
| 30 | C | A | 85 | E | B |
| 31 | A | A | 86 | E | A |
| 32 | C | A | 87 | E | A |
| 33 | C | A | 88 | C | A |
| 35 | D | A | 89 | E | B |
| 36 | D | B | 90 | E | A |
| 37 | D | C | 91 | A | A |
| 38 | C | A | 92 | C | A |
| 39 | C | A | 93 | B | A |
| 40 | C | A | 94 | E | A |
| 41 | D | A | 95 | C | A |
| 43 | C | A | 96 | E | A |
| 45 | A | A | 97 | E | A |
| 46 | A | C | 98 | E | A |
| 47 | A | A | 99 | B | A |
| 48 | D | A | 100 | E | A |
| 49 | D | A | 101 | D | A |
| 50 | D | A | | | |

| | | | | | |
|---|---|---|---|---|---|
| * A < 10, 10< B < 30,30 < C < 50,50 < D < 100, 100 < E | | | | | |

As shown in Table 4, it was confirmed that the compounds of Examples according to the present invention inhibited the growth of Ba/F3 HER2 A775_G776insYVMA and Ba/F3 HER2 G776delinsVC mutant cells.

Accordingly, it was possible to confirm that the compounds represented by Formula 1 according to the present invention not only have high inhibitory activity against EGFR mutant enzyme, but also have high growth inhibitory ability against cells with EGFR mutations, and from these results, could be treatment options of cancer with EGFR mutations, such as EGFR del19, EGFR A763_Y764insFHEA, Ba/F3 EGFR V769_D770insASV, and Ba/F3 EGFR D770_N771insSVD.

Additionally, it was possible to confirm that the compounds represented by Formula 1 according to the present invention not only have high inhibitory ability against HER2 mutatant enzyme, but also have high growth inhibitory ability against cells with HER2 mutations, and from these results, could be effectively used on the treatment of cancer where HER2 mutations, such HER2 A775_G776insYVMA, Ba/F3 HER2 A775_G776insYVMA, and Ba/F3 HER2 G776delinsVC, are expressed.

From the above results, it can be seen that the pyrimidine derivative compound according to the present invention can effectively inhibit EGFR and HER2 mutations, and thus can be effectively used as a pharmaceutical composition for the prevention or treatment of cancer.

## Claims

1. A compound represented by the following Formula 1, an isomer thereof, a solvate thereof, a hydrate thereof, or a pharmaceutically acceptable salt thereof 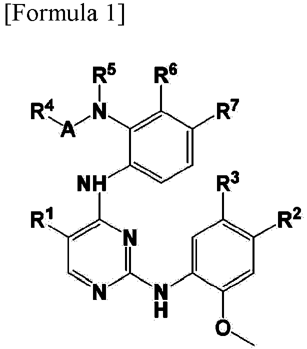
wherein in Formula 1 above,
A is -SOz- or -CO-;
R¹ is hydrogen, halogen, cyano, C₁₋₁₀ alkyl unsubstituted or substituted with one or more halogens, carboxy, or C₁₋₁₀ alkoxycarbonyl; and
R² is -ORₐ or -NR_{b1}R_{b2},
wherein Rₐ is C₁₋₆ alkyl substituted with NR_{b1}R_{b2}, R_{b1} and R_{b2} are each independently hydrogen, C₁₋₆ alkyl unsubstituted or substituted with NR_{c1}R_{c2}, or R_{b1} and R_{b2}, together with the nitrogen to which they are bound, form heterocycloalkyl of 3 to 8 atoms unsubstituted or substituted with C₁₋₆ alkyl or NR_{c1}R_{c2} comprising one or more heteroatoms selected from the group consisting of N, O, and S,
R_{c1} and R_{c2} are each independently hydrogen, C₁₋₆ alkyl, or R_{c1} and R_{c2}, together with the nitrogen to which they are bound, are able to form heterocycloalkyl of 3 to 8 atoms unsubstituted or substituted with C₁₋₆ alkyl comprising one or more heteroatoms selected from the group consisting of N, O, and S;
R³ is 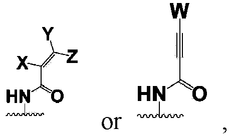
wherein W, X, Y, and Z are independently hydrogen, halogen, or C₁₋₆ alkyl unsubstituted or substituted with NR_{d1}R_{d2},
R_{d1} and R_{d2} are each independently hydrogen or C₁₋₆ alkyl;
R⁴ to R⁷ are each independently hydrogen, halogen, C₁₋₁₀ alkyl, C₅₋₁₀ 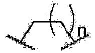 or 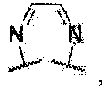 in which one or more single bonds in 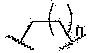 may be substituted with a double bond, and the remaining two are each independently hydrogen or C₁₋₆ alkyl,
n is an integer from 0 to 3, and
the heterocycloalkyl comprises at least one N.

2. The compound, the isomer thereof, the solvate thereof, the hydrate thereof, the pharmaceutically acceptable salt thereof of claim 1, wherein:
A is -SOz- or -CO-;
R¹ is hydrogen, halogen, cyano, C₁₋₆ alkyl unsubstituted or substituted with one or more halogens, carboxy, or C₁₋₆ alkoxycarbonyl; and
R² is -ORₐ or -NR_{b1}R_{b2},
wherein Rₐ is C₁₋₄ alkyl substituted with NR_{b1}R_{b2}, R_{b1} and R_{b2} are each independently hydrogen, C₁₋₄ alkyl unsubstituted or substituted with NR_{c1}R_{c2}, or R_{b1} and R_{b2}, together with the nitrogen to which they are bound, form heterocycloalkyl of 5 to 8 atoms unsubstituted or substituted with C₁₋₄ alkyl or NR_{c1}R_{c2} comprising one or more heteroatoms selected from the group consisting of N, O, and S,
R_{c1} and R_{c2} are each independently hydrogen, C₁₋₃ alkyl, or R_{c1} and R_{c2}, together with the nitrogen to which they are bound, form heterocycloalkyl of 3 to 6 atoms unsubstituted or substituted with C₁₋₄ alkyl including one or more heteroatoms selected from the group consisting of N, O, and S;
R³ is 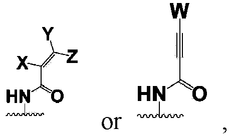
wherein W, X, and Z are independently hydrogen, halogen, or C₁₋₄ alkyl,
Y is hydrogen, or C₁₋₄ alkyl unsubstituted or substituted with NR_{d1}R_{d2},
R_{d1} and R_{d2} are each independently hydrogen or C₁₋₄ alkyl;
R⁴ to R⁷ are any one of the following 1) to 4), wherein:
1) R⁴ to R⁷ are each independently hydrogen, halogen, C₁₋₆ alkyl, or C₆₋₁₀ aryl unsubstituted or substituted with C₁₋₆ alkyl;
2) R⁴ and R⁵ together form 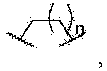 and R⁶ and R⁷ are hydrogen;
3) R⁵ and R⁶ together form 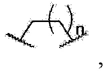 and R⁴ and R⁷ are each independently hydrogen or C₁₋₄ alkyl, in which one or more single bonds in 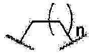 may be substituted with one or more double bonds;
4) R⁶ and R⁷ together form 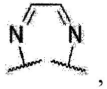 and R⁴ and R⁵ are each independently hydrogen or C₁₋₄ alkyl;
n is an integer from 1 to 3, and
the heterocycloalkyl comprises at least one N.

3. The compound, the isomer thereof, the solvate thereof, the hydrate thereof, or the pharmaceutically acceptable salt thereof of claim 1, wherein:
A is -SOz- or -CO-;
R¹ is hydrogen, halogen, cyano, C₁ alkyl unsubstituted or substituted with one or more halogens, carboxy, or C₁₋₃ alkoxycarbonyl; and
R² is -ORₐ or -NR_{b1}R_{b2},
wherein Rₐ is C₁₋₃ alkyl substituted with NR_{b1}R_{b2},
R_{b1} and R_{b2} are each independently hydrogen, C₁₋₃ alkyl unsubstituted or substituted with NR_{c1}R_{c2}, or R_{b1} and R_{b2}, together with the nitrogen to which they are bound, form heterocycloalkyl of 4 to 6 atoms unsubstituted or substituted with methyl or NR_{c1}R_{c2} comprising one or more heteroatoms selected from the group consisting of N and O; and
R_{c1} and R_{c2} are each independently hydrogen, methyl, isopropyl, or R_{c1} and R_{c2}, together with the nitrogen to which they are bound, form heterocycloalkyl of 4 to 6 atoms unsubstituted or substituted with methyl comprising one or more heteroatoms selected from the group consisting of N and O;
R³ is 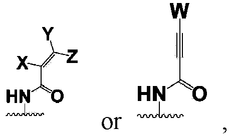
wherein W is methyl,
X is hydrogen or fluorine,
Y is hydrogen or methyl substituted with NR_{d1}R_{d2},
R_{d1} and R_{d2} are each independently hydrogen or methyl,
Z is hydrogen;
R⁴ to R⁷ are any one of the following 1) to 4), wherein:
1) R⁴ to R⁷ are each independently hydrogen, halogen, C₁₋₃ alkyl, phenyl unsubstituted or substituted with C₁₋₃ alkyl;
2) R⁴ and R⁵ together form 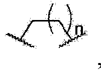 and R⁶ and R⁷ are hydrogen;
3) R⁵ and R⁶ together form 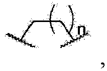 and R⁴ and R⁷ are each independently hydrogen or methyl, in which the single bond in 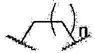 may be substituted with one or more double bonds;
4) R⁶ and R⁷ together form 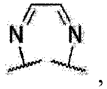 and R⁴ and R⁵ are each independently hydrogen or methyl;
n is an integer from 1 to 3, and
the heterocycloalkyl comprises at least one N.

4. The compound, the isomer thereof, the solvate thereof, the hydrate thereof, the pharmaceutically acceptable salt thereof of claim 1, wherein:
A is -SOz- or -CO-;
R¹ is hydrogen, chlorine, fluorine, methyl, cyano, CF₃, 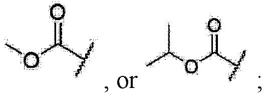
R² is 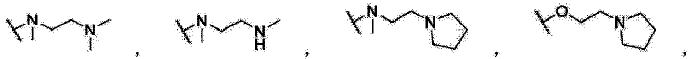 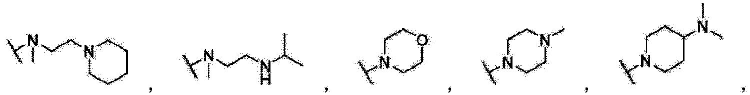 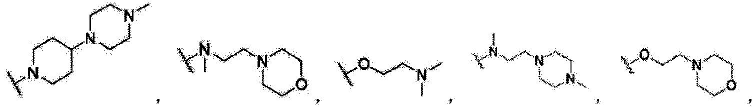 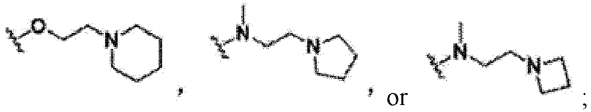
R³ is 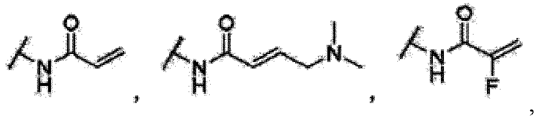 or 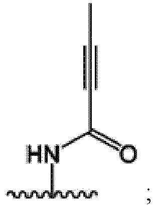 and
R⁴ to R⁷ are any one of the following 1) to 4), wherein:
1) R⁴ to R⁷ are each independently hydrogen, chlorine, methyl, ethyl, propyl, isopropyl, phenyl, or toluyl;
2) R⁴ and R⁵ together form 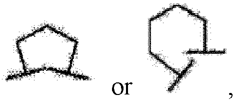 and R⁶ and R⁷ are hydrogen;
3) R⁵ and R⁶ together form 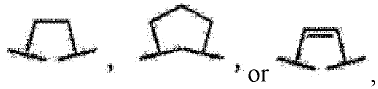 and R⁴ and R⁷ are each independently hydrogen or methyl; and
4) R⁶ and R⁷ together form 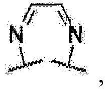 and R⁴ and R⁵ are each independently hydrogen or methyl.

5. The compound, the isomer thereof, the solvate thereof, the hydrate thereof, the pharmaceutically acceptable salt thereof of claim 1, wherein the compound represented by Formula 1 above is any one selected from the group of compounds below:
<1> *N-*(5-((5-chloro-4-((2-(propylsulfonamido)phenyl)amino)pyrimidin-2-yl)amino)-2-((2-(dimethylamino)ethyl)(methyl)amino)-4-methoxyphenyl)acrylamide;
<2> *N-*(5-((5-chloro-4-((2-(phenylsulfonamido)phenyl)amino)pyrimidin-2-yl)amino)-2-((2-(dimethylamino)ethyl)(methyl)amino)-4-methoxyphenyl)acrylamide;
<3> *N-*(5-((5-chloro-4-((2-((4-methylphenyl)sulfonamido)phenyl)amino)pyrimidin-2-yl)amino)-2-((2-(dimethylamino)ethyl)(methyl)amino)-4-methoxyphenyl)acrylamide;
<4> *N-*(5-((5-chloro-4-((2-(ethylsulfonamido)phenyl)amino)pyrimidin-2-yl)amino)-2-((2-(dimethylamino)ethyl)(methyl)amino)-4-methoxyphenyl)acrylamide;
<5> *N-*(5-((5-chloro-4-((2-((1-methylethyl)sulfonamido)phenyl)amino)pyrimidin-2-yl)amino)-2-((2-(dimethylamino)ethyl)(methyl)amino)-4-methoxyphenyl)acrylamide;
<6> *N-*(2-((2-(dimethylamino)ethyl)(methyl)amino)-4-methoxy-5-((4-((2-(phenylsulfonamido)phenyl)amino)pyrimidin-2-yl)amino)phenyl)acrylamide;
<7> *N-*(2-((2-(dimethylamino)ethyl)(methyl)amino)-4-methoxy-5-((4-((2-(methylsulfonamido)phenyl)amino)pyrimidin-2-yl)amino)phenyl)acrylamide;
<8> *N-*(2-((2-(dimethylamino)ethyl)(methyl)amino)-4-methoxy-5-((4-((2-(propylsulfonamido)phenyl)amino)pyrimidin-2-yl)amino)phenyl)acrylamide;
<9> *N-*(2-((2-(dimethylamino)ethyl)(methyl)amino)-4-methoxy-5-((4-((2-((4-methylphenyl)sulfonamido)phenyl)amino)pyrimidin-2-yl)amino)phenyl)acrylamide;
<10> *N-*(2-((2-(dimethylamino)ethyl)(methyl)amino)-5-((4-((2-(ethylsulfonamido)phenyl)amino)pyrimidin-2-yl)amino)-4-methoxyphenyl)acrylamide;
<11> *N-*(2-((2-(dimethylamino)ethyl)(methyl)amino)-5-((5-fluoro-4-((2-(methylsulfonamido)phenyl)amino)pyrimidin-2-yl)amino)-4-methoxyphenyl)acrylamide;
<12> *N-*(2-((2-(dimethylamino)ethyl)(methyl)amino)-5-((4-((2-(ethylsulfonamido)phenyl)amino)-5-fluoropyrimidin-2-yl)amino)-4-methoxyphenyl)acrylamide;
<13> *N-*(2-((2-(dimethylamino)ethyl)(methyl)amino)-5-((5-fluoro-4-((2-(propylsulfonamido)phenyl)amino)pyrimidin-2-yl)amino)-4-methoxyphenyl)acrylamide;
<14> *N-*(2-((2-(dimethylamino)ethyl)(methyl)amino)-5-((5-fluoro-4-((2-(phenylsulfonamido)phenyl)amino)pyrimidin-2-yl)amino)-4-methoxyphenyl)acrylamide;
<15> *N-*(2-((2-(dimethylamino)ethyl)(methyl)amino)-5-((5-fluoro-4-((2-((4-methylphenyl)sulfonamido)phenyl)amino)pyrimidin-2-yl)amino)-4-methoxyphenyl)acrylamide;
<16> *N-*(2-((2-(dimethylamino)ethyl)(methyl)amino)-4-methoxy-5-((5-methyl-4-((2-(methylsulfonamido)phenyl)amino)pyrimidin-2-yl)amino)phenyl)acrylamide;
<17> *N-*(2-((2-(dimethylamino)ethyl)(methyl)amino)-4-methoxy-5-((5-methyl-4-((2-((4-methylphenyl)sulfonamido)phenyl)amino)pyrimidin-2-yl)amino)phenyl)acrylamide;
<18> *N-*(2-((2-(dimethylamino)ethyl)(methyl)amino)-4-methoxy-5-((5-methyl-4-((2-(propylsulfonamido)phenyl)amino)pyrimidin-2-yl)amino)phenyl)acrylamide;
<19> *N-*(2-((2-(dimethylamino)ethyl)(methyl)amino)-5-((4-((2-(ethylsulfonamido)phenyl)amino)-5-methylpyrimidin-2-yl)amino)-4-methoxyphenyl)acrylamide;
<20> *N-*(2-((2-(dimethylamino)ethyl)(methyl)amino)-4-methoxy-5-((5-methyl-4-((2-(phenylsulfonamido)phenyl)amino)pyrimidin-2-yl)amino)phenyl)acrylamide;
<21> *N-*(5-((5-chloro-4-((2-(methylsulfonamido)phenyl)amino)pyrimidin-2-yl)amino)-4-methoxy-2-(methyl(2-(pyrrolidin-1-yl)ethyl)amino)phenyl)acrylamide;
<22> *N-*(5-((5-chloro-4-((2-(methylsulfonamido)phenyl)amino)pyrimidin-2-yl)amino)-4-methoxy-2-(2-(pyrrolidin-1-yl)ethoxy)phenyl)acrylamide;
<23> *N-*(5-((5-chloro-4-((2-(methylsulfonamido)phenyl)amino)pyrimidin-2-yl)amino)-4-methoxy-2-(methyl(2-(piperidin-1-yl)ethyl)amino)phenyl)acrylamide;
<24> *N-*(5-((5-chloro-4-((2-(methylsulfonamido)phenyl)amino)pyrimidin-2-yl)amino)-2-((2-(isopropylamino)ethyl)(methyl)amino)-4-methoxyphenyl)acrylamide;
<25> *N-*(5-((5-chloro-4-((2-(methylsulfonamido)phenyl)amino)pyrimidin-2-yl)amino)-4-methoxy-2-(methyl(2-(methylamino)ethyl)amino)phenyl)acrylamide;
<26> (E)-*N-*(5-((5-chloro-4-((2-(methylsulfonamido)phenyl)amino)pyrimidin-2-yl)amino)-4-methoxy-2-morpholinophenyl)-4-(dimethylamino)buten-2-amide;
<27> (E)-*N-*(5-((5-chloro-4-((2-(methylsulfonamido)phenyl)amino)pyrimidin-2-yl)amino)-4-methoxy-2-(4-methylpiperazin-1-yl)phenyl)-4-(dimethylamino)buten-2-amide;
<28> (E)-*N-*(5-((5-chloro-4-((2-(methylsulfonamido)phenyl)amino)pyrimidin-2-yl)amino)-2-((2-(dimethylamino)ethyl)(methyl)amino)-4-methoxyphenyl)-4-(dimethylamino)buten-2-amide;
<29> *N-*(5-((5-chloro-4-((2-(*N*-methylmethylsulfonamido)phenyl)amino)pyrimidin-2-yl)amino)-2-((2-(dimethylamino)ethyl)(methyl)amino)-4-methoxyphenyl)acrylamide;
<30> *N-*(5-((5-chloro-4-((2-(*N*-methylethylsulfonamido)phenyl)amino)pyrimidin-2-yl)amino)-2-((2-(dimethylamino)ethyl)(methyl)amino)-4-methoxyphenyl)acrylamide;
<31> *N-*(5-((5-chloro-4-((2-(*N*-ethylmethylsulfonamido)phenyl)amino)pyrimidin-2-yl)amino)-2-((2-(dimethylamino)ethyl)(methyl)amino)-4-methoxyphenyl)acrylamide;
<32> *N-*(5-((5-chloro-4-((2-(*N*-isopropylmethylsulfonamido)phenyl)amino)pyrimidin-2-yl)amino)-2-((2-(dimethylamino)ethyl)(methyl)amino)-4-methoxyphenyl)acrylamide;
<33> *N-*(5-((5-chloro-4-((2-(*N*-ethylethylsulfonamido)phenyl)amino)pyrimidin-2-yl)amino)-2-((2-(dimethylamino)ethyl)(methyl)amino)-4-methoxyphenyl)acrylamide;
<34> *N-*(5-((5-chloro-4-((3-chloro-2-(methylsulfonamido)phenyl)amino)pyrimidin-2-yl)amino)-2-((2-(dimethylamino)ethyl)(methyl)amino)-4-methoxyphenyl)acrylamide;
<35> *N*-(5-((5-chloro-4-((3-chloro-2-(*N-*methylmethylsulfonamido)phenyl)amino)pyrimidin-2-yl)amino)-2-((2-(dimethylamino)ethyl)(methyl)amino)-4-methoxyphenyl)acrylamide;
<36> *N-*(5-((5-chloro-4-((3-methyl-2-(methylsulfonamido)phenyl)amino)pyrimidin-2-yl)amino)-2-((2-(dimethylamino)ethyl)(methyl)amino)-4-methoxyphenyl)acrylamide;
<37> *N*-(5-((5-chloro-4-((3-isopropyl-2-(*N-*methylmethylsulfonamido)phenyl)amino)pyrimidin-2-yl)amino)-2-((2-(dimethylamino)ethyl)(methyl)amino)-4-methoxyphenyl)acrylamide;
<38> *N*-(5-((5-chloro-4-((3-methyl-2-(*N-*methylmethylsulfonamido)phenyl)amino)pyrimidin-2-yl)amino)-2-((2-(dimethylamino)ethyl)(methyl)amino)-4-methoxyphenyl)acrylamide;
<39> *N-*(5-((5-chloro-4-((2-(*N-*ethylmethylsulfonamido)-3-methylphenyl)amino)pyrimidin-2-yl)amino)-2-((2-(dimethylamino)ethyl)(methyl)amino)-4-methoxyphenyl)acrylamide;
<40> *N-*(5-((5-chloro-4-((3-ethyl-2-(methylsulfonamido)phenyl)amino)pyrimidin-2-yl)amino)-2-((2-(dimethylamino)ethyl)(methyl)amino)-4-methoxyphenyl)acrylamide;
<41> *N*-(5-((5-chloro-4-((3-ethyl-2-(*N-*methylmethylsulfonamido)phenyl)amino)pyrimidin-2-yl)amino)-2-((2-(dimethylamino)ethyl)(methyl)amino)-4-methoxyphenyl)acrylamide;
<42> *N-*(5-((5-chloro-4-((3-ethyl-2-(ethylsulfonamido)phenyl)amino)pyrimidin-2-yl)amino)-2-((2-(dimethylamino)ethyl)(methyl)amino)-4-methoxyphenyl)acrylamide;
<43> *N*-(5-((5-chloro-4-((3,4-dimethyl-2-(*N-*methylmethylsulfonamido)phenyl)amino)pyrimidin-2-yl)amino)-2-((2-(dimethylamino)ethyl)(methyl)amino)-4-methoxyphenyl)acrylamide;
<44> *N-*(5-((5-chloro-4-((3,4-dimethyl-2-(methylsulfonamido)phenyl)amino)pyrimidin-2-yl)amino)-2-((2-(dimethylamino)ethyl)(methyl)amino)-4-methoxyphenyl)acrylamide;
<45> *N*-(5-((5-chloro-4-((4-methyl-2-(*N-*methylmethylsulfonamido)phenyl)amino)pyrimidin-2-yl)amino)-2-((2-(dimethylamino)ethyl)(methyl)amino)-4-methoxyphenyl)acrylamide;
<46> *N-*(5-((5-chloro-4-((4-methyl-2-(methylsulfonamido)phenyl)amino)pyrimidin-2-yl)amino)-2-((2-(dimethylamino)ethyl)(methyl)amino)-4-methoxyphenyl)acrylamide;
<47> *N-*(5-((5-chloro-4-((1-(methylsulfonyl)-1,2,3,4-tetrahydroquinolin-8-yl)amino)pyrimidin-2-yl)amino)-2-((2-(dimethylamino)ethyl)(methyl)amino)-4-methoxyphenyl)acrylamide;
<48> *N-*(5-((5-chloro-4-((1-(methylsulfonyl)-1,2,3,4-tetrahydroquinolin-8-yl)amino)pyrimidin-2-yl)amino)-4-methoxy-2-(methyl(2-(methylamino)ethyl)amino)phenyl)acrylamide;
<49> *N-*(5-((5-chloro-4-((1-(methylsulfonyl)-1,2,3,4-tetrahydroquinolin-8-yl)amino)pyrimidin-2-yl)amino)-2-(4-(dimethylamino)piperidin-1-yl)-4-methoxyphenyl)acrylamide;
<50> *N-*(5-((5-chloro-4-((1-(methylsulfonyl)-1,2,3,4-tetrahydroquinolin-8-yl)amino)pyrimidin-2-yl)amino)-4-methoxy-2-(4-(4-methylpiperazin-1 -yl)piperidin-1 - yl)phenyl)acrylamide;
<51> methyl 2-((5-acrylamido-4-((2-(dimethylamino)ethyl)(methyl)amino)-2-methoxyphenyl)amino)-4-((1-(methylsulfonyl)-1,2,3,4-tetrahydroquinolin-8-yl)amino)pyrimidin-5-carboxylate;
<52> isopropyl 2-((5-acrylamido-4-((2-(dimethylamino)ethyl)(methyl)amino)-2-methoxyphenyl)amino)-4-((1-(methylsulfonyl)-1,2,3,4-tetrahydroquinolin-8-yl)amino)pyrimidin-5-carboxylate;
<53> *N-*(5-((5-chloro-4-((1-(methylsulfonyl)-1,2,3,4-tetrahydroquinolin-8-yl)amino)pyrimidin-2-yl)amino)-4-methoxy-2-(methyl(2-morpholinoethyl)amino)phenyl)acrylamide;
<54> *N-*(5-((5-chloro-4-((1-(methylsulfonyl)-1,2,3,4-tetrahydroquinolin-8-yl)amino)pyrimidin-2-yl)amino)-2-((2-(dimethylamino)ethyl)(methyl)amino)-4-methoxyphenyl)-2-fluoroacrylamide;
<55> *N-*(5-((5-chloro-4-((1-(methylsulfonyl)indolin-7-yl)amino)pyrimidin-2-yl)amino)-2-((2-(dimethylamino)ethyl)(methyl)amino)-4-methoxyphenyl)acrylamide;
<56> *N-*(5-((5-chloro-4-((1-(ethylsulfonyl)indolin-7-yl)amino)pyrimidin-2-yl)amino)-2-((2-(dimethylamino)ethyl)(methyl)amino)-4-methoxyphenyl)acrylamide;
<57> *N-*(5-((5-chloro-4-((1-(methylsulfonyl)indolin-7-yl)amino)pyrimidin-2-yl)amino)-4-methoxy-2-(methyl(2-(methylamino)ethyl)amino)phenyl)acrylamide;
<58> *N-*(5-((5-chloro-4-((1-(methylsulfonyl)indolin-7-yl)amino)pyrimidin-2-yl)amino)-2-(4-(dimethylamino)piperidin-1-yl)-4-methoxyphenyl)acrylamide;
<59> *N-*(5-((5-chloro-4-((1-(methylsulfonyl)indolin-7-yl)amino)pyrimidin-2-yl)amino)-4-methoxy-2-(4-(4-methylpiperazin-1 -yl)piperidin-1 -yl)phenyl)acrylamide;
<60> methyl 2-((5-acrylamido-4-((2-(dimethylamino)ethyl)(methyl)amino)-2-methoxyphenyl)amino)-4-((1-(methylsulfonyl)indolin-7-yl)amino)pyrimidin-5-carboxylate;
<61> isopropyl 2-((5-acrylamido-4-((2-(dimethylamino)ethyl)(methyl)amino)-2-methoxyphenyl)amino)-4-((1-(methylsulfonyl)indolin-7-yl)amino)pyrimidin-5-carboxylate;
<62> *N-*(5-((5-cyano-4-((1-(methylsulfonyl)indolin-7-yl)amino)pyrimidin-2-yl)amino)-2-((2-(dimethylamino)ethyl)(methyl)amino)-4-methoxyphenyl)acrylamide;
<63> *N-*(5-((5-chloro-4-((1-(methylsulfonyl)indolin-7-yl)amino)pyrimidin-2-yl)amino)-2-(2-(dimethylamino)ethoxy)-4-methoxyphenyl)acrylamide;
<64> *N-*(5-((5-chloro-4-((1-(methylsulfonyl)indolin-7-yl)amino)pyrimidin-2-yl)amino)-4-methoxy-2-(methyl(2-morpholinoethyl)amino)phenyl)acrylamide;
<65> *N-*(2-((2-(dimethylamino)ethyl)(methyl)amino)-4-methoxy-5-((4-((1-(methylsulfonyl)indolin-7-yl)amino)-5-(trifluoromethyl)pyrimidin-2-yl)amino)phenyl)acrylamide;
<66> *N-*(5-((5-chloro-4-((1-(methylsulfonyl)indolin-7-yl)amino)pyrimidin-2-yl)amino)-2-((2-(dimethylamino)ethyl)(methyl)amino)-4-methoxyphenyl)-2-fluoroacrylamide;
<67> *N-*(5-((5-chloro-4-((1-(methylsulfonyl)-1H-indol-7-yl)amino)pyrimidin-2-yl)amino)-2-((2-(dimethylamino)ethyl)(methyl)amino)-4-methoxyphenyl)acrylamide;
<68> *N-*(5-((5-chloro-4-((5-(*N-*methylmethylsulfonamido)quinoxalin-6-yl)amino)pyrimidin-2-yl)amino)-2-((2-(dimethylamino)ethyl)(methyl)amino)-4-methoxyphenyl)acrylamide;
<69> *N-*(6-((5-chloro-2-((4-((2-(dimethylamino)ethyl)(methyl)amino)-2-methoxyphenyl)amino)pyrimidin-4-yl)amino)quinoxalin-5-yl)methanesulfonamide;
<70> *N*-(5-((5 -chloro-4-((2-(1,1-dioxidoisothiazolidin-2-yl)phenyl)amino)pyrimidin-2-yl)amino)-2-((2-(dimethylamino)ethyl)(methyl)amino)-4-methoxyphenyl)acrylamide;
<71> *N*-(5-((5-chloro-4-((2-(1,1-dioxido-1,2-thiazinan-2-yl)phenyl)amino)pyrimidin-2-yl)amino)-2-((2-(dimethylamino)ethyl)(methyl)amino)-4-methoxyphenyl)acrylamide;
<72> *N-*(5-((5-chloro-4-((1-(methylsulfonyl)indolin-7-yl)amino)pyrimidin-2-yl)amino)-4-methoxy-2-(methyl(2-(piperidin-1-yl)ethyl)amino)phenyl)acrylamide;
<73> *N-*(5-((5-chloro-4-((1-(methylsulfonyl)indolin-7-yl)amino)pyrimidin-2-yl)amino)-4-methoxy-2-(methyl(2-(4-methylpiperazin-1-yl)ethyl)amino)phenyl)acrylamide;
<74> *N-*(5-((5-chloro-4-((1-(methylsulfonyl)indolin-7-yl)amino)pyrimidin-2-yl)amino)-4-methoxy-2-(methyl(2-(pyrrolidin-1-yl)ethyl)amino)phenyl)acrylamide;
<75> *N-*(5-((5-chloro-4-((1-(ethylsulfonyl)indolin-7-yl)amino)pyrimidin-2-yl)amino)-4-methoxy-2-(methyl(2-(pyrrolidin-1-yl)ethyl)amino)phenyl)acrylamide;
<76> *N*-(5-((5-chloro-4-((4-methyl-2-(*N-*methylmethylsulfonamido)phenyl)amino)pyrimidin-2-yl)amino)-4-methoxy-2-(methyl(2-(pyrrolidin-1-yl)ethyl)amino)phenyl)acrylamide;
<77> *N-*(5-((5-chloro-4-((1-(methylsulfonyl)indolin-7-yl)amino)pyrimidin-2-yl)amino)-4-methoxy-2-(2-morpholinoethoxy)phenyl)acrylamide;
<78> *N*-(5-((5-chloro-4-((1-(methylsulfonyl)indolin-7-yl)amino)pyrimidin-2-yl)amino)-4-methoxy-2-(2-(piperidin-1-yl)ethoxy)phenyl)acrylamide;
<79> *N-*(5-((5-chloro-4-((1-(methylsulfonyl)indolin-7-yl)amino)pyrimidin-2-yl)amino)-4-methoxy-2-(2-(pyrrolidin-1-yl)ethoxy)phenyl)acrylamide;
<80> *N-*(2-((2-(azetidin-1-yl)ethyl)(methyl)amino)-5-((5-chloro-4-((1-(methylsulfonyl)indolin-7-yl)amino)pyrimidin-2-yl)amino)-4-methoxyphenyl)acrylamide;
<81> *N-*(2-((2-(azetidin-1-yl)ethyl)(methyl)amino)-5-((5-chloro-4-((1-(ethylsulfonyl)indolin-7-yl)amino)pyrimidin-2-yl)amino)-4-methoxyphenyl)acrylamide;
<82> *N-*(2-((2-(azetidin-1-yl)ethyl)(methyl)amino)-5-((5-chloro-4-((4-methyl-2-(*N-*methylmethylsulfonamido)phenyl)amino)pyrimidin-2-yl)amino)-4-methoxyphenyl)acrylamide;
<83> *N*-(5-((5-chloro-4-((3,4-dimethyl-2-(*N-*methylacetamido)phenyl)amino)pyrimidin-2-yl)amino)-2-((2-(dimethylamino)ethyl)(methyl)amino)-4-methoxyphenyl)acrylamide;
<84> *N-*(5-((4-((2-acetamido-3,4-dimethylphenyl)amino)-5-chloropyrimidin-2-yl)amino)-2-((2-(dimethylamino)ethyl)(methyl)amino)-4-methoxyphenyl)acrylamide;
<85> *N-*(5-((5-chloro-4-((3-methyl-2-(*N-*methylacetamido)phenyl)amino)pyrimidin-2-yl)amino)-2-((2-(dimethylamino)ethyl)(methyl)amino)-4-methoxyphenyl)acrylamide;
<86> *N-*(5-((4-((2-acetamido-3-methylphenyl)amino)-5-chloropyrimidin-2-yl)amino)-2-((2-(dimethylamino) ethyl)(methyl)amino)-4-methoxyphenyl)acrylamide;
<87> *N-*(5-((5-chloro-4-((4-methyl-2-(*N-*methylacetamido)phenyl)amino)pyrimidin-2-yl)amino)-2-((2-(dimethylamino)ethyl)(methyl)amino)-4-methoxyphenyl)acrylamide;
<88> *N-*(5-((4-((2-acetamido-4-methylphenyl)amino)-5-chloropyrimidin-2-yl)amino)-2-((2-(dimethylamino) ethyl)(methyl)amino)-4-methoxyphenyl)acrylamide;
<89> *N-*(5-((5-chloro-4-((2-(*N-*methylacetamido)phenyl)amino)pyrimidin-2-yl)amino)-2-((2-(dimethylamino)ethyl)(methyl)amino)-4-methoxyphenyl)acrylamide;
<90> *N-*(5-((4-((2-acetamidophenyl)amino)-5-chloropyrimidin-2-yl)amino)-2-((2-(dimethylamino) ethyl)(methyl)amino)-4-methoxyphenyl)acrylamide;
<91> *N-*(5-((4-((1-acetylindolin-7-yl)amino)-5-chloropyrimidin-2-yl)amino)-2-((2-(dimethylamino)ethyl)(methyl)amino)-4-methoxyphenyl)acrylamide;
<92> *N-*(5-((4-((1-acetylindolin-7-yl)amino)-5-chloropyrimidin-2-yl)amino)-4-methoxy-2-(methyl(2-(methylamino)ethyl)amino)phenyl)acrylamide;
<93> *N-*(5-((5-chloro-4-((1-propionylindolin-7-yl)amino)pyrimidin-2-yl)amino)-2-((2-(dimethylamino) ethyl)(methyl)amino)-4-methoxyphenyl)acrylamide;
<94> *N-*(5-((5-chloro-4-((1-propionyl-1,2,3,4-tetrahydroquinolin-8-yl)amino)pyrimidin-2-yl)amino)-2-((2-(dimethylamino)ethyl)(methyl)amino)-4-methoxyphenyl)acrylamide;
<95> *N-*(5-((4-((1-acetyl-1,2,3,4-tetrahydroquinolin-8-yl)amino)-5-chloropyrimidin-2-yl)amino)-2-((2-(dimethylamino)ethyl)(methyl)amino)-4-methoxyphenyl)acrylamide;
<96> *N-*(5-((5-chloro-4-((1-(methylsulfonyl)indolin-7-yl)amino)pyrimidin-2-yl)amino)-2-((2-(dimethylamino)ethyl)(methyl)amino)-4-methoxyphenyl)but-2-inamide;
<97> *N-*(5-((4-((1-acetylindolin-7-yl)amino)-5-chloropyrimidin-2-yl)amino)-2-((2-(dimethylamino) ethyl)(methyl)amino)-4-methoxyphenyl) but-2-inamide;
<98> *N*-(5-((5-chloro-4-((4-methyl-2-(*N-*methylmethylsulfonamido)phenyl)amino)pyrimidin-2-yl)amino)-2-((2-(dimethylamino)ethyl)(methyl)amino)-4-methoxyphenyl) but-2-inamide;
<99> *N-*(5-((4-((1-acetylindolin-7-yl)amino)-5-cyanopyrimidin-2-yl)amino)-2-((2-(dimethylamino) ethyl)(methyl)amino)-4-methoxyphenyl)acrylamide;
<100> *N*-(5-((4-((1-acetylindolin-7-yl)amino)pyrimidin-2-yl)amino)-2-((2-(dimethylamino)ethyl) (methyl)amino)-4-methoxyphenyl)acrylamide; and
<101> *N*-(5 -((4-((1-acetylindolin-7-yl)amino)-5-fluoropyrimidin-2-yl)amino)-2-((2-(dimethylamino)ethyl) (methyl)amino)-4-methoxyphenyl)acrylamide.

6. A method for preparing a compound represented by Formula 1 of claim 1, comprising reacting a compound represented by Formula 2 with a compound represented by Formula 3 to prepare a compound represented by Formula 1, as represented by Reaction Scheme 1 below: 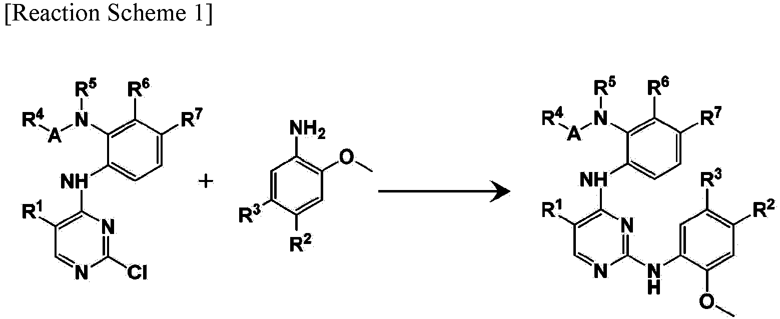 wherein in Reaction Scheme 1 above, A, R¹, R², R³, R⁴, R⁵, R⁶, and R⁷ are as defined in Formula 1 of claim 1.

7. A pharmaceutical composition for the prevention or treatment of cancer comprising the compound represented by Formula 1, an isomer thereof, a solvate thereof, a hydrate thereof, or a pharmaceutically acceptable salt thereof of claim 1 as an active ingredient.

8. A pharmaceutical composition wherein the compound represented by Formula 1, an isomer thereof, a solvate thereof, a hydrate thereof, or a pharmaceutically acceptable salt thereof of claim 1 inhibits epidermal growth factor receptor (EGFR) mutations or HER2 mutations.

9. The pharmaceutical composition of claim 8, wherein:
the EGFR mutation may comprise V769_D770insASV or D770_N771insSVD; and
the HER2 mutation may comprise A775_G776insYVMA or G776delinsVC.

10. The pharmaceutical composition of claim 7,
wherein the cancer is one or more selected from the group consisting of pseudomyxoma, intrahepatic biliary tract cancer, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphocytic leukemia, basal cell cancer, ovarian epithelial cancer, ovarian germ cell cancer, male breast cancer, brain cancer, pituitary adenoma, multiple myeloma, gallbladder cancer, biliary tract cancer, colorectal cancer, chronic myeloid leukemia, chronic lymphocytic leukemia, retinoblastoma, choroidal melanoma, ampullary cancer of Vater, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, pediatric leukemia, small intestine cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, urethral cancer, cancer of unknown primary site, gastric lymphoma, stomach cancer, gastric carcinoid, gastrointestinal stromal cancer, Wilms cancer, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid, vaginal cancer, spinal cord cancer, acoustic neuroma, pancreatic cancer, salivary gland cancer, Kaposi's sarcoma, Paget's disease, tonsil cancer, squamous cell carcinoma, pulmonary adenocarcinoma, lung cancer, lung squamous cell carcinoma, skin cancer, anal cancer, rhabdomyosarcoma, laryngeal cancer, pleural cancer, and thymic cancer.

11. The pharmaceutical composition of claim 7, wherein the pharmaceutical composition improves the anticancer effect by co-administering in combination with an anticancer agent.

12. A health functional food for the prevention or improvement of cancer comprising the compound represented by Formula 1, an isomer thereof, a solvate thereof, a hydrate thereof, or a pharmaceutically acceptable salt thereof of claim 1 as an active ingredient.
